(19) Europäisches Patentamt
European Patent Office
Office européen des brevets

(11) EP 2 226 633 A1

(12) EUROPEAN PATENT APPLICATION

(43) Date of publication:
**08.09.2010 Bulletin 2010/36**

(51) Int Cl.:
***G01N 33/50*** *(2006.01)*

(21) Application number: **09003341.6**

(22) Date of filing: **06.03.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Charité - Universitätsmedizin Berlin**
**10117 Berlin (DE)**

(72) Inventors:
- **Meisel, Andreas Prof. Dr.**
  **13127 Berlin (DE)**
- **Priller, Josef Prof. Dr.**
  **10435 Berlin (DE)**
- **Bonnas, Christel Dr.**
  **82377 Penzberg (DE)**

(74) Representative: **Bösl, Raphael Konrad**
**Isenbruck Bösl Hörschler Wichmann LLP**
**Prinzregentenstrasse 68**
**81675 München (DE)**

# (54) Uses and methods for the identification of a cytoprotective compound involving gp130 or LIFRalpha

(57) The present invention relates to the use of a protein comprising glycoprotein 130 (gp130) and/or leukemia inhibitory factor receptor alpha-chain (LIFRα) for the identification of a cytoprotective compound. Other aspects of the invention relates to a method of screening for a potential cytoprotective compound using the protein.



Printed by Jouve, 75001 PARIS (FR)

## Description

The present invention relates to the use of a protein comprising glycoprotein 130 (gp130) and/or leukemia inhibitory factor receptor alpha-chain (LIFRα) for the identification of a cytoprotective compound. Other aspects of the invention relates to methods of screening for a potential cytoprotective compound using the protein.

The cytokine superfamily is defined by shared structural feature of both ligands and receptors (Bravo *et al.,* 2000). The ligands share all the 'four-helix-bundle' such as erythropoietin (EPO), the granulocyte colony-stimulating factor (G-CSF), the interleukins or the 'gp-130' cytokines. The receptors are characterized by a cytokine receptor homology domain (CHD) in the extracellular region containing a conserved WSXWS motif (Bravo et al., 2000). The cytokine receptor family can be classified into the hematopoietic cytokine receptor family, the interferon receptor family, the tumor necrosis factor receptor family and the chemokine receptor family (Boulanger et *al.,* 2004).

Members of the leukemia inhibitory factor receptor family (LIFR) belong to the gp130 system, a set of ligands and receptors eliciting biological responses via oligomeric receptor complexes containing one or more copies of the common receptor chain gp130 (Bravo et al., 2000). The glycoprotein gp130, also known as CD130, was first identified as signal transducing subunit of the IL-6 receptor. Knockout mice for gp130 are not viable revealing thereby the importance of gp130 as part of many different types of signaling complexes (Yoshida et al., 1996). The LIF receptor subfamily consists of receptor complexes composed of the LIFRα-chain and the gp130 signaling chain and may associate with one or two further subunits such as Ciliary Neurotrophic Factor (CNTFR)Rα (Bravo *et al.,* 2000; Boulanger and Garcia, 2004).

Members of this subfamily are the receptors for LIF (LIFR), oncostatin M (OSMR) (Gearing et al., 1992), ciliary neurotrophic factor (CNTFR) (Ip et al., 1992), cardiotrophin 1 (CT-1R) (Pennica, King et al., 1995), Cardiotrophin-like cytokine (CLCR) (Elson et al., 2000) and the recently described neuropoietin (NPR) (Derouet et al., 2004). The receptor mediating biological activities of LIF comprises the LIFRα-chain, which binds LIF with low affinity, and gp130, which does not itself bind LIF but is essential for high affinity complex formation and signal transduction (Gearing et al., 1992). All cytokines binding to receptors of the LIFR family have multiple biological actions. Shared actions of this cytokine family include *in vitro* and *in vivo* neuroprotective potential and induction of astroglial differentiation of neural progenitor cells (Carpenter et al., 1999; Derouet et al., 2004; Murphy et al., 1991; Ochiai et al., 2001; Ohno et al., 2006; Pennica et al., 1995; Rajan et al., 1998; Rose et al., 1994; Uemura et al., 2002; Weiss et al., 2006).

Hematopoietic growth factors, like erythropoietin (EPO), are pleiotropic cytokines involved in the development, maintenance and protection of a variety of tissues (Metcalf, 2008). EPO, which was first isolated from the urine of anemic patients (Miyake et al., 1977), is mainly produced by peritubular interstitial cells in the kidneys and acts as a humoral agent to stimulate red blood cell production in the bone marrow. However, EPO gene expression was also detected in human and murine brain (Marti et al., 1996), raising the question of its function in the nervous system.

Experimental evidence suggests that EPO can protect neurons from cell death (Noguchi et al, 2007). The neuroprotective activity of EPO is observed as early as embryonic day 10.5 in the developing mouse brain (Yu et al., 2002). In the adult nervous system, persistent expression of EPO and upregulation of EPO synthesis under hypoxic conditions via the hypoxia-inducible factor (H1F) supports the notion of EPO as an endogenous neuroprotectant (Marti et al., 1996; Chikuma et al., 2000; Prass et al, 2003). Preconditioning with EPO was shown to reduce excitotoxic and hypoxic damage of cultured neurons (Siren et al., 2001; Digicaylioglu et al., 2001; Ruscher et al., 2002). Moreover, administration of recombinant EPO resulted in significant tissue protection in rodent models of cerebral ischemia (Bernaudin et al., 1999; Sakanaka et al., 1997; Brines et al., 2000; Siren et al., 2001), multiple sclerosis (Sattler et al., 2004), spinal cord injury (Celik et al., 2002), Parkinson's disease (Puskovic et al, 2006) and peripheral nerve injury (Campana and Myers, 2003). The tissue-protective function of EPO also extends beyond the nervous system, as demonstrated in animal models of myocardial infarction and acute renal injury (Calvillo et al., 2003; Vesey et al., 2004). EPO can cross the blood-brain barrier in rodents and humans (Brines et al., 2000; Banks et al., 2004; Ehrenreich et al., 2004; Statler et al., 2007), suggesting that circulating EPO may penetrate into the brain tissue. These experimental results have promoted the recent evaluation of recombinant EPO as a neuroprotective therapy in stroke and schizophrenia (Ehrenreich et al., 2002; Ehrenreich et al., 2004). However, the amounts of peripherally administered EPO are limited by hematopoietic effects.

In the hematopoietic system, EPO binds to a specific homodimeric receptor $(EPOR)_2$, which is expressed at highest density on erythroid progenitor cells in the bone marrow, but can also be found in other tissues, including the brain (Marti et al., 1996). The EPOR, a member of the class I cytokine receptor superfamily, exists as an unliganded homodimer on the cell surface (Livnah et al., 1999). Ligand binding induces a conformational change, which results in the activation of intracellular signal transduction pathways through the Janus kinases (JAK) (Witthuhn et al., 1993). Recently, a second EPO heteroreceptor was identified, which selectively mediates tissue protection (Brines et al., 2004). EPO binds to this heteroreceptor composed of EPOR and the common β receptor (βCR), which is shared by many hematopoietic cytokine receptors as the signal-transducing unit. The complexity is increased by tissue-specific splicing of EPOR transcripts (Nakamura et al., 1992; Chin et al, 2000).

As previously described for commercially available rhEPO (Siren et al., 2001; Ruscher et al., 2002), Janus kinase-2 (JAK-2), phosphoinositide-3 kinase (PI3K) and the downstream effector serine/threonine protein kinase B (Akt) are involved in EPO-mediated neuroprotection by inactivation of the proapoptotic protein BAD. Signalling of receptors bound by EPO depends on their association with JAKs, a common feature of all members of the cytokine receptor superfamily (Ihle, 1995).

However, the use of EPO as a cytoprotective compound has the drawback that the cytoprotective activies of EPO can not be separated from its hematopoietic activities. Howver, under certain circumstances EPO's hematopoietic activities may be undesired adverse effects.

In the light of such drawbacks associated with the use of known factors as cytoprotective compounds there is a great need for the identification of novel cytoprotective compounds which do not show additional effects (such as hematopoietic activities) which are undesired. Therefore, efforts need to be directed to the identification of new cytoprotective compounds which are devoid of additional undesired effects, particukarly hematopoietic activities. Thus, it is the object of the present invention to provide a new means for the identification of cytoprotective compounds, which overcomes such drawbacks.

This object is solved by the subject matters as provided by the claims. The invention is based on the novel finding that alternative splice variants of EPO identified by the inventors have which are non-hematopoietic and do not bind to the classical EPO receptor. Instead, they provide cytoprotection a through alternative binding site involving leukaemia inhibitory factor receptor family. The EPO isoforms induce anti-apoptotic mechanisms by activating Janus kinase-2 and downstream effectors Akt and nuclear factor-kappaB. Thus, there is a novel endogenous tissue-protective systems which may be an interesting target for new therapeutics.

In particular, the object is solved by the use of a protein or protein complex comprising glycoprotein 130 (gp130) and/or leukemia inhibitory factor receptor alpha-chain (LIFR$\alpha$) for the identification of a cytoprotective compound. The present invention is based on the finding that a substance which binds to a protein comprising gp130 or LIFR$\alpha$ may represent a potential cytoprotective compound, the cytoprotective effect of which can be confirmed by further tests.

A protein is an organic compound composed of amino acids arranged in a linear chain and essentially joined together by peptide bonds between the carboxyl and amino groups of adjacent amino acid residues. The residues in a protein may be chemically modified by post-translational modification, which can happen either before the protein is used in the cell, or as part of control mechanisms. Proteins can also work together to achieve a particular function, and they often associate to form stable protein complexes.

A protein complex is a group of two or more proteins. Protein complexes are a form of quaternary structure. Proteins in a protein complex are linked by non-covalent protein-protein interactions, and different protein complexes have different degrees of stability over time. Protein complex formation often serves to activate or inhibit one or more of the complex members. A method that is commonly used for identifying the members of protein complexes is immunoprecipitation.

In the present invention, the protein or protein complex comprises gp130 and/or LIFR$\alpha$, preferably LIFR$\alpha$, more preferably gp130 and LIFR$\alpha$. If the protein or protein complex comprises gp130 and LIFR$\alpha$ and optionally further components, they may be joined by peptide or other bond into one protein or may be joined by non-covalent protein-protein interactions into a protein complex.

A cytoprotective compound is defined as being a compound which protects cells from disadvantages effects of noxious chemicals or other stimuli. Preferably, cytoprotection is defined as increasing viability or survival of a test cell by at least 10 %, more preferably at least 25 %, still more preferably at least 50 %, preferably at least 60 %, more preferably at least 70 %, 80 % or 90 %, still more preferably 95 % if compared to a control in the absence of cytoprotecion.. The cytoprocetive activity may be determined as described in the examples, particularly in Examples I.16 and I.19.

In particular, the cytoprotective compounds to be identified by the methods of the present invention acting their natural environment, for example within an animal cell, via a receptor of the cytokine receptor family. In a preferred embodiment, the cytoprotective compounds as identified by the methods of the present invention act via a receptor of the LIFR family. In another preferred embodiment, the cytoprotective compounds to be identified by the methods of the present invention act via a receptor comprising gp130 and/or LIFR$\alpha$, preferably LIFR$\alpha$, more preferably gp130 and LIFR$\alpha$. Such a receptor may be the classical dipartite LIFR, or a more complex receptor involving LIFR$\alpha$ and gp130 such as CNTFR and CT-1R.

Examples of cytoprotective compounds exemplarily identified with the methods of the present invention (see Example) are alternatively spliced human erythropoietin (EPO) transcripts denoted hS3 with a complete loss of exon 3 (SEQ ID No. 2 with or without leader) or denoted hS4 with a partial loss of exon 4 (SEQ ID No. 3 with or without leader). In murine tissue, one EPO variant was found with a complete loss of exon 4 denoted mS (SEQ ID No. 5 with or without leader), which shows cytoprotective activities. Amino acid sequences of these variants with leader are given in Fig. 5A.

Cytoprotective compounds to be identified by the present invention encompass numerous chemical classes, including but not limited to, small organic or inorganic compounds, natural or synthetic molecules, such as antibodies, proteins or fragments thereof, antisense nucleotides, interfering RNA (iRNA) and ribozymes. Preferably, the compounds to be identified by the present invention are small organic compounds (i.e., those having a molecular weight of more than 100 Daltons yet less than about 1000 Daltons). Compounds to be identified by the present invention comprise functional chemical groups necessary for structural interactions with polypeptides and can include at least an amine, carbonyl, hydroxyl or carboxyl group, two of the functional chemical groups or three or more of the functional chemical groups. The candidate compounds can comprise cyclic carbon or heterocyclic structure and/or aromatic or polyaromatic structures substituted with one of the above identified functional groups. Candidate compounds also can be biomolecules, such as peptides, saccharides, fatty acids, sterols, isoprenoids, purines, pyrimidines, derivatives or structural analogs of the above or combinations thereof and the like. Where the compound is a nucleic acid, the compound typically is a DNA or RNA molecule, although modified nucleic acids having non-natural bonds or subunits are also contemplated. Modified nucleic acids can comprise nucleoside analogs, nucleotide analogs, non-nucleoside analogs, non-nucleotide analogs and others.

Compounds to be identified by the present invention can be obtained from a wide variety of sources including libraries of synthetic or natural compounds. For example, numerous means are available for random and directed synthesis of a wide variety of organic compounds and biomolecules, including expression of randomized oligonucleotides, synthetic organic combinatorial libraries, phage display libraries of random peptides and the like. Compounds to be identified by the present invention can also be obtained using any of the numerous approaches in combinatorial library methods known in the art, including biological library methods; spatially addressable, parallel solid phase or solution phase library methods, synthetic library methods requiring deconvolution, the "one-bead one-compound" library method, and synthetic library methods using affinity chromatography selection (Lam, 1997). Alternatively, libraries of natural compounds in the form of bacterial, fungal, plant and animal extracts are available or readily produced. Additionally, natural and synthetically produced libraries and compounds can be readily modified through conventional chemical, physical and biochemical means.

Further, known pharmacological agents can be subjected to directed or random chemical modifications, such as acylation, alkylation, esterification, amidation, etc. to produce structural analogs of the agents. Compounds to be identified by the present invention can be selected randomly or can be based on existing compounds that bind to gp130 and/or LIFRα or a protein (complex) comprising gp130 and/or LIFRα. Therefore, a source of compounds to be identified by the present invention could be any compound known to bind to gp130 and/or LIFRα or a protein comprising gp130 and/or LIFRα in which the structure of the compound is changed at one or more than one position of the molecule such that the binding capacity and optionally the cytoprotective activity of the starting compound is maintained, however, other undesired activities are eliminated.

As detailed above, gp130 (also known as IL6ST, IL6-beta or CD130) is a transmembrane protein which is the founding member of the class of tall cytokine receptors. It forms one subunit of type I cytokine receptors within the IL-6 receptor family. It is often referred to as the common gp130 subunit, and is important for signal transduction following cytokine engagement. As with other type I cytokine receptors, gp130 possesses a WSXWS amino acid motif that ensures correct protein folding and ligand binding. It interacts with Janus kinases to elicit an intracellular signal following receptor interaction with its ligand. Structurally, gp130 is composed of five fibronectin type-III domains and one immunoglobulin-like C2-type (immunoglobulin-like) domain in its extracellular portion. The sequence of gp130 of different species (e.g. Homo sapiens, Mus musculus, Canis familiaris and Gallus gallus) is known in the art and may be derived from the Homepage of the National Center for Biotechnology Information (http://www.ncbi.nlm.nih.gov/).

The human glycoprotein gp130, also known as CD130, Interleukin-6 receptor subunit beta, IL-6R-beta, Interleukin-6 signal transducer, Membrane glycoprotein 130, CDw130, Oncostatin-M receptor alpha subunit, is a protein of 130 kDa containing 918 amino acids. It is encoded on chromosome 5q11 with 17 exons. The protein, genomic and coding polynucleotide sequences of human gp130 are known in the state of the art (EP-B 0 411 946). They are also available from the NCBI for example, under the data base accession numbers EF064722 (coding sequence) and ABK41905 (protein sequence), the accession numbers M57230 (coding sequence) and AAA59155 (protein sequence) and the accession number BC117402 (coding sequence) and AAI17403 (protein sequence).

Also the mouse coding sequence and the corresponding protein sequence has been determined and is available at NCBI, for example, under the data base accession number M83336 (coding sequence) and AAA37723 (protein sequence).

Gp130 is a shared receptor for at least nine cytokines and can signal either as a homodimer or as a heterodimer with Leukemia Inhibitory Factor Receptor alpha (LIFRα) which can associate with additional cytokine receptor subunits, such as ciliary neurotrophic factor (CNTF)Ralpha (Boulanger and Garcia, 2004). CNTF, LIF, cardiotrophin-1, cardiotrophin-like cytokine, neuropoietin and oncostatin M all act through the gp130/LIFR receptor subfamily and share neuroprotective functions (Lo et al.;1995; Ikeda et al.; 1996; Bordet et al.; 1999; Derouet et al.; 2004; Schuettauf et al.; 2005; Weiss et al.; 2006).

Gp130 can be derived from any sequence available that allows for its specific purpose according to the different aspects of the present invention. Preferably, gp130 is human gp130.

The leukemia inhibitory factor is a polyfunctional cytokine that affects the differentiation, survival, and proliferation of a wide variety of cells in the adult and the embryo. LIF action appears to be mediated through a high-affinity receptor complex composed of a low-affinity LIF binding chain (LIFRα) and a high-affinity converter subunit, gp130. Both LIFRα and gp130 are members of a family of cytokine receptors that includes components of the receptors for the majority of hematopoietic cytokines and for cytokines that affect other systems, including the ciliary neurotrophic factor, growth hormone and prolactin.
The sequence of LIFRα of different species (e.g. Homo sapiens, Rattus norvegicus, Mus musculus, Canis lupus familiaris and Gallus gallus) is known in the art and may be derived from the Homepage of the National Centre for Biotechnology Information; National Library of Medicine, Building 38A, Bethesda, MD20894, USA (http://www.ncbi.nlm.nih.gov/).

Human leukemia inhibitory factor receptor alpha is a protein of 118 kDa, also known as Cluster of differentiation 118 (CD118) with 1097 amino acids. The protein, genomic and coding polynucleotide sequences are known in the state of the art and are e. g. publicly available from the NCBI, for example, under the data base accession numbers NM_001127671 (coding sequence) and NP_001121143 (protein sequence) and the accession numbers NM_002310 (coding sequence) and NP_002301 (protein sequence). It is located on chromosome 5p13-p12. The mouse LIFRα is available under NM_013584 (coding sequence) and NP_038612 (protein sequence). It is located on chromosome 15:7.1-7.14 Mb. The protein is also available under P42702 (human).

LIFRα can be derived from any sequence available that allows for its specific purpose according to the different aspects of the present invention. Preferably, LIFRα is human LIFRα.

Suitable methods for identification of a cytoprotecztive compound are described in the following, for example in the context of the methods of the invention.

In a preferred aspect of the present invention, the protein or protein complex comprises or consists of a receptor of the formula:

$$(X / gp130 / Y)_n,$$

wherein
X is

- CNTFRα or
- gp80 or
- absent,

Y is

- leukemia inhibitory factor receptor alpha-chain (LIFRα) or
- interleukin 6 receptor (IL-6 R) or
- interleukin 11 receptor (IL-11 R) or
- interleukin 27 receptor (IL-27 R) or
- oncostatin M receptor α (OSMRα),
  and n is 1 or 2.

The ciliary neurotrophic factor receptor also known as CNTFR binds the ciliary neurotrophic factor. This receptor and its cognate ligand support the survival of neurons. This receptor is most closely related to the interleukin-6 receptor. This receptor possesses an unusual attachment to the cell membrane through a glycophosphatidylinositol linkage.

Interleukin 6 receptor, interleukin 11 receptor, interleukin 27 receptor and oncostatin M receptor are type I cytokine receptors. The IL6 receptor is a protein complex consisting of a IL-6 receptor subunit (IL6R) and interleukin 6 signal transducer gp130. IL6R also denotes the human gene encoding this subunit. Alternatively spliced transcript variants encoding distinct isoforms have been reported. IL6R subunit also shared by many other cytokines. Interleukin 11 receptor is a heterodimer comprised of interleukin 11 receptor alpha subunit (IL-11 R) and an incompletely characterized beta subunit. Interleukin 27 receptor subunit is a heterodimer composed of the interleukin 27 receptor alpha subunit (IL-27 R) and glycoprotein 130. IL27RA essential for transcriptional activation of STAT1.

In an even more preferred aspect of the present invention, wherein the protein or protein complex comprises or consists of gp130 and LIFRα, particularly of a receptor of the formula:

X / gp130 / LIFRα

wherein X is CNTFRα or gp80 or absent (see above).

The present invention is further directed to a method of screening for a potential cytoprotective compound.The method of screening for a potential cytoprotective compound comprises the following steps:

(a) contacting a protein or protein complex as defined above with a substance; and
(b) detecting interaction of the substance with the protein or protein complex, whereby identifying the substance as a potential cytoprotective compound.

In one embodiment the method further comprises the steps of:

(c) contacting a cell with the potential cytoprotective compound identified in step (b);
and
(d) detecting a cytoprotective effect of the potential cytoprotective compound on the cell, whereby identifying the potential cytoprotective compound as cytoprotective compound.

For the method of the present invention, the protein or protein complex as defined above is contacted with a substance. The substance tested with the method of the present invention may be any test substance or test compound of any chemical nature. It may already be known as a drug or medicament for a disease. Alternatively, it may be a known chemical compound not yet known to have a therapeutic effect in another embodiment and the compound may be a novel or so far unknown chemical compound. The agent may be also a mixture of test substances or test compounds.

In the context of the present invention, the protein (complex) is contacted with the substance for a time and under conditions suitable for interaction with the protein (complex) and detecting the same. Suitable conditions include appropriate temperature and solution to avoid e.g. denaturation of proteins involved or to maintain viable cells, if present. Suitable conditions will depend from the particular test system chosen and the skilled person will be able to select the same based on his general knowledge. The contacting may be carried out in a cell-based assay or a cell-free assay.

After the contacting of the protein (complex) with the substance, the effect of the substance on the protein (complex) is detected. In the following, a series of different detection systems will be described in more detail. However, it should be understood that these are exemplary and other test systems may be also appropriate.

For the method of the invention any suitable detection may be used. Suitable methods may be chosen depending from the characteristics of the test system including the protein (complex) and substance(s) to be tested. As detailed above, the binding/activation of the protein (complex) is followed by a signal cascade involving e.g. Janus kinase 2 (Jak2), Janus kinase 1 (Jak1), Janus kinase 3 (Jak3), protein kinase B (Akt), IκB kinase (IKK) and/or nuclear factor-kappa B (NF-κB). Accordingly, the interaction of the substance with the protein (complex) or a component upstream in the signal transduction of may be determined. Interaction may be determined directly or indirectly. "Directly" means that the binding of the substance to the protein (complex) is determined (e.g. using a labeled marker in order to detect protein/substance complexes). "Indirectly" means that an effect downstream in the signal transduction is determined (e.g. activity or amount of Janus kinase 2 (Jak2), Janus kinase 1 (Jak1), Janus kinase 3 (Jak3), protein kinase B (Akt), IκB kinase (IKK) and/or nuclear factor-kappa B (NF-κB). Suitable methods are detailed e.g. in the examples.

In the first case agent protein interactions are measured. A series of tests are known in the art in which the test system may be used and to which the test system may be adapted. This may be a heterogeneous or homogeneous assay. As used herein, a heterogeneous assay is an assay which includes one or more washing steps, whereas in a homogeneous assay such washing steps are not necessary. The reagents and compounds are only mixed and measured.

In an embodiment the assay is an ELISA (enzyme linked immuno sorbent assay), a DELFIA (dissociation enhanced lanthanide fluoro immuno assay), an SPA (scintillation proximity assay) a flashplate assay, a FRET (fluorescence resonance energy transfer) assay, TR-FRET (time-resolved fluorescence resonance energy transfer) assay, a FP (fluorescence polarisation) assay, an ALPHA (amplified luminescent proximity homogenous assay), an EFC (enzyme fragment complementation) assay, a two hybrid assay or a coimmunoprecipitation assay.

ELISA (enzyme linked immuno sorbent assay)-based assays are offered by various companies. It is a biochemical technique used to detect the presence of an antibody or an antigen in a sample. Performing an ELISA involves at least one antibody with specificity for a particular antigen (e.g a segment of the first or second protein). In general, an unknown amount of antigen in a sample is immobilized on a surface. One then washes a particular antibody over the surface. This antibody is linked to an enzyme that produces a detecable signal such as a change in colour or fluorescene. For example, the sample with an unknown amount of antigen is immobilized on a solid support (usually a microtiter plate) either non-specifically (via adsorption to the surface) or specifically (via capture by another antibody specific to the same antigen, in a "sandwich" ELISA). After the antigen is immobilized the detection antibody is added, forming a complex with the antigen. The detection antibody can be covalently linked to an enzyme, or can itself be detected by a secondary antibody which is linked to an enzyme through bioconjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. Older ELISAs utilize chromogenic substrates, though newer assays employ fluorogenic substrates with much higher sensitivity.

DELFIA (dissociation enhanced lanthanide fluoro immuno assay)-based assays are solid phase assay. The antibody is usually labelled with Europium or another lanthanide and the Europium fluorescence is detected after having washed away un-bound Europium-labelled antibodies.

SPA (scintillation proximity assay) and the flashplate assay usually exploit biotin/avidin interactions for capturing radiolabelled substrates. Generally the reaction mixture includes the kinase, a biotinylated peptide substrate and $\gamma$-[$^{33}$P] ATP. After the reaction, the biotinylated peptides are captured by streptavidin. In the SPA detection, streptavidin is bound on scintillant containing beads whereas in the flashplate detection, streptavidin is bound to the interior of the well of scintillant containing microplates. Once immobilized, the radiolabelled substrate is close enough to the scintillant to stimulate the emission of light.

Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically $<10$ nm). As both molecules are fluorescent, the energy transfer is often referred to as "fluorescence resonance energy transfer", although the energy is not actually transferred by fluorescence. FRET is a useful tool to detect and quantify protein-agent interactions, protein-protein interactions, protein-DNA interactions, and protein-conformational changes. For monitoring binding of a protein to an agent, one protein to another or a protein to DNA, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Suitable neighbors for FRET are known in the art and the skilled practitioner will be able to choose a suitable combination of labels for both antibodies. As used herein with respect to donor and corresponding acceptor, "corresponding" refers to an acceptor fluorescent moiety having an emission spectrum that overlaps with the excitation spectrum of the donor. However, both signals should be separable from each other. Accordingly, the wavelength maximum of the emission spectrum of the acceptor should preferably be at least 30 nm, more preferably at least 50 nm, such as at least 80 nm, at least 100 nm or at least 150 nm greater than the wavelength maximum of the excitation spectrum of the donor.

Representative donor fluorescent moieties that can be used with various acceptor fluorescent moieties in FRET technology include fluorescein, Lucifer Yellow, B-phyco-erythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC -Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO).

Alternatively, time-resolved fluorescence resonance energy transfer (TR-FRET) may be used for the test system of the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro- to millisecond measurements. Fluorescence lanthanide chelates have been used as energy donors in the late seventies. The commonly used lanthanides include samarium (Sm), europium (Eu), terbium (Tb) and dysprosium (Dy). Because of their specific photophysical and spectral properties, complexes of lanthanides are of major interest for fluorescence application in biology. Specifically, they have a large stroke's shift and extremely long emission half-lives (from microseconds to milliseconds) when compared to more traditional fluorophores.

Usually, organic chromophores are used as acceptors. These include allophycocyanin (APC). Suitable details on TR-FRET as well as acceptors are described in WO 98/15830.

Fluorescence polarisation (FP)-based assays are assays which use polarized light to excite fluorescent substrate peptides in solution. These fluorescent peptides are free in solution and tumble, causing the emitted light to become depolarised. When the substrate peptide binds to a larger molecule, its tumbling rates are greatly decreased, and the emitted light remains highly polarized (see also Example 4.3).

In a further embodiment of the invention, the test system of the invention is adapted for an amplified luminescence proximity homogeneous assay (ALPHA). ALPHA is a solution-based assay which was originally developed by Packard BioScience. ALPHA is a luminescence-based proximity assay, wherein one interaction partner is attached to donor beads, while the other is coupled to acceptor beads, both with a diameter of only about 250 nm. A photosensitizer compound is embedded into the donor bead. With this compound upon illumination with laser light at a wavelength of about 680 nm, ambient oxygen is converted into energy-rich, short-life singlet oxygen. When no acceptor bead is in proximity, the singlet oxygen decays without producing a signal. If donor and acceptor bead are brought together (ca. 250 nm) by the biological interaction of the attached biomolecules, the singlet oxygen released by the donor bead initiates a luminescence/fluorescence cascade in the nearby acceptor bead, leading to a highly amplified signal in the 520-620 nm range. The luminescence signal is detected in a suitable reader. For more details regarding ALPHA techniques, see Ullman et al., 1994, Proc. Natl. Acad. Sci., USA 91, 5426-5430.

EFC (enzyme fragment complementation)-based assays or equivalent assays can be used in particular for high-throughput screening of compounds. The EFC assay is based on an engineered β-galactosidase enzyme that consists of two fragments - the enzyme acceptor (EA) and the enzyme donor (ED). When the fragments are separated, there is no β-galactosidase activity, but when the fragments are together they associate (complement) to form active enzyme. The EFC assay utilizes an ED-analyte conjugate in which the analyte may be recognized by a specific binding protein, such as an antibody or receptor. In the absence of the specific binding protein, the ED-analyte conjugate is capable of complementing EA to form active β-galactosidase, producing a positive luminescent signal. If the ED-analyte conjugate is bound by a specific binding protein, complementation with EA is prevented, and there is no signal. If free analyte is provided (in a sample), it will compete with the ED-analyte conjugate for binding to the specific binding protein. Free analyte will release ED-analyte conjugate for complementation with EA, producing a signal dependent upon the amount of free analyte present in the sample.

Two-hybrid screening is a molecular biology technique used to discover protein-protein interactions by testing for physical interactions (such as binding) between two proteins. The premise behind the test is the activation of downstream reporter gene(s) by the binding of a transcription factor onto an upstream activating sequence. For the purposes of two-hybrid screening, the transcription factor is split into two separate fragments, called the binding domain (BD) and activating domain (AD). The BD is the domain responsible for binding to the UAS and the AD is the domain responsible for activation of transcription.

Co-immunoprecipitation can use for the identification of protein complexes by precipitating one protein believed to be in a complex, additional members of the complex are captured as well and can be identified. The protein complexes, once bound to the specific antibody, are removed from the bulk solution by capture with an antibody-binding protein attached to a solid support such as an agarose bead. These antibody-binding proteins (Protein A, Protein G, Protein L) were initially isolated from bacteria and recognize a wide variety of antibodies. Following the initial capture of a protein or protein complex, the solid support is washed several times to remove any proteins not specifically and tightly bound through the antibody. After washing, the precipitated protein(s) are eluted and analyzed using gel electrophoresis, mass spectrometry, western blotting, or any number of other methods for identifying constituents in the complex. Thus, co-immunoprecipitation is a standard method to assess protein-protein interaction.

In another preferred embodiment of the invention the means for detecting the interaction between the first and second protein may be adapted to measure one or more components downstream of the protein (complex) as defined above in the signal cascade. The measuring may include the determination of the activity or concentration Janus kinase 2 (Jak2), Janus kinase 1 (Jak1), Janus kinase 3 (Jak3), protein kinase B (Akt), IκB kinase (IKK) and/or nuclear factor-kappa B (NF-κB). The concentration may be measured in response to a potential modulator as described above. Means and methods for determining concentrations of one or more nucleic acids or proteins are well known to the skilled person and include such involving RT-PCR, mass spectrometry etc. Suitable methods are also described in, e.g., Examples 1.6 and I.11 to 13.

Exemplary methods and their use are described in the Examples, particularly I.6-I.21

Preferably, the method is adapted for high-through put screening. In this method a large number of compounds is screened against the agents in either cell-free or whole-cell assays. Typically, these screenings are carried out in 96 well plates using automated, robotic station based technologies or in higher- density array ("chip") formats.

If a cell-based assay is used, the cell may be isolated (optionally genetically modified), maintained and cultured as known to the skilled person. Aside from temperature and gas mixture, the most commonly varied factor in cell culture systems is the growth medium. Recipes for growth media can vary in pH, glucose concentration, growth factor and the presence of other nutrient components among others. Growth factors used for supplement media are often derived from animal blood such as calf serum. Genetically modified cells may be obtained by inserting the full-length coding sequence of the ion channel, as known to the skilled person. The skilled person in the art knows how to derive a nucleic acid sequence coding for the components of the protein (complex) as defined above and how to isolate or produce such a nucleic acid sequence using standard techniques of molecular biology. This can be accomplished, for example, by the use and combination of existing sequences using restriction enzymes. The nucleic acid may be combined with further elements, e.g., a promoter and a transcription start and stop signal and a translation start and stop signal in order to provide for expression of the protein (complex) or component thereof. The resulting nucleic acid sequence may be introduced into cells e.g. using a virus as a carrier or by transfection including e.g. by electroporation, heat shock, magnetofection, nucleofection and the use of transfection agents (see also below). Cell culture and transfection of cells is also described in Example I.5.

Principally, any cell may be protected by the cytoprotective compounds as identified by the methods of the present invention; however it is expected that the cell to be protected by the cytoprotective compound as identified by the present invention carries a receptor of the cytokine receptor family. The receptor may be a receptor of the LIFR family. In a preferred embodiment, the cell carries a receptor comprising gp130 and/or LIFRα. Such a receptor may be the classical dipartite LIFR, or a more complex receptor involving LIFRα and gp130 such as CNTFR and CT-1R. Thus, the cell is preferably a cell which expresses gp130 or LIFRα or preferably which expresses gp130 and LIFRα. The cell involved in the methods of the invention can optionally be genetically modified to express LIFRα and/or gp130. Genetically modified means that the cell has been transformed with a nucleotide sequence which expresses gp130 and/or LIFRα. Such transformation with foreign genetic material may occur by transfection of eukaryotic cells in tissue cultures, conjugation by cell-cell contact between bacteria or transduction by viruses (see also below).

Thus, nucleic acid constructs comprising a nucleotide sequence encoding gp130 and/or LIFRα may be operably linked to one or more control sequences that direct the expression of the coding sequence in a suitable host cell under conditions compatible with the control sequences. A nucleotide sequence encoding the above polypeptides may be manipulated in a variety of ways to provide for expression of the polypeptides. Manipulation of the nucleotide sequence prior to its insertion into a vector may be desirable or necessary depending on the expression vector. The techniques for modifying nucleotide sequences utilizing recombinant DNA methods are well known in the art.

The present invention may involve a vector comprising the above mentioned nucleotide sequence in such a manner that the vector is replicable and can express the protein encoded by the nucleotide sequence in a host cell. In addition, the present invention discloses a host cell transformed by this vector. This host-vector system is not particularly restricted and fusion protein expression systems with other proteins can also be used. Examples of the fusion protein expression system include those using tags such as MBP (maltose binding protein), GST (glutathione-S-transferase), HA (hemagglutinin), His (hexahistidine), myc, Fas, and the like. The tags may be used for purification and/or detection of the marked proteins or their interaction partners. Tagging of Proteins in also described in Example I.4.

Examples of the vector include plasmid vectors (e.g., expression vectors for prokaryotic cells, yeasts, insect cells, and animal cells), virus vectors (e.g., retrovirus vectors,adenovirus vectors, adeno-associated virus vectors, herpesvirus vectors, Sendai virus vectors, HIV vectors, and vaccinia virus vectors), and liposome vectors (e.g., cationic liposome vectors).

In order to express the desired amino acid sequence practically by introducing the vector according to the present invention into a host cell, the vector may contain, in addition to the base sequence according to the present invention, other sequences for controlling the expression (e.g., promoter sequences, terminator sequences and enhancer sequences) and gene markers for selecting microorganisms, insect cells, animal culture cells, or the like (e.g., neomycin resistance genes and kanamycin resistance genes). Furthermore, the vector may contain the base sequence according to the present invention in a repeated form (e.g., in tandem) . These base sequences may also be introduced into a vector according to the conventional manner, and microorganisms, insect cells, animal cultured cells, and the like may be transformed by the vector based on the method conventionally used in this field.

The vector may be constructed based on the procedure and manner which are conventionally used in the field of genetic engineering.

Furthermore, examples of the host cell include Escherichia coli, yeasts, insect cells and animal cells such as COS cells [e.g., C0S7 cells), mink lung epithelial cells (e.g., MvlLu), lymphocytes, fibroblasts, CHO cells, blood cells, tumor cells, and the like.

The transformed host cells are cultured in an appropriate medium, and the protein according to the present invention may be obtained from the culture product. The protein according to the present invention may be recovered from the culture medium and purified in the conventional manner.

The cell used for the cytoprotection test (e.g. in steps c) and d)) of the method of the invention ), preferably expressing the protein or protein complex as defined for the uses ofc the present invention, may be selected from the group consisting of a myoblast (such as rat heart myoblast cell line H9c2 (e.g. ATCC CRL446)), a neuronal cell, a neural stem cell, a neural progenitor cell, a murine pro-B IL-3 cell (Ba/F3 cells (e.g. DSM ACC26)), and a myeloid leukemia cell (such as mouse myeloid leukemia cell line M1 (e.g. (ATCC TIB192)), a hematopoietic stem cell, a hematopoietic precursor cell and a mesodermal stromal (stem) cell.

The cytoprotective effect of the potential cytoprotective compound may be detected as described in the Examples. Exemplary methods for measuring cytoprotection are given in Examples I.9, I.10, I, 16 and I.19.

In another embodiment, the cytoprotective effect of the potential cytoprotective compound on the cell is determined by detecting viability or survival of the contacted cell, optionally under conditions being disadvantageous to the cell. The person skilled in the art knows tests for the detection of the viability and survival of the contacted cells such as the MTT assay which is an assay for measuring the vitality of cells. Exemplary methods for measuring cytoprotection are given in Examples 1.9, I.10, I.16 and I.19.

Conditions which are disadvantageous to the cell are conditions which impair the viability and the survival of a cell. Such conditions may, for example, be hypoxic conditions, conditions of oxygen-glucose deprivation.

In a still further embodiment, the cytoprotective effect of the potential cytoprotective compound on the cell is determined by detecting a component downstream in the signal cascade of the protein, particularly Janus kinase 2 (Jak2), Janus kinase 1 (Jak1), Janus kinase 3 (Jak3), protein kinase B (Akt), IκB kinase (IKK), nuclear factor-kappa B (NF-κB) and/or the family of signal tranducers and activators of transcription (STAT).

Janus kinase (Jak) is a family of intracellular non-receptor tyrosine kinases that transduce cytokine-mediated signals via the JAK-STAT pathway. The Jaks possess two near-identical phosphate-transferring domains. One domain exhibits the kinase activity while the other negatively regulates the kinase activity of the first. There are four Jak family members: Janus kinase 1 (Jak1), Janus kinase 2 (Jak2), Janus kinase 3 (Jak3) and Tyrosine kinase 2 (Tyk2).

The protein, genomic and coding polynucleotide sequences are known in the state of the art and are e. g. publicly available from the NCBI, for example, under the data base accession numbers NM_002227 (coding sequence) and NP_002218 (protein sequence) for Jak1, the accession numbers NM_004972 (coding sequence) and NP_004963 (protein sequence) for Jak2, the accession numbers NM_000215 (coding sequence) and NP_000206 (protein sequence), for Jak3 and the accession numbers NM_003331 (coding sequence) and NP_003322 (protein sequence), for Tyk2. See also NG007872 for Tyk2.

Since members of the type I and type II cytokine receptor families possess usually no catalytic kinase activity, they rely on the Jak family of tyrosine kinases to phosphorylate and activate downstream proteins involved in their signal transduction pathways. The receptors exist as paired polypeptides thus exhibiting two intracellular signal-transducing domains. Jaaaks associate with a proline-rich region in each intracellular domain, which is adjacent to the cell membrane and called a box1/box2 region. After the receptor associates with its respective cytokine/ligand it goes through a conformational change, bringing the two Jaks close enough to phosphorylate each other. The Jak autophosphorylation induces a conformational change within itself enabling it to transduce the intracellular signal by further phosphorylating and activating transcription factors called STATs (Kisseleva et al.; 2002). The activated STATs dissociate from the receptor and form dimers before translocating to the cell nucleus where they regulate transcription of selected genes.

Some examples of the molecules that utilize JAK/STAT signaling pathway are colony-stimulating factor, prolactin, growth hormone, and many cytokines.

Jaks range from 120-140 kDa in size and have seven defined regions of homology called Janus homology domain 1-7 (JH1-7). JH1 is the kinase domain important for the enzymatic activity of the JAK and contains typical features of a tyrosine kinase such as conserved tyrosines necessary for Jak activation (e.g. Y1038/Y1039 in Jak1, Y1007/Y1008 in Jak2, Y980/Y981 in Jak3, and Y1054/Y1055 in Tyk2). Phosphorylation of these dual tyrosines leads to the conformational changes in the Jak protein to facilitate binding of substrate. JH2 is a pseudokinase domain, a domain structurally similar to a tyrosine kinase and is essential for a normal kinase activity yet lacks enzymatic activity. This domain may be involved in regulating the activity of JH1. The JH3-JH4 domain of Jaks shares homology with Src-homology-2 (SH2) domains. The amino terminal ($NH_2$) end (JH4-JH7) of Jaks is called a FERM domain (short for band 4.1 ezrin, radixin and moesin); this domain is also found in the focal adhesion kinase (Fak) family and is involved in association of Jaks with cytokine receptors and/or other kinases (Kisseleva et al,; 2002). In humans, there are three genes in the "Akt family": Akt1, Akt2, and Akt3. These genes code for the highly homologous protein kinases AKT1/PKBα, AKT2/PKBβ and AKT3/PKBy that are members of the serine/threonine-specific protein kinase family (EC 2.7.11.1)

Akt1 is an important molecule in mammalian cellular signalling. Akt1 is involved in cellular survival pathways by inhibiting apoptotic processes. Akt1 is also able to induce protein synthesis pathways, and is therefore a key signaling protein in the cellular pathways that lead to skeletal muscle hypertrophy, and general tissue growth. Since it can block apoptosis, and thereby promote cell survival, Akt1 has been implicated as a major factor in many types of cancer.

Akt2 is an important signaling molecule in the insulin signaling pathway. It is required to induce glucose transport (Robert et. al., 2003)

The role of Akt3 is less clear, though it appears to be predominantly expressed in brain. It has been reported that mice lacking Akt3 have small brains (Yang et al.; 2004)

The protein and coding polynucleotide sequences of the Akt1 protein are known in the state of the art. They are available from the NCBI (National Centre for Biotechnology Information; National Library of Medicine, Building 38A, Bethesda, MD20894, USA; www.ncbi.nih.gov) under the data base accession number NM_005163 (coding sequence) and NP_005154 (protein sequence). The genetic locus of Akt1 is on chromosome 14 q32.32-32.33., The protein and coding polynucleotide sequences of the Akt2 protein are known in the state of the art and available from the NCBI (National Centre for Biotechnology Information; National Library of Medicine, Building 38A, Bethesda, MD20894, USA; www.ncbi.nih.gov) under the data base accession number NM_001626 (coding sequence) and NP_001617 (protein sequence). The genomic sequence is located on chromosome 19 q13.1-13.2. The protein and coding polynucleotide sequences of the Akt3 protein are known in the state of the art and available from the NCBI (National Centre for Biotechnology Information; National Library of Medicine, Building 38A, Bethesda, MD20894, USA; www.ncbi.nih.gov) under the data base accession numbers NM_005465 (coding sequence) and NP_005456 (protein sequence) (isoform 1) and NM_181690 (coding sequence) and NP_859029 (protein sequence) (isoform 2). The genomic sequence is located on chromosome 1 q43-44.

Akt possesses a protein domain known as a PH domain, or Pleckstrin Homology domain. This domain binds to phosphoinositides with high affinity. In the case of the PH domain of Akt, it binds either phosphatidylinositol (3,4,5)-trisphosphate or phosphatidylinositol (3,4)-bisphosphate. Once correctly positioned in the membrane via binding of PIP3, Akt can then be phosphorylated by its activating kinases, phosphoinositide dependent kinase 1 (PDPK1 at threonine 308) and mTORC2 (at serine 473). First, the mammalian target of rapamycin complex 2 (mTORC2); mTORC2 therefore functionally acts as the long-sought PDK2 molecule, although other molecules, including Integrin-Linked Kinase (ILK) and Mitogen-Activated Protein Kinase Activated Protein Kinase-2 (MAPKAPK2) can also serve as PDK2. Phosphorylation by mTORC2 stimulates the subsequent phosphorylation of Akt by PDK1. Activated Akt can then go on to activate or deactivate its myriad substrates via its kinase activity.

Akt regulates cellular survival (Ramaswamy et al.; 1999) and metabolism by binding and regulating many downstream effectors, e.g. Nuclear Factor-κB, Bcl-2 family proteins and murine double minute 2 (MDM2).

Akt could promote growth factor-mediated cell survival both directly and indirectly. BAD is a pro-apoptotic protein of the Bcl-2 family. Akt could phosphorylate BAD on Ser136 (BAD phosphorylation by Akt), which makes BAD dissociate from the Bcl-2/Bcl-X complex and lose the pro-apoptotic function (BAD interaction with Bcl-2). Akt could also activate NF-κB via regulating IκB kinase (IKK), thus result in transcription of pro-survival genes (regulation of NF-kB).

The IκB kinase (IKK) enzyme complex is part of the upstream NF-κB signal transduction cascade. The IκBα (inhibitor of kappa B) protein inactivates the NF-κB transcription factor by masking the nuclear localization signals (NLS) of NF-κB proteins and keeping them sequestered in an inactive state in the cytoplasm (Jacobs et al.; 1998; Régnier et al.; 1997; Mercurio et.; 1997). IKK specifically phosphorylates the inhibitory IκBα protein (Karin, 1999). This phosphorylation results in the dissociation of IκBα from NF-κB and thereby activates NF-κB.

The IκB kinase complex is comprised of three subunits each encoded by a separate gene:

IKKα (also known as IKK1), IKKβ (also known as IKK2) and IKKγ (also known as NEMO). The α- and β-subunits together are catalytically active whereas the γ-subunit serves a regulatory function.

The protein and coding polynucleotide sequences of the IKK1 protein are known in the state of the art. They are available from the NCBI (National Centre for Biotechnology Information; National Library of Medicine, Building 38A, Bethesda, MD20894, USA; www.ncbi.nih.gov), for example, under the data base accession number NM_001278 (coding sequence) and NP_001269 (protein sequence). The protein sequence is also available under 015111. The genetic locus of IKK1 is on chromosome 10 q24 - q25. The protein and coding polynucleotide sequences of the IKK2 protein are known in the state of the art and available from the NCBI (National Centre for Biotechnology Information; National Library of Medicine, Building 38A, Bethesda, MD20894, USA; www.ncbi.nih.gov) under the data base accession number NBM_001556 (coding sequence) and NP_001547 (protein sequence). The protein sequence is also available under 014920. The genomic sequence is located on chromosome 8 p11.2. The protein and coding polynucleotide sequences of the NEMO protein are known in the state of the art and available from the NCBI (National Centre for Biotechnology Information; National Library of Medicine, Building 38A, Bethesda, MD20894, USA; www.ncbi.nih.gov) under the data base accession number NM_003639 (coding sequence) and NP_003630. The protein sequence is also available under Q9Y6K9. The genomic sequence is located on chromosome X q28.

NF-κB (nuclear factor kappa-light-chain-enhancer of activated B cells) is a protein complex that acts as transcription factor. NF-κB is found in almost all animal cell types and is involved in cellular responses to stimuli such as stress, cytokines, free radicals, ultraviolet irradiation, oxidized LDL, and bacterial or viral antigens (Gilmore, 2006; Brasier; 2006; Perkins, 2007; Gilmore. 1999; Tian and Brasier, 2003). NF-κB plays a key role in regulating the immune response to infection. Consistent with this role, incorrect regulation of NF-κB has been linked to cancer, inflammatory and autoimmune diseases, septic shock, viral infection, and improper immune development. NF-κB has also been implicated in processes of synaptic plasticity and memory (Albensi and Mattson, 2000). NF-κB family members share structural homology with the retroviral oncoprotein v-Rel, resulting in their classification as NF-κB/Rel proteins (Gilmore, 2006). There are five proteins in the mammalian NF-κB family (Nabel and Verma, 1993):

Class I: NF-κB1 (also called p105→p50) - NFKB and NF-κB2 (also called p100→p52) - NFKB2 and Class II: RelA (also named p65) - RELA, RelB - RELB and c-Rel - REL.

The protein, genomic and coding polynucleotide sequences are known in the state of the art and are e. g. publicly available from the NCBI (National Centre for Biotechnology Information; National Library of Medicine, Building 38A, Bethesda, MD20894, USA; www.ncbi.nih.gov), for example, under the data base accession numbers NM_003998 (coding sequence) and NP_003989 (protein sequence) for NFKB1, the accession numbers NM_002502 (coding sequence) and NP_002493 (protein sequence) for NFKB2, the accession numbers NM_021975 (coding sequence) and NP_068810 (protein sequence) for RELA, the accession numbers NM_006509 (coding sequence) and NP_006500 (protein sequence) for RELB and the accession numbers NM_002908 (coding sequence) and NP_002899 (protein sequence) for REL. See also the protein sequences P19838 for NFKB1, Q00653 for NFKB2, Q04206 for RELA, Q01201 for RELB and Q04864 for REL.

In addition, there are NF-κB proteins in invertebrates, such as the fruit fly Drosophila, sea urchins, sea anemones, and sponges (Ghosh et al., 1998).

All proteins of the NF-κB family share a Rel homology domain in their N-terminus. A subfamily of NF-κB proteins, including RelA, RelB, and c-Rel, have a transactivation domain in their C-termini. In contrast, the NF-κB 1 and NF-κB2 proteins are synthesized as large precursors, p105, and p100, which undergo processing to generate the mature NF-κB subunits, p50 and p52, respectively. The processing of p105 and p100 is mediated by the ubiquitin/proteasome pathway and involves selective degradation of their C-terminal region containing ankyrin repeats. Whereas the generation of p52 from p100 is a tightly-regulated process, p50 is produced from constitutive processing of p105 (Karin and Ben-Neriah, 2000; Senftleben et al., 2001).

Part of NF-κB's importance in regulating cellular responses is that it belongs to the category of "rapid-acting" primary transcription factors, i.e., transcription factors that are present in cells in an inactive state and do not require new protein synthesis to be activated (other members of this family include transcription factors such as c-Jun, STATs, and nuclear hormone receptors). This allows NF-κB to act as a "first responder" to harmful cellular stimuli. Stimulation of a wide variety of cell-surface receptors, such as RANK, TNFR, leads directly to NF-κB activation and fairly rapid changes in gene expression (Gilmore, 2006).

The Signal Transducers and Activator of Transcription (STAT, also called signal transduction and transcription) proteins regulate many aspects of cell growth, survival and differentiation. The transcription factors of this family are activated by the Janus Kinase Jak and dysregulation of this pathway is frequently observed in primary tumors and leads to increased angiogenesis, enhanced survival of tumors and immunosuppression.

The seven mammalian STAT family members identified are: STAT1, STAT2, STAT3, STAT4, STAT5 (STAT5A and STAT5B), and STAT6.

The protein, genomic and coding polynucleotide sequences are known in the state of the art and are e. g. publicly available from the NCBI (National Centre for Biotechnology Information; National Library of Medicine, Building 38A, Bethesda, MD20894, USA; www.ncbi.nih.gov), for example, under the data base accession numbers NM_007315 (coding sequence) and NP_009330 (protein sequence) for STAT1, transkript variant alpha, the accession numbers NM_139266 (coding sequence) and NP_644671 (protein sequence) for STAT1, transkript variant beta, the accession numbers NM_005419 (coding sequence) and NP_005410 (protein sequence) and the accession numbers AB384838 (coding sequence) and BAG 10694 (protein sequence) for STAT2, the accession numbers NM_139276 (coding sequence) and NP_644805 (protein sequence) and the accession numbers AB385032 (coding sequence) and BAG10888 (protein sequence) for STAT3, the accession numbers NM_003151 (coding sequence) and NP_003142 (protein sequence) and the accession numbers BC031212 (coding sequence) and AAH31212(protein sequence) for STAT4, the accession numbers NM_003152 (coding sequence) and NP_003143 (protein sequence) for STAT5A, the accession numbers NM_012448 (coding sequence) and NP_36580 (protein sequence) and the accession numbers AB384774 (coding sequence) and BAG10630 (protein sequence) for STAT5B, and the accession numbers AB385006 (coding sequence) and BAG 10862 (protein sequence) for STAT6.

STAT1 homodimers are involved in type II interferon signalling, and binds to the GAS (Interferon-Gamma Activated Sequence) promoter to induce expression of ISG (Interferon Stimulated Genes). In type I interferon signaling, STAT1-STAT2 heterodimer combines with IRF9 (Interferon Response Factor) to form ISGF3 (Interferon Stimulated Gene Factor), which binds to the ISRE (Interferon Stimulated Response Element) promoter to induce ISG expression.

STAT proteins were originally described as latent cytoplasmic transcription factors that require phosphorylation for nuclear retention. The unphosphorylated STAT proteins shuttle between cytosol and the nucleus waiting for its activation signal. Once the activated transcription factor reaches the nucleus it binds to consensus DNA-recognition motif called gamma activated sites (GAS) in the promoter region of cytokine inducible genes and activates transcription of these genes.

Extracellular binding of cytokines induces activation of the intracellular Janus kinase that phosphorylates a specific tyrosine residue in the STAT protein which promotes the dimerization of STAT monomers via their SH2 domain. The phosphorylated dimer is then actively transported in the nucleus via importin a/b and RanGDP complex. Once inside the nucleus the active STAT dimer binds to cytokine inducible promoter regions of genes containing gamma activated site (GAS) motif and activate transcription of these genes. The STAT protein can be dephosphorylated by nuclear phosphatases which leads to inactivation of STAT and the transcription factor becomes transported out of the nucleus by exportin crm1/RanGTP.

The JAK-STAT signaling pathway takes part in the regulation of cellular responses to cytokines and growth factors. Employing Janus kinases (Jaks) and Signal Transducers and Activators of Transcription (STATs), the pathway transduces the signal carried by these extracellular polypeptides to the cell nucleus, where activated STAT proteins modify gene expression. STATs were originally discovered as targets of Janus kinases.

Assays for the analysis of the signaling cascade associated with receptors comprising gp130and or LIFRα and for the detection of components being part of the signaling cascade can be found below in the experimental part. In particular, suitable assays for detecting a component in the signal cascade of the protein comprising gp130 and/or LIFRα are found under the heading "Signalling cascades in primary cortical neurons", "Akt kinase assay" and "AG490 kinase inhibitor experiment". Although these experiments have been specifically described for testing EPO and EPO variants it is obvious to those skilled in the art that any compound which is to be identified as a cytoprotective compound can be used in these tests instead of EPO or the EPO variants. Moreover, although in the tests the use of specific antibodies directed against Bad, phospho-Bad Ser 136, phospho-Bad Ser 112, phosphor-pIKK, phospho-Jak2, phospho-Jak1, phospho-Stat5 and jak2 is disclosed, antibodies directed against further components of the gp130/LIFRα signaling cascade are known and available and can be used for detection of those components.

Other approaches of detecting a component being involved in the signaling cascade downstream the gp130 and/or LIFRα comprising receptor may use polynucleotide fragments of polynucleotides encoding such a component and comprising at least 10 nucleotides capable of hybridisation under stringent conditions. The skilled person will be able to design suitable ologonucleotide probes or primers that specifically hybridise under stringent conditions as defined herein to nucleic acid (RNA or DNA) encoding downstream components, thus detecting the presence of transcripts and consequently of proteins of the downstream signaling cascade of the pg130/LIFRα receptor.

In a further embodiment, the identification of a potential cytoprotective compound as a cytoprotective compound occurs via the detection of the binding of the compound to gp130 and/or LIFRα. As could be shown by the present inventors, binding of cytoprotective compounds to members of receptor comprising gp130 and/or LIFRα is involved in cyto/neuroprotection by using the signaling cascade involving in particular Janus kinase 2 (Jak2), Janus kinase 1 (Jak1), Janus kinase 3 (Jak3), protein kinase B (Akt), IκB kinase (IKK), nuclear factor-kappa B (NF-κB) and/or the family of signal tranducers and activators of transcription (STAT).

Suitable assays for detecting the binding of a compound to another compound are known in the art and are, for example, displacement assays of a radioactively labeled ligand from its receptor by the binding of a test compound (radioactive displacement, see e.g. Examples I.17 and I.18) or the M 1 proliferation assay in combination with the MTT assay, in which the capability of the M1 cell line to differentiate into mature macrophages and granulocytes is tested in the presence of test compounds. Suppression of proliferation and differentiation of the M1 cell line indicates the binding of potential cytoprotective compounds to gp130 and/or LIFRα and the activation of the downstream signaling cascade. Further tests include pulldown assays in which prey proteins containing cell lysates or culture supernatants are tested for their binding to immobilized bait proteins being the potential cytoprotective compounds to be identified by the methods of this invention. All these tests are described in the experimental part of the present specification. It is thereby obvious to those of skill in the art that the tests can be adapted to the identification of compounds as cytoprotective compounds. Moeover, the detection of compounds involved in the signaling cascade downstreamof a gp130/LIFRa receptor also serves as evidence for the binding of a potential cytoprotective compound to gp130 and/or LIFRα. Such tests for the detection of such components are described in the present invention.

In a further embodiment the protein or protein complex is a naturally occurring receptor; such as such as LIFR, CNTFR or CT-1R.

In a still further embodiment the identifying as a (potential) cytoprotective compound is by detecting binding of the compound to the protein or protein complex, particularly to gp130 and/or LIFRα. The identifying may be carried out as detailed above.

In a further embodiment, the compound to be identified by the present invention is neuroprotective and/or modulates differentiation in the neuronal/glial lineage. Neuroprotection may be determined as described in Examples I. 9/10.

A neuroprotective compound is a compound which protects the neurological system from damage or which helps to limit the damage suffered by a nerve or neural tissue such as spinal cord, brain or nerve, when the blood supply is cut off or there is a traumatic injury. The capability of neuroprotection of a potential neuroprotective compound can be tested in an in vitro model of cerebral ischemia using rat primary cortical neurons as described below with respect to neuroprotection of EPO and EPO variants. It is obvious to the skilled person that the assay can be adapted to the identification of other potential neuroprotective compounds by replacing EPO or EPO variants by those compounds (cf. Examples I.9/10).

The term "modulates" in the context of differentiation within the present invention may be an enhancement, a diminishing or prevention or total inhibition of differentiation and is preferably an enhancement of differentiation.

In a further embodiment, the compound is not hematopoietic, i.e. the compound may be any compound that does not induce the formation of blood cellular components. Thus, the compound may be any type of compound and encompasses numerous chemical classes as generally described above for cytoprotective compounds, as long as it is able to bind to gp130 and/or LIFRα and mediates cytoprotection via the signaling cascade involving gp130 and/or LIFRα or a complex comprising gp130 and/or LIFRα and as long as the compound is not hematopoietic such as EPO. Preferably, absence of hematopoietic activity compound is defined as increasing hematopoiesis 1 by at most 25 %, more preferably at most 10 %, still more preferably at most 5 %, preferably at least if compared to a control in the absence of a hematopoiesis-inducing agent. An exemplary in vivo and in vivo test for hematopoietic activity is given in Examples 1.7 and 1.8, respectively.

In a still further embodiment, the protein comprising gp130 and/or LIFRα binds to an erythropoietin (EPO) variant (vEPO) and promotes the cytoprotective effects thereof.

EPO was first isolated from urine of anemic patients (Miyake et al., 1977). EPO gene expression was also detected in human and murine brain (Marti et al., 1996). The human protein and coding polynucleotide sequences of EPO are known in the state of the art and are e. g. publicly available from the NCBI under the data base accession number NM_000799 (coding sequence) and NP_000790 (protein sequence). They are also available under the accession numbers BC093628 (coding sequence) and AAH93628 (protein sequence), under the accession numbers BC111937 (coding sequence) and AAI11938 (protein sequence), under the accession numbers AF202308 (coding sequence) and AAF23132 (protein sequence), under the accession numbers AF202314 (coding sequence) and AF23134 (protein sequence) and under the accession numbers M11319 (coding sequence) and AAA52400 (protein sequence). The protein and coding polynucleotide sequences of Mus musculus are known in the state of the art and are e. g. publicly available from the NCBI under the data base accession number NM_007942 (coding sequence) and NP_031968 (protein sequence) and under the accession numbers BC 119265 (coding sequence) and AAI9266 (protein sequence).

The EPO used in the present invention is preferably a protein comprising or consisting of the sequence according to Fig. 5A (SEQ ID No 1 with or without leader). More preferably, variants may be used, e.g. those described herein. Specific embodiments of EPO variants useful in the present invention involve human EPO variants with a complete loss of exon 3 (denoted hS3), such as the nucleotide sequence as shown in Fig. 5A, human EPO variants with a partial loss of exon 4 (denoted hS4), such as the nucleotide sequence as shown in Fig. 5A, and the mouse EPO variant with a complete deletion of exon 4 (denoted mS), such as the nucleotide sequence as shown in Fig. 5A. Alternatively, the methods may be used in order to identify new cytoprotectibve EPO variants, preferably those without hematopoietic activity, by modifying EPO or the above EPO variants by substitutions, deletions, additions and/or insertions of amino acids by naturally or non-naturally occurring amino acids so far as they maintain the cytoprotective activity of the EPO parent compound, however, do not possess hematopoietic. Moreover, EPO can be modified by the addition of chemical groups as far as the modified EPO has the above properties. One method of preparing EPO variants is described in Example I.3.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, and materials are described herein.

The invention is further illustrated by the following figures and examples, although it will be understood that the figures and examples are included merely for purposes of illustration and are not intended to limit the scope of the invention unless otherwise specifically indicated.

## FIGURES

**Figure 1:** Alternatively spliced human and murine EPO transcripts

(A) Agarose gel stained with ethidium bromide showing products generated by PCR using primers flanking the human EPO mRNA. cDNA was prepared from human kidney poly $(A)^+$ RNA (Stratagene). Three products were amplified corresponding to hEPO (582 bp), the splicing variant hS3 (495 bp) and the splicing variant hS4 (486 bp).
(B) Nested PCR products generated from murine brain cDNA by primers flanking the murine EPO mRNA. The major product corresponds in size to the murine EPO transcript (mEPO clone). The faint second product coincides with the murine spliced EPO transcript (mS clone).
(C) Immunoprecipitation (IP) of mEPO and splice variant mS from murine brain, kidney and serum preparations. Protein extracts were generated from $CoCl_2$*treated mice showing increased EPO-levels and from native control mice (untreated). Western blot of proteins immunoprecipitated with the anti-mEPO antibody (R&D Systems). I: The blot was probed with an anti-rhEPO antibody (Santa-Cruz Biotechnologies). The band migrating at appoximately 38 kDa corresponding to mEPO was present in the brain, serum and kidney extracts of $CoCl_2$-treated mice, but not in the kidney extract from control mice. A second signal consistent in size with the recombinant splice variant mS (30 kDa) was detected in the kidney protein extract of $CoCl_2$-treated mice. II: The target specificity of the antibody was shown by probing the same blot with the anti-rhEPO preincubated with Darbepoietin.

(D) Multiple alignments of human and murine EPO transcripts. Dashes are incorporated to optimise alignment in spliced-out regions.

(E) Exon structure of alternative splice variants of human and murine EPO discovered by PCR. Splice variant hS3 of human EPO lacks exon 3. Human EPO splice variant hS4 lacks the N-terminal part of exon 4. The exclusive murine EPO splice variant mS lacks the entire exon 4. All exons deleted in the splice variants contain classical 5' donor (GU) and 3' acceptor splice sites (AG).

**Figure 2:** Hematopoiesis stimulating functions of EPO and the EPO splice variants

The hematopoiesis stimulating potentials of EPO and the EPO splice variants were evaluated using *in vitro* and *in vivo* methods.

(A+B) Methylcellulose colony-forming assays of bone marrow cells were performed in presence or absence of EPO and its variants. Bone marrow cells were grown in methylcellulose containing 200 U/1 of commercially available recombinant human EPO (Roche) or equivalent concentrations of EPO and EPO variants expressed in HEK 293 cells as indicated. Colony counts of erythroid colony forming units (cfu-e) were assessed on each individual sample at least three times and results are presented as average ±s.d. for colonies counted under each condition. Asterix: differences from rhEPO- or mEPO-treated plates in the same group (Mann-Whitney test, $p<0.05$).

(C+D) Male C57B16 mice were injected every two to three days (i.p.) with either saline, rhEPO (ERYPO from Janssen-Cilag, 42 μg/kg purified hEPO from CHO cells (42 μg/kg) or purified hS3 from CHO cells (42 μg/kg). Blood samples were collected 48 h after the third dose and hematocrit levels were analysed. Data are presented as means ±s.d (n = 5-10). Asterix: differences from saline-treated group (ANOVA 1, Bonferrroni, $p<0.05$).

**Figure 3:** Neuroprotection and cytoprotection mediated by EPO and EPO variants

(A) EPO and the EPO variants induce tolerance against OGD in rat primary cortical neurons. Primary cortical neurons were pretreated for 48 h with 100 U/1 commercial rhEPO (Roche) or equimolar concentrations of human EPO variants, respectively. In another set of experiments primary cortical neurons were pretreated for 48 h with 300 U/1 murine EPO or an equimolar concentration of the murine EPO variant mS. Recombinant human and murine EPO significantly protected primary cortical neurons from cell death induced by a 120 min OGD. Cell death was measured as LDH release for 24 h after OGD. Pretreatment with the human and murine EPO splice variants induced a more pronounced tolerance against OGD compared to rhEPO and mEPO. Data were obtained from three independent experiments. Data were normalized and presented as means ±s.d. Asterix: differences from MOCK treated controls (Mann-Whitney. $p<0.05$.)

(B) Dose-response curves illustrating the neuroprotective effect mediated by human EPO and its splice variants on primary cortical neurons. Primary cortical neurons were pretreated for 48 h with 0, 0.03, 0.3, 3, 8, 15, 30, 75 or 150 pM rhEPO (Roche), hEPO or the human EPO splice variants hS3 and hS4, respectively, as indicated on the horizontal axis. The vertical axis represents the protective effect from cell death induced by a 120 min OGD. The neuroprotective effect was evaluated as reduction in the 24 h lasting LDH release after OGD. Data obtained from 7 to 21 experiments were normalized and presented as average ±SEM. Asterix: differences from untreated controls (0 pM) (ANOVA 1, $p<0.05$)

(C) Human EPO and the human EPO splice variant hS3 significantly protected an embryonal rat heart-derived cell line from apoptotic cell death induced by oxygen and serum deprivation. H9c2 cultures were pretreated for 48 h with 90 pM hEPO or hS3, respectively. Thus, cardiac myocytes were incubated in serum-free medium in hypoxic condition for 24 h. Cell death was assessed as LDH release in the 24 h lasting reoxygenation period. Data were obtained from seven independent experiments. Data were normalized and plotted as means ±s.d. Asterix: differences from MOCK treated controls (ANOVA 1, $p<0.001$)

**Figure 4:** The human EPO splice variant hS3 uses the same signaling pathway as hEPO for mediating neuroprotection in rat primary cortical neurons

(A) Primary cortical neuronal cultures were starved in a balanced salt solution for 1 h and exposed for 15 min to hEPO (90 pM) or the splice variant hS3 (90 pM), respectively. Immunoblots of cell lysates were probed with antibodies specific to pJAKI, pJak2, Jak2, pIKK, pBAD (Serl36), pBAD (Ser 112) and BAD (representative of three independent experiments).

(B) Protein extracts were prepared from rat cortical neurons exposed for 30 min to rhEPO (30 pM), hEPO (30 pM) or hS3 (30 pM) and immunoprecipitated with a pan-Akt antibody. The immunoprecipitates were incubated for 30 min with the Akt Substrate GSK-3a/β and then immunoblotted for pGSK-3a/β.

(C) The JAK2-inhibitor AG-490 abolished hEPO and hS3 induced tolerance against OGD in rat primary cortical neurons. Primary cortical neurons were treated for 48 h with hEPO (30 pM) and hS3 (30 pM) in presence or absence of 5 μM AG490. Cell death was measured as LDH release for 24 h after a 120 min OGD. Data were obtained from nine independent experiments. Data were normalized and plotted as means ±s.d. Asterix: differences from control cultures (ANOVA1, Kruskal-Wallis, p<0.05)

**Figure 5:** Protein structure predictions of EPO and EPO variants

(A) Multiple protein sequence alignment of human and murine EPO and EPO splice variants. Residues corresponding to the leader sequence are highlighted.

(B) Schematic representation of human and murine EPO and EPO splice variants. Human and murine EPO consist of 4 helices A, B, C and D and a mini-helix B' important for receptor binding. Deletions in the splice variants are indicated as dotted regions.

(C) Visualisation of the tertiary protein structures of hEPO (1buy.pdb), hS3 and mS in AstexViewer ™. The structure prediction of hS3 is based on a hEPO-model with 1.90 Å resolution (1eerA.pdb) and has an E-value of 5.80704e-62 and a sequence identity of 80%. The structure prediction of mS is based on a model of mEPO with 2.80 Å resolution (1cn4C.pdb) and has an E-value of 3.01905e-29 and 48% sequence identity with the model.

**Figure 6:** EPO variants do not bind to the homodimeric $(EPOR)_2$

(A) Point mutations in the A-helix of hEPO at position 14 do not abolish A-helix mediated tolerance against OGD in primary cortical neurons. Arg 14 was shown to be important for the hematopoietic activity of hEPO (Wen et al., 1994). Primary cortical neurons were pretreated for 48 h with hEPO-derived A-helix (30 pM) or A-helix muteins MutE (30 pM, aa exchange to Glu) and Mut A (30pM, aa exchange to Ala), respectively. Primary cortical neurons were exposed to 120 min OGD and LDH release was measured 24 h after reoxygenation. Data were obtained from four independent experiments. Data were normalized and plotted as means ±s.d. Asterix: differences from control cultures (ANOVA1, Kruskal-Wallis, p<0.05)

(B) hEPO but not hS3 promotes cell survival in IL-3 deprived Ba/F3 cells stably transfected with EPOR (Ba/F3-EPOR). IL-3 dependent Ba/F3 and Ba/F3-EPOR cells were grown in the absence of IL-3 but in presence of hEPO (300 pM) or the human EPO splice variant hS3 (300 pM). Viability was assessed after 48 h. Data were obtained from three independent experiments and presented as means ±s.d. Asterix: differences from control cultures grown in presence of IL-3 (ANOVA 1, p<0.05).

(C) hEPO but not the human splice variants hS3 and hS4 displaces iodinated rhEPO from the homodimeric $(EPOR)_2$. Ba/F3 cells stably transfected with the EPOR were saturated with iodinated rhEPO (1251-rhEPO). Displacement using increasing concentrations of unlabeled hEPO and hEPO splice variants was conducted at 37°C. The plot is representative of three independent experiments. Absolute count numbers were normalized and presented as means ±s.d..

**Figure 7:** EPO variants can mediate neuroprotection through the LIF receptor family

(A) Pulldown experiment with GST-matmEPO and GST-matmS expressed in an *E.coli* strain bound to GST agarose. Lysates of the BAF3/EPOR cell lines were used as protein pool containing possible interaction partners. Analysis of pulldown eluates on Western Blots with the anti-EPOR antibody from Santa-Cruz.

(B-D) Pulldown experiment with GST-tagged *E.coli* proteins of matmE, matmS and mathS3. strain bound to GST agarose. Prey proteins were obtained from lysates of rat cortex neurons (B), supernatants of HEK 293 cultures expressing and secreting V5-His-tagged murine soluble LIFR (C) and lysates from HEK 293cells (D). Analysis of pulldown eluates on Western Blots with the anti-LIFR antibody from Santa-Cruz (B+D) and the anti-V5 antibody from Serotec (C).

(E) Primary cortical neurons from rat were pretreated with 30 pM hEPO or 30 pM hS3. Sister cultures were pretreated with hEPO or hS3 medium that was preincubated for 1 h with soluble receptors sEPOR and sLIFR. LDH releases measured 24 h after OGD are plotted as % to control. The error bars are indicating the range of standard deviations in five independent experiments. Statistical analysis using ANOVA1. *p<0.05.

(F) Influence of anti-LIFRα and anti-gp130 antibodies on neuroprotection mediated by hEPO and hS3. LDH releases measured 24 h after OGD are plotted as % to control. The error bars are indicating the range of standard deviations in six independent experiments. Statistical analysis using ANOVA1. *p<0.05.

As shown in (A), murine EPO but not its splice variant interacts with EPOR as shown by GST pull-down-assay. GST-fusion proteins of the mature forms of mEPO (matmE) and mS (matmS) were used to pull-down interacting proteins from Ba/F3 cell lysates stably transfected with EPOR. Protein complexes were analysed by Western Blotting using an anti-EPOR antibody. Pull-down assays of (B-D) show interaction of the murine and human EPO splice variants with LIFRα species. Crude protein extracts, prepared from primary cortical neurons (B) or HEK 293 cells (D), or supernatants of HEK 293 cultures expressing V5-His~tagged murine soluble LIFRα (C), were incubated with GST-fusion proteins matmE, matmS or mathS3, respectively. Protein complexes were resolved on SDS-PAGE and immunoblotted with anti-LIFR antibody (B+D) and anti-V5 antibody (C). As shown in (E), the soluble LIF receptor abolishes hS3 but not hEPO mediated tolerance against OGD in primary cortical neurons. Primary cortical neurons were treated for 48 h with hEPO (30 pM) or hS3 (30 pM), respectively, before exposure to 120 min OGD. OGD-induced cell death was evaluated by 24 h LDH release into the culture medium. Coapplication of soluble receptor EPOR (sEPOR) completely abrogated hEPO but not hS3 mediated neuroprotection. However, coapplication of soluble receptor LIFR (sLIFR) abolished neuroprotective actions of hS3 but not of hEPO. Data were obtained from five independent experiments. Data were normalized and plotted as means ±s.d. Asterix: differences between treatment groups as indicated (ANOVA1, $p<0.05$). As shown in (F), neuroprotection conferred by hS3 but not by hEPO was attenuated by coapplication of anti-LIFRα and anti-gp130 blocking antibodies. Primary cortical neurons were pre-incubated with anti-LIFRα or anti-gp130 blocking antibodies for 1 h before application of hEPO (30 pM) or hS3 (30 pM), respectively. 120 min OGD was induced after 48 h treatment and cell death was evaluated by mean of LDH release 24 h after reoxygenation. Data were obtained from seven independent experiments. Data were normalized and plotted as means ±s.d. Asterix: differences to control cultures (ANOVA1, $p<0.05$).

**Figure 8:** The vEPO are probably not binding the 'classical' LIFR receptor composed of LIFRα/gp130

(A) Protocol of the radioactive displacement assay on transfected and native Ba/F3-cells. 4 Mio cells were preincubated with the inhibitor of transglutaminase activity MDC before saturation with radioactive labelled mLIF. Unlabelled mLIF or vEPO were added in different concentrations to test for competition. Radioactivity bound to the cells was counted after 1.5 h at RT.

(B) Unspecific binding of $^{125}$I-mLIF to Ba/F3 cells was determined by incubating GFP-transfected control cells with radioactivelly labelled mLIF. 20 % of the counts were found to be based on unspecific binding. Specific binding of mLIF was determined by measuring the amount of $^{125}$I-mLIF bound to Ba/F3-cells expressing gp130 and LIFRα in presence and absence of unlabeled mLIF. Statistical analysis using t-test. *$p<0.001$.

(C) mLIF but not the human recombinant splice variant hS3 produced in HEK293 cells displaces iodinated mLIF from the heterodimeric LIF receptor. Ba/F3 cells transiently transfected with gp130 and LIFRα were saturated with iodinated mLIF ($^{125}$I-mLIF). Displacement using unlabeled hS3 and mLIF was conducted at RT. The plot is representative of four independent experiments. Purified recombinant hS3 was used from different batches. Thus, the final concentration varied between 12 and 60μ g. Absolute count numbers were normalized and presented as means ±s.d. Statistical analysis using ANOVA1. *$p<0.05$.

(D) mLIF but none of the GST fusion proteins mathS3, matmS or matmE (0.5 - 2 μg) expressed in *E.coli* displaced iodinated mLIF from the heterodimeric LIF receptor.

As described previously, Ba/F3 cells transiently transfected with gp130 and LIFRα were saturated with iodinated mLIF and displacement with the unlabeled proteins was conducted at RT. The plot is representative of two independent experiments. Absolute count numbers were normalized and presented as means ±s.d. Statistical analysis using t-test. *$p<0.01$.

**Figure 9:** The vEPO are mediating a mild LIF-like effect potentially through another member of the gp130-family

IL-6, LIF, OSM, interleukin 11 (IL-11) and ciliary neurotrophic factor (CNTF) bind to composite receptors containing a common signal transducer gp130. These six factors show some *in vitro* biological activities in common such as induction of differentiation and growth arrest of the murine M1 myeloid leukemic cell line. Same seems to be true for hS3.

Mouse myeloid leukemia (M1) cells were seeded at the density of 150,000 cells/ml in presence or absence of 15 nM hEPO, hS3 or equivalent volumes of MOCK-medium or in presence or absence of 500 nM peptide P16 in 96-well plates. According to the experimental conditions murine LIF was added at a final concentration of 50 ng/ml. Proliferation of the cultures was measured by mean of the MTT-test 24 h (1), 48 h (2) and 72 h (3) after seeding. Mean proliferation rates in control conditions (=MOCK, w/o mLIF) were considered 100%. Experiments were repeated three times with 6 wells per condition and data was plotted + s.d.

A: M1 cells were grown in absence of murine LIF. In three independent experiments there was a weak but significant decrease in proliferation 72 h after seeding in presence of the splice variant hS3. Asterix: differences to MOCK-treatment ($p^* < 0.05$, ANOVA1, Bonferroni t-test).
B: M1 cells were grown in presence of 50 ng/ml murine LIF and EPO variants. mLIF was a potent inhibitor of M1 proliferation. No synergistic effects from EPO variants were observed. (no statistical significance, ANOVA1).

**Figure 10:** The vEPO receptor is composed of more than 2 chains
Crosslinking of vEPO expressed as GST-fusion proteins on H9c2 cells and murine NSC reveals more than two interaction partners suggesting a minimum trimeric heteroreceptor.

(A) Crosslinking of mathS3 and matmS on H9c2 cells. Signals were detected using the α-rhEPO antibody from Santa-Cruz.
(B) Crosslinking of mathS3 and matmS on murine NSC. Signals were detected using the α-rhEPO antibody from Santa-Cruz.
(C) Immunoprecipitations were performed on lysates of NSC after crosslinking of mathS3 and matmS. The α-mEPO antibody from R&D was used for immunoprecipitation, signals were detected using the α-rhEPO antibody from Santa-Cruz.

## EXAMPLES

### I. Experimental procedures

1. PCR isolation and cloning of novel alternatively spliced transcripts of murine and human EPO

Total RNA from kidney and brain of C57BL/6 mice (Charles River Laboratories) and SV129/S6 mice (Bundesinstitut für Risikobewertung, Berlin) was extracted according to the Trizol Reagent protocol from Invitrogen. Human adult kidney (male) and fetal brain (male) poly A+ RNA was purchased from Stratagene. For cDNA synthesis 3 μg RNA and 3 μl random hexamer primers (10 μM) in a final volume of 15 μl of DEPC-treated water were heated to 70°C for 10 min. Components added to the reaction mix included 6 μl of 5x reaction buffer, 2 μl of 2.5 mM dNTPs, 1 μl of RNase inhibitor (1 U/μl) and 1 μl of Moloney murine leukemia virus (MMLV) RNase H-reverse transcriptase (Promega). The RT reaction was incubated for 1 h at 37°C.

The open-reading frame of murine EPO was isolated by a nested RT-PCR approach. cDNA amplification was performed in a 100 μl reaction volume using Pfu Turbo Hotstart Polymerase (Stratagene) according to the manufacturer's protocol. Primers (MWG-Biotech AG) used in the first round of PCR corresponded to sequences in the 5'UTR and 3'UTR of the mEPO mRNA (5'-GAA CTT CCA AGG ATG AAG ACT TGC AGC-3', 5'-GTG GCA GCA GCA TGT CAC CTG TC-3' SEQ ID No 6). A second DNA amplification was set up in fresh tubes using inner nested primers encompassing the start-codon and the stopcodon of mEPO (5'-TAT GGA TCC ATG GGG GTG CCC GAA CGT CCC AC-3' SEQ ID No 7, 5'-TAT GGA TCC TCA CCT GTC CCC TCT CCT GCA GAC-3' SEQ ID No 8) and 2 μl of the PCR product obtained in the first PCR round. The open-reading frame of human EPO was amplified by PCR using a primer pair corresponding to the coding region of hEPO (5'-GAT GGG GGT GCA CGA ATG TCC TGC-3' SEQ ID No 9, 5'-CAC ACC TGG TCA TCT GTC CCC TGT C-3' SEQ ID No 10). After a hot start at 95°C for 3 minutes, the DNA PCR reactions were performed at 95°C for 30 seconds, 65 to 70°C for 30 seconds depending on the annealing temperature of the primer pair, and 72°C for 60 seconds for 20 to 35 cycles, with a final extension time of 10 minutes at 72°C.

The PCR products were separated on ethidium bromide-stained 1.2% agarose gels and isolated using the Gel Extraction Kit from Qiagen. All PCR products were subcloned into the pCR-Blunt II-TOPO Vector (Invitrogen) and were sequenced at both strands.
The Access-RT-PCR System from Promega, used as alternative PCR method, was performed according to the manufacturer's instructions.

2. Immunoprecipitation of EPO and EPQ variants from murine tissues

Male 129S6 mice or C57B16 mice (8-10 weeks, Bundesinstituts für Risikobewertung, Berlin) were subcutanely injected with 60 mg/kg $CoCl_2$ (Sigma-Aldrich) and sacrificed after 18 hours. Organs were instantly removed and homogenized in NP-40 lysis-buffer (50 mM HEPES-KOH pH 7.4, 10% glycerol, 100 mM NaCl, 1% Nonidet P-40 (NP-40), 200 U/1 Benzonase (Sigma-Aldrich), 1.5 mM $MgCl_2$, 1x Protease-Inhibitor-Cocktail (Roche)). Serum was prepared from blood collected from the heart of anesthesized mice. Erythropoietin concentrations in serum, kidney extracts and brain extracts were measured in a commercial ELISA assay for murine EPO (R&D Systems) according to supplier's protocol. Immunoprecipitations were performed over-night at 4°C with 0.5 μg anti-mEPO antibody (R&D Systems). Antibody-protein complexes were captured by incubation with streptavidin-agarose (Thermo-Scientific) for 1 h at room temperature. After thorough washing of the agarose with PBS (Biochrom) proteins were released by boiling in sample buffer (125 mM Tris-HCl (pH 6.8), 1% SDS, 20% glycerol, 10% β-Mercaptoethanol, 0.008% bromophenol blue). Eluates were separated on polyacrylamide gels and analysed on Western blots (1:500, anti-rhEPO, H-162, Santa-Cruz). To show specificity of the western blot signals we preincubated the primary antibody with 10 μg of the recombinant human erythropoietin DarbepoetinA (Amgen Europe, Netherlands) for 2 h at room temperature prior to blot incubation.

3. Generation of A-helix muteins

The hEPO A-helix motif was amplified from the hEPO cDNA template with forward primer A (5'-TAT GGA TCC ATG GGG GTGCAC GAA TGT-3' SEQ ID No 11) containing a *Bam*HI site and reverse primer B (5'-CAA GAT ATC TCA CGT GAT ATT CTC GGC CTC C-3' SEQ ID No 12) containing an *Eco*RV site and stop codon. The PCR product was purified as described previously and introduced into the pcDNA3.1/V5-His vector from Invitrogen using *Bam*HI and *Eco*RV restriction sites leading to the pcDNA3.1-hEPO-HelixA construct. Human EPO A-helix muteins were generated by site-directed mutagenesis using pcDNA3.1-hEPO-HelixA as template. Amino acid changes at Arg14 were introduced by combination of two separate PCR reactions for each mutein. Mismatch primers were designed to change hEPO A-helix cDNA sequence from AGG to GCG for mutant A (mutA_for: 5'-AGT CCT GGA GGC GTA CCT C-3' SEQ ID No 13, mutA_rev: 5'-GAG GTA CGC CTC CAG GAC T-3' SEQ ID No 14) or from AGG to GAG for mutant E (mutE_for: 5'-AGT CCT GGA GGC GTA CCT C-3' SEQ ID No 15, mutE_rev' 5'-GAG GTA CTC CTC CAG GAC T-3' SEQ ID No 16). The first round PCRs were performed using the flanking primers A or B and the appropriate mismatch primers. The products from the first PCR reactions were used in equal amounts as templates for the second PCR using the flanking primers A and B. PCR products were introduced into pcDNA3.1/V5-His vector leading to pcDNA3.1-hEPO-mutA and pcDNA3.1-hEPO-mutE constructs. All PCR reactions were conducted with Pfu Turbo Hotstart Polymerase (Stratagene) as described previously.

4. Construction and Expression of His-tagged proteins

DNAs (cDNAs) encoding human and murine EPO and EPO splice variant were amplified from appropriate pCR-Blunt II-TOPO clones using P*fu* polymerase (Stratagene) and primers that generated 5' *Bam*HI and 3' *Eco*RI cloning sites and a deletion of the EPO stop codon (murine primers: 5'-TAT GGA TCC ATG GGG GTG CCC GAA CGT CCC AC-3' SEQ ID No 17, 5'-TCG GAA TTC TCA CCT GTC CCC TCT CCT GCA GAC-3' SEQ ID No 18; human primers: 5'-TAT GGA TCC ATG GGG GTGCAC GAA TGT-3' SEQ ID No 19, 5'-AGA GAA TTC TCT GTC CCC TGT CCT GCA-3' SEQ ID No 20). The PCR products were agarose gel purified, digested with restriction enzymes, and subcloned into pcDNA3.1/V5-His (Invitrogen) to create fusion constructs encoding EPO and EPO splice variant fusion proteins with a V5-polyhistidine epitope at the C-terminus. Human EPO and human hS3 splice variant were further subcloned into the pVITRO4 plasmid from InvivoGen. cDNAs encoding the hEPO-V5-His cassette and the hS3-V5-His cassettes were amplified from pcDNA3.1-V5/His-hEPO and pcDNA3.1-V5/His-hS3, respectively, using the following primers: 5'-AAC CAT GGG GGT GCA CGA ATG TCC TGC CTG-3' SEQ ID No 21, 5'-AAG GAT CCT CAA TGG TGA TGG TGA TGA TGA CCG GTA C-3' SEQ ID No 22. The resulting PCR products were agarose gel purified and cloned in sense orientation into pVITRO4 via *Nco*I and *Bam*HI restriction sites.

5. Cell lines and transfections

Human embryonic kidney cells (HEK293, BD Biosciences) were grown in DMEM (Biochrom, 1 g/l glucose) containing 10% FCS in tissue culture flasks at 37°C and 5% CO2. For subcultivation cells were split every 2 to 3 days after reaching 80 - 90% confluence. Freestyle HEK293 cells were grown as agitated suspension cultures in Freestyle293 Expression Medium at 37°C and 8% CO2 on an orbital shaker platform rotating at 125 rpm. Cells were subcultured every 2 to 3 days in disposable sterile Erlenmeyer flasks (Coming). Chinese Hamster Ovary cells adapted to serum-free medium (CHO-S, Gibco) were grown as agitated suspension culture in a chemically-defined, protein-free CD-CHO medium (Gibco) supplemented with 100 μM hypoxanthine (Gibco), 16 μM thymidine (Gibco) and 8 mM L-glutamine (Biochrom) under same $CO_2$- and shaking conditions as Freestyle HEK293 cells.

Adherent HEK293 cells (BD Biosciences) were transiently transfected with plasmid vectors that express hEPO-Helix A and hEPO-helix A muteins. Transfection procedure was based on the protocol of Lipofectamine 2000 (Invitrogen). Cells (80% confluent) grown in six-well plates were first overlaid with 2 ml per well of serum- and antibiotics-free DMEM medium for 20 min and then incubated for 4-6 h with the DNA-Lipofectamine 2000 complexes (500 μl per well). After transfection the mix was removed and replaced by 2 ml of growth medium. Culture supernatants were harvested after 48 h and used for preconditioning experiments.

The His-tagged recombinant human and murine erythropoietin variants were expressed in Freestyle HEK293 cells for *in vitro* assays and in CHO-S cells for *in vivo* experiments. Transfection of Freestyle293 cells with pcDNA3.1-V5/His-constructs and pVITRO4-V5/His-constructs was performed according to manufacturer's protocol using 293fectin™ (Invitrogen). CHO-S cells were transiently transfected with pVITRO4-constructs using Fecturin™ as indicated by the supplier (Polyplus transfection). Medium from suspension HEK293 and CHO cultures was harvested after 2-3 days. Cells were pelleted by centrifugation at 1200 rpm and supernatant was further concentrated by using Vivapore 10/20 ultrafiltration systems (Sartorius AG) at 4˚C over-night. His-tagged proteins were purified with BD TALON™ Metal Affinity Resin (BD Biosciences) from concentrated cell culture supernatants according to the protocol provided by the supplier. Imidazole (Sigma-Aldrich) contained in the elution buffer was removed by dialysis using Slide-A-Lyzer Dialysis Cassettes from Pierce (ThermoFisher Scientific).

6. Pulldown assay

For the generation of glutathione S-transferase (GST) fusion constructs of mature EPO and EPO splice variants lacking the N-terminal secretion signal peptide the fragments were amplified by polymerase chain reaction using the following primers for matmEPO and matmS: 5'-TAT GGA TCC GCT CCC CCA CGC CTC ATC TGC-3' SEQ ID No 23, 5'-TCG GAA TTC TCA CCT GTC CCC TCT CCT GCA GAC-3' SEQ ID No 24 and the following primers for mathS3: 5'-TAT GGA TCC GCC CCA CCA CGC CTC ATC TGT - 3' SEQ ID No 25, 5'-TCG GAA TTC TCA TCT GTC CCC TGT CCT GCA GG-3' SEQ ID No 26. The polymerase chain reaction products were cleaved with *Bam*HI and *Eco*RI and inserted into the same sites of pGEX-6P-1 (GE Healthcare) in frame with the GST-tag to generate pGEX-6P1-matmE, pGEX-6P1matmS and pGEX-6P1-mathS3. Competent BL-21-RIL bacteria suitable for high efficiency protein expression were transformed with plasmid DNA. Cultures transformed with empty pGEX-6P1 were used as MOCK-control. Ampicillin and chloramphenicol containing LB-medium was inoculated with over-night cultures of transformed bacteria and grown at 37˚C to an optical density OD600 of 0.6.

Protein expression was induced with 1 mM IPTG (Sigma-Aldrich) and the cultures were grown an additional 3-4 hours at 37˚C. Cells were pelleted for 20 min at 2500 rpm and 4˚C and resuspended in PBS containing 0.5% triton and a protease inhibitor cocktail (Roche). Cells were lysed by sonication and the cell debris was removed by ultracentrifugation at 14.000 rpm. Supernatants were incubated with GST-agarose beads (Santa-Cruz) at 4˚C for 1 hour.

After thorough washing of the GST-agarose with PBS, crude cell extracts containing prey proteins were added followed by an incubation on a rotating wheel at 4 ˚C over-night.

The reactions were again washed 3 times with PBS, and specifically bound proteins were pelleted, resuspended in sample buffer, and analyzed by SDS-PAGE followed by Western blot analysis using anti-EPOR, anti-LIFR, anti-gp130 or anti-beta chain antibodies (Santa Cruz Biotechnology).

7. Erythroid Colony formation assay

Bone marrow cells were harvested from tibia and femur of male C57BL/6 mice (8-10 weeks, Bundesinstituts für Risikobewertung, Berlin) by flushing the bones with PBS (Biochrom) and seeded in Metho Cult SF 3236 methylcellulose (StemCeli Technologie Inc) at the density of 225.000 cells per 35 $mm^2$ Petri dish. 60 pM EPO, 60 pM EPO variants or 200 U/1 rhEPO from Roche, used as positive control, were added to the methylcellulose respectively. Plates were incubated at 37˚C in a humified atmosphere containing 5% $CO_2$ for 48 h. Reddish colonies containing at least six hemoglobinised cells were taken into account for evaluation of the experiments.

8. *In vivo* hematopoiesis assay

*In vivo* hematopoietic activity of vEPO was assayed in male C57B16 mice (8-10 weeks, Bundesinstituts für Risikobewertung, Berlin). Mice were injected intraperitoneally with 42 fig/kg recombinant human erythropoietin ERYPO (Janssen Cilag), purified hEPO produced in CHO-S (hEPO) cells, purified hS3 produced in CHO-S cells (hS3) cells or equivalent volumes of PBS (pH 7.4) as controi. Injections were repeated every 2 to 3 days for a total of three applications with 5 to 10 mice per group. Hematocrit was measured 48 h after the last injection using heparinized microcapillaries.

9. Preparation of rat primary cortical neurons

Primary cultures of neuronal cells from cerebral hemispheres of 17 to 18-day-old Wistar rat embryos (Bundesinstitut für gesundheitlichen Verbraucherschutz und Veterinärmedizin) were prepared as previously described (Ruscher et al., 2002). Cerebral cortices were isolated and incubated with 0.05% trypsin/EDTA (Biochrom) for 15 min at 37˚C. After rinsing with MEM (Gibco) supplemented with 10% FCS (Biochrom), 100 IE/l insulin (Aventis), 10 mM HEPES (Biochrom) and 44 mM glucose, tissues were carefully triturated with fire-polished Pasteur-pipettes. Cells were plated at $3x10^5$ cells/well in 24-well plates double-coated with poly-L-lysine (Biochrom) and collagen G (Biochrom) in Neurobasal medium (Gibco) supplemented with 2% B27 (Gibco), 0.5 mM L-glutamine, 1% Pen/Strep, and 25 μM glutamate (Sigma-Aldrich). Neuronal cultures were maintained at 5% $CO_2$ and 37˚C.

10. Induction of neuroprotection with EPO variants in an *in vitro* model of cerebral ischemia

On day in vitro 8 (DIV 8) two third of the culture medium was removed and replaced for 48 h with fresh NBM/B27 (Neurobasal medium supplemented with B27, 0.5 mM L-glutamine, 1% Pen/Strep) containing recombinant murine and human EPO, EPO splice variants or A-helix derivatives. Neuronal cultures were deprived of oxygen and glucose in a deoxygenated aglycaemic solution (BSS0) containing 6.8 g/l NaCl, 5.4 g/l KCl, 0.8 g/l $MgSO_4*7H_2O$, 1.0 g/l $NaH_2PO_4$, 26.2 g/l $NaHCO_3$, 0.265 g/l $CaCl_2$ and 0.0001 g/l glycine (pH 7.4). Hypoxic atmosphere was generated by a dedicated humified gas-tight incubator (Concept 400, Ruskinn Technologies) flushed with a gas mix containing 5% $CO_2$, 85% $N_2$ and 10% $H_2$. Oxygen-glucose deprivation (OGD) was terminated after 2.5 to 3 h by replacing the aglycaemic solution with oxygenated medium consisting of 50% NBM/B27 and 50% stored culture medium. Control cultures were treated with the oxygenated glycaemic BSS0 solution (BSS20; $BSS_0$ supplemented with 4.5 g/l D-Glucose) and incubated at 37°C in a normoxic atmosphere containing 5% $CO_2$. Cell death was determined by 24 h lactate dehydrogenase (LDH) release into the medium. 20-25 μl cell of medium from each culture well was transferred to 96-well culture plates and mixed with 100 μl of a 0.15 mg/ml β-NADH (Sigma-Aldrich) solution. Enzymatic reaction was started with 25 μl of 22.7 mM pyruvate (Sigma-Aldrich). Optical density was measured at 340 nm using a microplate reader (Thermo Labsystems, $MRX^{TC}$ Revelation). The kinetic of the reaction was established from 10 counts with 30 sec intervals. LDH-standard was obtained from Greiner (system calibrator). The maximal releasable LDH was obtained in each well by 30 min incubation with 0.5% triton X-100 at the end of each experiment.

11. Signalling cascades in primary cortical neurons

For analysis of signalling pathways, primary cortical neurons were grown for eight days in 6-well plates and starved for 60 min by changing the cell culture medium to the aglycaemic BSS20 solution. After Stimulation with EPO or EPO variants, cells were harvested in cell lysis butTer (New England Biolabs), incubated on ice for 5 min, sonicated for 1 min at 4°C and centrifuged at 18,000 g for 10 min at 4°C. Whole protein concentration was determined by using a bicinchoninic acid (BCA) protein assay (ThermoFisher Scientific). Cell lysates were diluted in sample buffer and boiled for 5 min. Equivalent amounts of protein were separated on 12% SDS-polyacryiamid gels. Proteins were blotted onto nitrocellulose membranes for 35 min to 90 min. Blocking was performed for 1 h in TBST supplemented with 5% bovine serum albumin (Invitrogen). Primary polyclonal antibodies against Bad (1:500), phospho-Bad Ser 136 (1:100), phospho-Bad Ser 112 (1:500), and phospho-pIKK (1:500) were purchased from New England Biolabs. Primary polyclonal antibodies against phospho-Jak2 (1:200), phospho-Jak1 (1:200), phospho-Stat5 (1:200) and Jak2 (1:250) were purchased from Santa Cruz Biotechnology. Incubations with primary antibodies were performed over-night at 4°C in 5% BSA in TBST. The secondary antibody (goat anti-rabbit HRP; 1:1000; GE Healthcare) was added for 2 h at room-temperature in 1% non fat-dry milk in TBST. Signals were visualized on photographic films (Hyperfilm, GE Healthcare) using chemiluminescence (Santa-Cruz Biotechnology).

12. Akt kinase assay

Cortical neuronal cultures grown for eight days in 6-well plates were stimulated for 30 min with 30 pM EPO or EPO variants and harvested in cell lysis buffer (New England Biolabs). A non-radioactive Akt kinase assay was performed according to the protocol provided by the supplier (New England Biolabs).

13. AG490 kinase inhibitor experiment

The Janus kinase 2 inhibitor AG490 was obtained from Calbiochem (Schwalbach, Germany). 5 μM AG490 was applied to neuronal cultures 1 h before preconditioning with EPO or EPO variants and was washed out with the NBM culture medium immediately before OGD.

14. Blocking experiments

Human soluble receptors sLIFR alpha and sEPOR were purchased from R&D Systems. The soluble receptors were pre-incubated with the preconditioning medium for 1 h on a rotating shaker at 4°C before application to neuronal cultures. Final concentrations of soluble receptors in neuronal cultures were adjusted to 1.5 μg/ml sEPOR and 2.5 μg/ml sLIFR respectively. Antibodies directed against epitopes in the N-termini of gp130 and LIFRα proteins were purchased from Hypromatrix (Worcester MA). Neuronal cultures were preincubated for 30 min with 1 μg/ml antibody before addition of medium containing hEPO or the hEPO splice variant hS3. Soluble receptors and antibodies were washed out with the NBM culture medium immediately before OGD.

15. H9c2

The rat heart myoblast cell line H9c2 was obtained from ECACC (European Collection of Cell Cultures). H9c2 cells were grown in DMEM (4.5 g/l glucose, Biochrom) supplemented with 10% fetal calf serum and splitted twice a week before reaching confluency. For experiments cells were plated in medium containing 120 pM EPO or EPO variants at the density of 15,000 cells per well in 24-well plates. After 48 h, cultures were submitted to oxygen and serum deprivation. Medium was replaced by serum-free DMEM and cultures stayed for 24 h in the anaerobic Workstation. Control cultures were left in serum-free DMEM in a normoxic incubator. At the end of the experiment medium was replaced to fresh serum-free DMEM and LDH release into the medium was assessed 24 h later as described previously.

16. Ba/F3-rescue-experiments

A murine pro-B IL-3 dependent cell line stably transfected with the erythropoietin receptor (Ba/F3-EPOR) was provided by PD Dr. rer. nat. Ursula Klingmüller (Deutsches Krebsforschungszentrum Heidelberg). Ba/F3 and Ba/F3-EPOR cells were cultured in RPMI (Biochrom) supplemented with 10% FCS and 1 ng/ml IL-3 (Promega). Puromycin (InvivoGen) was used for positive selection of Ba/F3-EPOR. For rescue experiments, cells were seeded at $10^5$ cells/per ml in 96-well plates. Cells were grown for 48 h either in presence or absence of IL-3, hEPO (300 pM) or hS3 (300 pM). LDH release into the medium and total LDH contents were measured as parameters in order to determine vitality of cells in different culture conditions.

17. Radioactive displacement from Ba/F3-EPOR cells

Radioactive displacement assays were performed with Ba/F3-EPOR cells ($5 \times 10^6$ cells/ml) in RPMI supplemented with 10% FCS and 1 ng/ml IL-3. 200 μl cell suspension ($1 \times 10^6$ cells) was incubated with 1 nM $^{125}$I-rhEPO (GE Healthcare) for 45 min at 37°C. Excess unbound radioactivity was washed out before incubating the cells with hEPO or human EPO splice variants in increasing concentrations for 90 min at 37°C. Cells were pelleted, washed once with medium and resuspended in 'Hisafe3' scintillator medium (GE Healthcare). Cell-associated radioactivity was determined in a gamma counter.

18. Radioactive displacement from Ba/F3-gp130/LIFRa cells

For cloning of the murine gp130 chain, the signal transduction sub-unit of the heterodimeric LIF receptor, *XhoI* and *PmeI* restriction sites were added to the PCR products by using overhang sense primers and overhang antisense primers (Gpl30_fw: ATC TCG AGA TGT CAG CAC CAA GGA TTT GGC TAG CG SEQ ID No 27 and Gp130_rev: AGG TTT AAA CTC ACT GCG GCA TGT AGC CAC CTT G SEQ ID No 28). The resulting PCR product was inserted into the pcDNA3.1/V5-His vector.

Ba/F3 cells were transiently transfected with murine gp130 and LIFRα by electroporation using Amaxa system's proprietary solutions and transfection conditions. 48 h after transfection cells were harvested, transferred to PBS/BSA (0.5%) and used for experiments. $4 \times 10^6$ cells per assay were preincubated for 30 min at 37 °C with 50 μM Dansylcadaverine (MDC), an inhibitor of transglutaminase, to prevent receptor internalization. Subsequently, cells were incubated under slight agitation for 4 h at 4°C with 0.5 nM $^{125}$I-mLIF. Unbound radioactivity was displaced by washing the cell pellet twice with 1 ml PBS, respectively. Cells were resuspended in PBS/BSA (0.5%) in presence of cold mLIF or vEPO and incubated for further 90 min at RT. Cell-associated radioactivity was determined by pelleting the cells (1200 rpm, 4°C, 2 min), washing them once with 1 ml PBS and resuspending them in 'Hisafe3' scintillator medium (GE Healthcare). Radioactive counts were measured in a gamma counter.

19. M1 proliferation assay

The mouse myeloid leukemia M1 cell line is known to differentiate into mature macrophages and granulocytes *in vitro* when treated with various stimulators such as LIF, OSM or IL-6. This differentiation is accompanied with growth arrest. The mouse myeloid leukemia M1 cell line was purchased from the cell culture collection ECACC. Suspension cultures were maintained at a density of 200,000 to 900,000 cells per ml in RPMI 1640 supplemented with 10% FCS GOLD, 2 mM L-Glutamine and 1% Pen/Strep in 25 $cm^2$ or 75 $cm^2$ culture flasks (Sarstedt). For proliferation experiments in presence of the EPO variants, cells were seeded at the density of 150,000 cells per ml in presence or absence of 15 nM hEPO, hS3 or equivalent volumes of MOCK-medium or in presence or absence of 500 nM peptide P16 in 96 well plates (Falcon, BD Biosciences). Each condition was repeated in presence of 50 ng/ml murine LIF (Chemicon). Proliferation of the cells was evaluated by measuring the vitality by means of the MTT-test as this test was shown to correlate with the level of LIF used for stimulation (Ohno *el al.,* 1991). For MTT-assay cells were incubated for 2.5 h at 37°C with 0.05 mg/ml Thiazolyl blue (Sigma) before addition of equal volumes of 10% SDS in 0.01 M HCl. The next day, optical density was measured in a plate reader (Thermo Labsystems, MRXTC Revelation) at 550 nm wave length.

20. Crosslinking Experiments

Bis(sulfosuccinimidyl)suberate (BS3) is a homobifunctional, water-soluble, non-cleavable, membrane impermeable crosslinker and was purchased from Pierce.

In brief, H9c2 cells were harvested by scraping the cells from the flasks. Neural spheres were collected by centrifugation and triturated with a 200 μl pipette tip in order to obtain a single cell suspension. H9c2 cells and NSC were resuspended in PBS (pH 8.0). Proteins expressed in *E. coli* and purified via the GST-tag were added to the cell suspensions in a final volume of 350 μl. Proteins were incubated with the cells on a rotating wheel for 4 h at 4˚C. BS3 stock solution (50mM) was prepared in $H_2O$ shortly before use and added to the cells for 30 min at 4˚C. The reaction was stopped by adding Tris-HCl at a final concentration of 20 mM and incubation at RT for 15 min. Cells were washed thrice with PBS (pH 8.0) before lysis in 1x CLB (Cell signalling).

21. siRNA in primary cortical neurons

Primary cortical neurons were seeded in 6-well plates and transfected on DIV 7 with gp130 and LIFRα siRNAs. Control cultures were transfected with a Cy3-labelled negative control siRNA. All siRNAs were purchased from Ambion. In brief, cultures were transfected with 8 μg/well siRNA using TransMessenger Transfection Reagent from Qiagen according to the protocol provided by the supplier. 24 h after transfection neuronal cultures were kept for 1 h in BSS20 solution before 30 min stimulation with 50 ng/ml hS3 or equivalent amounts of MOCK-medium. Cultures were rinsed once with PBS and lysed in 70 μl/well cell lysis buffer (New England Biolabs) supplemented with 1 mM PMSF. After a 5 min incubation period on ice, cells were sonicated and centrifuged for 10 min at 14,000 rpm and 4˚C. Protein concentration was determined by using a bicinchoninic acid (BCA) protein assay (Pierce). The proteins separated by SDS-PAGE gel electrophoresis were then transferred onto nitrocellulose membranes. After blocking with 5% bovine serum albumin (Invitrogen) in Tris-buffered saline at room temperature for 1 h, the membranes were incubated with primary antibody at 4˚C overnight. Primary polyclonal antibodies against phospho-Akt Ser473 (1:1000) and Akt (1:1000) were purchased from New England Biolabs. Primary polyclonal antibodies against LIFR (1:250) were purchased from Santa Cruz Biotechnology. Horseradish peroxidase-conjugated secondary antibody (1:1000, GE Healthcare) was then added for 2 h at room-temperature in 1% non fat-dry milk in TBST.

Proteins were visualized on photographic films (GE Healthcare) using chemiluminescence (Santa-Cruz Biotechnology).

22. Homology modelling

The Homology modelling of mS and hS3 was performed at the SWISS-MODEL Protein Modelling Server (SW1SS-MODEL Version 36.0003, (Arnold *et al,* 2006; Guex *et al,* 1997; Kopp *et al,* 2004; Peitsch, 1995; Schwede *el al,* 2003) database using the templates leerA.pdb (rhEPO), lbuyA.pdb (rhEPO) and lcn4C.pdb (murine erythropoietin) as scaffolds. The templates were obtained from the RCSB Protein database (http://www.rcsb.org/pdb/home/home.do). Default parameters were not changed for construction of the 3D models of mS and hS3. Manual corrections were not performed. VisuaHzation of structure predictions were done in the Astex Viewer™ developed by Astex Technology Limited (Cambridge, UK).

23. Statistical Analysis

Data are presented as mean ± SD. For statistical comparisons of 2 groups, a Student's t-test was used. For > 2 groups, a 1-way ANOVA was applied. Values for $p<0.05$ were considered statistically significant.

**II. Results**

1. Alternative splicing of EPO mRNA in kidney and brain

We generated cDNA from the kidneys of three adult mice and performed nested PCR for EPO using the primers described above. In all three independent reactions, a PCR product of 579 bp length was obtained and confirmed to correspond to murine EPO cDNA by sequencing. In the case of human kidney, an expected PCR product of 582 bp was obtained, which corresponded to human EPO cDNA (Figure 1A). Surprisingly, several additional smaller sized bands were observed for both mouse and human kidney (Figure 1A). In order to minimize the possibility that these bands resulted from skipping over looped-out regions during reverse transcription, we used AMV reverse transcriptase and *Thermus flavus* DNA polymerase for cDNA synthesis. This removed a number of the additional bands, but the prominent smaller sized PCR products of approximately 490 bp and 400 bp length for human and mouse kidney, respectively, were retained. Sequencing of these additional PCR products revealed alternatively spliced EPO transcripts (Figures 1 D). In the human tissue, two EPO variants were detected with either complete loss of exon 3 (denoted hS3) or partial loss of exon 4 (denoted hS4). In the murine tissue, one EPO variant was found with complete deletion of exon 4 (denoted mS). In all cases, splicing followed the classical GU-AG rule (Figure 1E). Since EPO expression was also found in the nervous system (Marti et al., 1996), we repeated the nested PCR for EPO using human fetal brain polyA+ RNA. In this assay, EPO and hS3 transcripts were detected. Moreover, we obtained cDNA from the brains of three hypoxic adult mice. The expression levels of EPO mRNA are notoriously low in adult murine brain, but can be induced by hypoxic conditions via activation of HIF (Prass et al., 2003). In all three independent samples, the presence of EPO and mS transcripts was confirmed (Figure 3B). In order to determine whether any of the EPO splice variants was also expressed on the protein level, we treated adult mice with cobalt chloride ($CoCl_2$), which is known to induce EPO mRNA expression (Goldberg et al., 1988). After 18 hours, samples from brain, kidney and blood were obtained and EPO was immunoprecipitated using a polyclonal antibody against recombinant mouse EPO (Figure IC). Western blot analysis and ELISA revealed 3.8 pg EPO/mg protein in the serum and 5.2 pg EPO/mg protein in the kidney of an untreated adult mouse, while no EPO expression was detectable in brain. After $CoCl_2$ administration, EPO expression reached up to 226.4 pg EPO/mg protein in the serum, 116.7 pg EPO/mg protein in the kidney and 5.7 pg EPO/mg protein in the brain. Importantly, when large amounts of immunoprecipitate from kidneys of $CoCl_2$-treated mice were analyzed by Western blotting, bands corresponding in size to EPO and recombinant mS were detected by an antibody direct against recombinant human EPO (Figure IC). Preabsorption of the antibody with recombinant human EPO confirmed the specificity of the signals. The data suggest that EPO splice variants are also translated *in vivo* under hypoxic conditions.

<u>2. EPO variants are devoid of hematopoietic activity</u>

We speculated that alternatively spliced EPO variants may be functionally distinct from EPO. Thus, we generated recombinant human EPO (hEPO), murine EPO (mEPO), hS3, hS4 and mS in HEK293 and CHO cells. A C-terminal V5/His tag was used to purify the recombinant proteins via metal affinity chromatography. Hematopoietic clonogenic capacities of the recombinant proteins were assessed in methylcellulose using murine bone marrow cells. Commercially available recombinant hEPO from Roche (200 U/l) strongly induced the formation of colony forming units-erythroblast (cfu-e), while equimolar concentrations of recombinant hS3 or hS4 did not reveal any hematopoietic activity (Figure 2A). We produced recombinant mEPO in HEK cells and found its hematopoietic activity to be comparable to rhEPO from Roche in colony forming assays (Figure 2B). mS produced in HEK cells failed to induce the formation of cfu-e (Figure 2B), suggesting that all EPO variants are devoid of hematopoietic activity *in vitro.* We next sought to determine whether alternatively spliced EPO variants are also functionally distinct from EPO *in vivo.* Adult mice were injected i.p. with 42 ng/kg (5,000 U/kg) commercially available recombinant hEPO from JANSSEN-CILAG as outlined in Figure 2C. After 7 days, a significant increase in hematocrit was observed in the mice (Figure 2D). For *in vivo* assays of hematopoietic activity, we produced recombinant hEPO and hS3 in CHO cells, since hEPO produced in HEK cells only induced hematopoiesis *in vitro* (data. not shown). The extent of hematocrit increase in mice treated with 42 $\mu$g/kg hEPO was comparable to that of animals treated with the same concentration of rhEPO from JANSSEN-CILAG (Figure 2D). Thus, our recombinant V5/His-tagged EPO protein retains full biological activity. No change in hematocrit was observed in mice treated with 42 $\mu$g /kg hS3 compared to PBS-treated controls (Figure 2D). In conclusion, murine and human EPO splice variants are devoid of hematopoietic activity *in vitro* and *in vivo.*

<u>3. EPO variants are cytoprotective</u>

Primarily known as a hematopoietic cytokine, EPO was also found to be a potent tissue-protective agent. Recently, synthetic EPO derivatives were generated, which confer neuroprotection without stimulation of erythropoiesis in rodents (Erbayraktar et al., 2002; Leist et al., 2004). These results suggested that EPO's hematopoietic and cytoprotective activities can be separated. Given that the EPO splice variants hS3, hS4 and mS did not promote erythropoiesis, we wanted to determine whether they may be part of an endogenous cytoprotective system. An *in vitro* model of cerebral ischemia (oxygen glucose deprivation, OGD) established by Ruscher et al. (2002) was used to test the neuroprotective ability of EPO and its splice variants. Commercially available rhEPO from Roche (100 U/l) protected rat primary cortical neurons from cell death when administered 48 hours before OGD (Figure 3A). A similar effect was observed with equimolar concentrations of hS3, hS4, mS and mEPO produced in HEK cells (Figure 3A). The neuroprotective effects of human EPO and human EPO variants were concentration-dependent with half-maximal effects (EC50) between3-8 pM and maximal effects ($EC_{max}$) between 30-75 pM, which corresponds to 100-250 U/l rhEPO from Roche as indicated by the supplier (Figure 3B). At higher doses, the neuroprotective abilities of EPO and EPO variants were lost as described in other *in vitro* and *in vivo* models of neuroprotection (Sakanaka et al., 1998; Keswani et al., 2004). To test for more general cytoprotective abilities, cardiac myoblasts were pretreated with 90 pM hEPO or hS3 for 2 days before serum and oxygen deprivation. The degree of myoblast cell death was significantly attenuated by hEPO and hS3 compared with controls (Figure 3C). The data indicate that murine and human EPO splice variants are as efficacious as EPO in conferring cytoprotection, while devoid of hematopoietic activity.

4. EPO and EPO variants share the same signal transduction pathways mediating neuroprotection

As previously described for commercially available rhEPO (Siren et al., 2001; Ruscher et al., 2002), Janus kinase-2 (JAK-2), phosphoinositide-3 kinase (PI3K) and the downstream effector serine/threonine protein kinase B (Akt) are involved in EPO-mediated neuroprotection by inactivation of the proapoptotic protein BAD. We confirmed these findings with 30-90 pM hEPO produced in HEK cells using Western blot analysis, Akt kinase assay and OGD assay in the presence of the specific JAK-2 inhibitor, AG490 (Figures 4A-C). In addition, another target of Akt, the inhibitor of κB kinase (IKK), was phosphorylated within 15 minutes after hEPO treatment in primary neuronal cultures (Figure 4A). This suggests additional involvement of the nuclear factor-kappaB (NF-κB) pathway, as previously described for EPO-mediated neuroprotection (Digicaylioglu and Lipton, 2001). At equimolar concentrations to hEPO, hS3 produced in HEK cells stimulated the phosphorylation of JAK-2 and the downstream effectors, Akt and IKK (Figures 4 A and B). As a result of Akt activation, phosphorylation of BAD at serine residues 112 and 136 was increased (Figure 4A). Like hEPO, the neuroprotective effects of hS3 depended on the activation of JAK-2 as demonstrated by the loss of cytoprotection in OGD assays performed in the presence of AG490 (Figure 4C). The data suggest that EPO and EPO variants share the same signal transduction pathways mediating cytoprotection.

5. Protein structure predictions of EPO variants

Murine and human EPO consist of four-helix bundles, typical of members of the hematopoietic growth factor family (Cheetham et al., 1998). The four long α-helices (A, B, C and D), which are oriented in an up-up-down-down direction, are connected by two long cross-over loops (AB and CD) and one short loop (BC). The short α-helical segment (B') located at the carboxy end of the AB loop has been suggested to be important for EPO receptor binding (Cheetham et al., 1998). Based on the protein sequence, the primary protein structure of hS3 suggests loss of the AB loop containing the short α-helix B' (Figures 5A and B). This results in a dramatic change in tertiary structure compared with rhEPO as predicted by automated homology modeling (Figure 5C). In the case of hS4, the protein is predicted to have lost helix B (Figure 5B). The protein sequence of murine mS suggests loss of the two helices B and C (Figure 5B), resulting in a two-helical tertiary structure prediction (Figure 5C). We expect that the changes in protein structure of EPO variants compared to EPO result in disturbed binding of the variants to the homodimeric $(EPOR)_2$. The first, high affinity EPO receptor binding site involves residues at the interface of the AB loop with helix D (Cheetham et al., 1998), which are lost in the human EPO variant hS3. The interaction of residues in the AB loop with the amino terminus of the B helix, which is lost in hS4, was suggested to play an important structural role in EPO receptor binding (Cheetham et al., 1998). Finally, the murine EPO variant mS lacks residues within the C helix, which constitute part of the low affinity EPO receptor binding site (Cheetham et al., 1998).

6. EPO variants do not bind to the homodimeric $(EPOR)_2$

All murine and human EPO variants share the conserved helices A and D (Figure 5B). Earlier mutagenesis and structural studies revealed that the functionally important domains of many hematopoietic cytokines encompass portions of helix A (Kaushansky and Karplus, 1993). Moreover, an essential functional domain for the hematopoietic activity of hEPO was found to consist of residues $Arg^{14}$ of helix A, $Arg^{103}$ and $Ser^{104}$ of helix C (Wen et al., 1994). We therefore tested the importance of residue $Arg^{14}$ of helix A for the neuroprotective activities of EPO and its variants. First, we expressed hEPO helix A in HEK cells and found significant neuroprotection conferred by the supernatants in the OGD model (Figure 6A). Next, we generated $Arg^{14}\rightarrow$Ala and $Arg^{14}\rightarrow$Glu mutants of hEPO helix A by site-directed mutagenesis. In earlier studies, the mutein $Arg^{14}\rightarrow$Ala showed substantially reduced hematopoietic activity, whereas the mutein $Arg^{14}\rightarrow$Glu resulted in total loss of biological activity (Wen et al., 1994). Interestingly, neither mutein had any effect on the neuroprotective activity of helix A (Figure 6A). Thus, we hypothesized that EPO variants do not activate the homodimeric $(EPOR)_2$. Ba/F3 cells transduced with the EPOR (generously provided by Dr. Klingmüller, Heidelberg, Germany) can be used as a bioassay for EPOR binding and activation. Ba/F3 cells normally depend on IL-3, which can be compensated by EPO in Ba/F3-EpoR cells (Jubinsky et al., 1997). We found that 300 pM hEPO produced in HEK cells rescued Ba/F3-EpoR, but not Ba/F3 cells, from cell death as a result of IL-3 withdrawal (Figure 6B). In contrast, 300 pM hS3 produced in HEK cells failed to rescue Ba/F3-EpoR cells (Figure 6B). The bioassay data were supplemented by radioactive binding assays. In Ba/F3»EpoR cells, binding of commercially available [$^{125}$I]-rhEPO from Amersham (1 nM) was displaced by cold hEPO produced in HEK cells in a dose-dependent manner (Figure 6C). Neither increasing concentrations of hS3 or hS4 produced in HEK cells (10 pM-10 nM), nor high concentrations of P16 (10 pM-10 μM) were able to displace [$^{125}$I]-rhEPO binding (Figure 6C). Our results suggest that human EPO variants and peptides derived from helix A do not bind to the homodimeric $(EPOR)_2$.

7. EPQ variants can mediate neuroprotection through the LIF receptor family

In an attempt to characterize the binding sites for EPO variants, we performed pull-down assays using protein extracts derived from Ba/F3-EpoR cells as the prey. GST-tagged mEPO and mS produced in *E. coli* were used as bait proteins. In three independent experiments, EPOR was captured by mEPO, but not by mS (Figure 7A). We also did not find an interaction between mS and βcR (data not shown). These results suggested that EPO variants may not interact with homodimeric $(EPOR)_2$, EPOR or βcR. We therefore turned to other members of the cytokine receptor superfamily, particulary those expressed in neural tissue, given that hS3, hS4 and mS conferred significant neuroprotection. Using protein extracts derived from rat primary cortical neurons as the prey and GST-tagged mEPO, mS and hS3 produced in *E. coli* as the bait, LIFRα was identified as an interaction partner for mS (Figure 7B). For further confirmation of these findings, we produced His-tagged murine soluble LIFR (msLIFR) in HEK cells. Using the supernatants as the prey, msLIFR was captured by mS, but not by mEPO or hS3 (Figure 7C). Working on the assumption that hS3 may have a higher affinity towards human than rodent LIFRα (hLIFRα), we used protein extracts derived from HEK cells as the prey. Indeed, hS3 interacted with hLIFRα (Figure 7D). A somewhat weaker interaction was also found for mS, while mEPO did not capture hLIFRα (Figure 7D). In order to assess whether the interaction of EPO variants with members of the LIFR family may be involved in neuroprotection, we reverted to OGD experiments. Coadministration of soluble LIFR (sLIFR) with 30 pM hS3 completely abolished the neuroprotection conferred by the EPO variant (Figure 7E). In contrast, sLIFR did not block the neuroprotective effects of 30 pM hEPO (Figure 7E). Parallel experiments using soluble EPOR (sEPOR) revealed that EPOR was involved in the neuroprotection mediated by hEPO, but not by hS3 (Figure 7E). These results are in agreement with earlier studies suggesting involvement of EPOR in the tissue protection by EPO (Sakanaka et al., 1998; Ruscher et al., 2002; Brines et al., 2004). Pretreatment of primary neuronal cell cultures with antibodies against the amino terminus of LIFRα or the signal transducing receptor subunit, gp130, had no effect on hEPO-mediated neuroprotection (Figure 7F). However, the antibody against gp130 completely abolished the neuroprotection conferred by 30 pM hS3 (Figure 7F). Pretreatment of neuronal cultures with the antibody against LIFRα only resulted in a mild inhibitory effect on hS3-mediated neuroprotection (Figure 7F), which may result from incomplete blocking of the ligand binding site. In conclusion, our data suggest that human and murine EPO variants can mediate neuroprotection through the LIF receptor family.

From the former experiments we expected the LIFRα and the gp130 chain to be involved in the receptor binding of the EPO splice variant hS3. To test if the classical heterodimeric LIF receptor composed of one LIFRα-chain and one gp130-chain is sufficient to mediate the vEPO effects, we established a radioactive binding assay to test the ability of the EPO variants to displace mLIF from its receptor binding site (Figure 8A). The principle behind this test was to saturate LIFR-binding sites on BaF3 cells transiently transfected with LIFRα and gp130 with radioactive mLIF before incubation of the cells with non-radioactive mLIF and EPO variants. Binding of non-radioactive mLIF displaces radioactive mLIF thereby lowering radioactivity measured in the cell pellet (Figure 8B). GFP-transfected control cells were shown not to bind specifically mLIF (Figure 8B).

BaF3 cells were used 48 h after transfection with gp130 and LIFRα and saturated with radioactive labeled mLIF. Saturated cells were incubated with different concentrations of unlabelled mLIF, EPO isoforms or control medium to test for displacement capacities of the variants. After thorough washing bound radioactivity was counted and displacement calculated as the difference between the amount of radioactivity in the absence of non-labeled mLIF and the amount of radioactivity bound in the presence of non-labeled mLIF or EPO variants. Counts measured in the absence of non-labeled mLIF were defined as 100%.

Importantly, only mLIF was able to displace $^{125}$I-rhEPO from transfected BaF3 cells. Neither glycosylated hS3 expressed in HEK293 cells nor vEPO expressed in *E.coli* (matmS, mathS3 or matmE) were able to displace $^{125}$I-mLIF from its binding site (Figure 8C and D).

The LIFR pulldown experiments and the functional assays on primary cortical neurons provided strong evidence implicating the LIFRα or gp130 in mediating the effect of the EPO splice variants. The glycoprotein LIF was initially identified based on its ability to induce suppression of proliferation and differentiation in the mouse myeloid leukemia cell line M1. To test if the EPO variants might also activate the LIFR, M1 cells were treated with murine LIF (mLIF, 50 ng/ml), hEPO (15 nM), hS3 (15 nM) or P16 (500 nM), respectively. The quantitative colorimetric MTT assay was used to measure proliferation of the cultures since the MTT-test was shown by Ohno et al. (Ohno *et al.,* 1991) to correlate with levels of LIF stimulation. The suppression of proliferation mediated by LIF was found to be maximal 72 h after onset of the stimulation.

Interestingly, in three independent experiments a weak but significant suppression of proliferation (around 10%) was observed in cultures treated with hS3 as single factor compared to MOCK-treated control cultures (Figure 9A). In contrast, hEPO- and P16-treated showed similar proliferation rates as control cultures. When cultures were treated with a combination of mLIF and EPO variants, no statistically significant differences were observed between treatment groups. Three days after seeding proliferation rates of the cultures had dropped by 30 - 55% compared to control cultures (MOCK-cultures without mLIF-treatment) (Figure 9B).

Crosslinking experiments were performed to identify the unknown receptor of the EPO splice variants mS and hS3. To ensure cell-surface specific crosslinking for identification of surface receptors it is best to use membrane-impermeable cross-linkers such as BS3 (Pierce). We used proteins expressed in *E.coli* for these experiments due to higher concentrations available, higher purity compared to HEK293-expressed proteins and due to lower aggregation tendencies. After crosslinking of matmS and mathS3 to the target cells (H9c2 and NSC), proteins were revealed using the anti-rhEPO antibody from Santa-Cruz previously shown to recognize mathS3 and matmS.

The subsequent immunoprecipitation experiments were performed to purify the crosslinked complexes from the total protein lysate. The anti-mEPO antibody was used for the immunoprecipitation reactions as this protein as well recognizes the EPO variants. The precipitated complexes were analysed on Western Blots using the anti-rhEPO antibody from Santa-Cruz.

The crosslinking experiment reveals four individual complexes of the sizes >250 kDa, 170 kDa, 100 kDa and 70 kDa. The 40 kDa protein corresponds to unbound mathS3. Thus, the receptor components are probably of sizes: 130 kDa, 60 kDa and 30 kDa. The fourth complex (>250 kDa) may contain LIFRα (190 kDa), but may neither migrate in the gel nor be immunoprecipitated due to the very high molecular weight.

In this study, we identified EPO splice variants in humans and mice. The EPO variants were as cytoprotective as EPO, but did not stimulate erythropoiesis. We found binding of the EPO variants to the LIF receptor family instead of the classical homodimeric $(EPOR)_2$.

Processing of EPOR transcripts has been known for a long time. In early-stage erythroid progenitor cells, the cytoplasmic region of EPOR is truncated by alternative splicing, resulting in the transduction of a mitogenic signal, whereas the full-length EPOR expressed on late-stage progenitors acts to prevent apoptosis (Nakamura et al., 1992). Truncation of the extracellular domain of EPOR has also been described as a result of the loss of exon 5 by alternative splicing (Barron et al., 1994). In the adult brain, the level of the mature EPOR transcript is significantly lower than in hematopoietic tissue, and the EPOR transcript is alternatively or inefficiently processed (Chin et al., 2000). EPO is highly conserved across species with approximately 80% homology between human and rodent EPO (Wen et al., 1993). To date, no alternative splicing of mammalian EPO transcripts has been described. However, EPO transcripts in the *Fugu rubripes* brain are generated from a distal promoter and include an alternatively spliced first coding exon (Chou et al., 2004). Here we provide evidence for alternative splicing of human and murine EPO mRNAs in kidney and brain. Splicing occurs by partial or complete exon skipping and follows the classical GU-AG rule. We find no evidence for tissue specificity of the mammalian EPO splice variants.

Alternative splicing is a major contributor to protein diversity in eukaryonts, and different protein isoforms can have divergent functions. EPO is best known for its erythropoietic properties. It is a vital hematopoietic cytokine since mice deficient in EPO die *in utero* owing to failure of definitive fetal liver erythropoiesis (Wu et al., 1995). None of the EPO variants we discovered in humans and mice show any hematopoietic activity. As a member of the cytokine superfamily with homology to mediators of growth and inflammation, EPO has also come to be recognized as a paracrine/autocrine mediator of tissue protection (Erbayraktar et al., 2003). Interestingly, all EPO splice variants share the cytoprotective function with EPO. The levels of protein expression were too low to isolate EPO or EPO variants from healthy tissue by an immunoprecipitation approach. Mimicking hypoxic conditions by $CoCl_2$-treatment to induce HIF, we observed the co-regulated synthesis of EPO and its splice variant in the mouse kidney. Thus, we speculate that EPO variants may be part of an endogenous tissue-protective system.

In line with the absence of hematopoietic activity, EPO variants do not bind to the homodimeric $(EPOR)_2$. This may be explained by the predicted changes in tertiary protein structure of the EPO variants. Alternative splicing results in the deletion of the high affinity EPOR binding site in hS3, the low affinity EPOR binding site in mS and the structurally important B helix in hS4 (Cheetham et al., 1998). Recently, the EPOR/βCR heteroreceptor was identified as a tissue-protective receptor for EPO (Brines et al., 2004). However, none of the EPO splice variants displayed binding to either EPOR or βCR, excluding the EPOR/βCR heteroreceptor as the primary binding site for the endogenous EPO variants.

Using pull-down assays with rat primary tissue and human cell lines, we captured LIFRα as a potential interaction partner of the human and murine EPO variants. The classical LIFR is composed of two subunits: LIFRα, which binds LIF with low affinity, and gp130, which does not bind LIF, but is essential for high affinity complex formation and signal transduction (Gearing et al., 1992). The failure to pull-down gp130 with the EPO variants as bait proteins may result from the fact that gp130 is not directly involved in ligand binding. However, LIFRα and gp130 are critical for the neuroprotection conferred by the EPO variants. Interestingly, LIFR and EPOR are both members of the hematopoietic superfamily of cytokine receptors, characterized by a cytokine binding homology region with four conserved cysteine residues and a WSXWS tryptophan motif (Boulanger and Garcia, 2004). The subfamily of gp130 cytokine receptors consists of heterodimers of gp130 with LIFRα, which can associate with additional cytokine receptor subunits, such as ciliary neurotrophtc factor (CNTF)Rα (Boulanger and Garcia, 2004). CNTF, LIF, cardiotrophin-1, cardiotrophin-like cytokine, neuropoietin and oncostatin M all act through the gp130/LIFR receptor subfamily and share neuroprotective functions (Lo et al., 1995; Ikeda et al., 1996; Bordet et al., 1999; Derouet et al., 2004; Schuettauf et ah, 2005; Weiss et al., 2006). We propose that the alternatively spliced EPO variants are new members of this family of neuroregulatory cytokines (Murphy et al., 1997). Whereas LIF, CNTF, cardiotrophin-1, EPO and EPO variants may bind to different receptors, they share the same signal transduction pathways mediating neuroprotection. Thus, signaling by these receptors depends upon their association with JAKs, a common feature of all members of the cytokine receptor superfamily (Ihle, 1995). The downstream effectors PI3K/Akt and NF-κB are involved in the neuroprotection conferred by LIF (Middleton et al., 2000; Chang et al., 2004), EPO (Digicayiioglu and Lipton, 2001; Siren et al., 2001; Ruscher et al., 2002), CNTF and cardiotrophin-1 (Middleton et al., 2000; Dolcet et al., 2001). These downstream effectors also mediate the neuroprotection by EPO variants.

Several findings favor the hypothesis that the unknown receptor mediating the effects of the EPO splice variants is a member of the LIFR family. LIFR pulldown experiments revealed protein-protein interactions of LIFRα-chains derived from different species with the EPO splice variants hS3 and mS. Gp130 isoforms were not captured in these assays. However, this is in congruence with the finding that gp130 also does not bind LIF (Gearing et al., 1992). Furthermore, in receptor blocking and receptor competition experiments on primary cortical rat neurons we showed the biological importance of these protein-protein interactions. Both, soluble LIFR and antibodies directed towards the N-termini of the LIFRα-chain and gp130 blocked at least partially hS3-mediated neuroprotection in our OGD-model. Regarding their biological effects, the EPO splice variants fit perfectly into the group of cytokines binding to members of the LIFR family. In addition to neuroprotective and cytoprotective properties, the human splice variant hS3 promoted differentiation of murine neural stem cells (NSC) / neural precursor cells (NPC) into the astroglial lineage. The signal transduction of hS3 in primary cortical neurons was found to involve Jak2-, Akt- and NF-κB-pathways. These pathways were not only described for EPOR-mediated signaling but also for LIFR-mediated signaling in neuronal cultures (Chang et al., 2004; Middleton et al., 2000).

However, in proliferation assays using murine M1 myeloid leukemic cells growth inhibition after hS3 treatment was found to be much less pronounced than after treatment with mLIF. Although LIF was shown to be the most potent inhibitor of growth of M1 cells, also other cytokines such as IL-6 or OSM share this ability. Half maximal inhibition is achieved with an approximately five time higher concentration of OSM or with an approximately 100 time higher concentration of IL-6 compared to LIF (Bruce et al., 1992).

The radioactive displacement assay on BaF3 cells cotransfected with gp130 and LIFRα may exclude the classical dipartite LIFR as receptor mediating the effects of the hS3. Another more complex receptor involving LIFRα and gp130, such as CNTFR, the CT-1R or an unknown receptor, might be envisioned. Preliminary crosslinking experiments suggest a trimeric or multimeric receptor complex binding hS3 and mS.

However, the proteins of the EPO family are predicted to have very different tertiary protein structures. This suggests that we might have to deal with a whole non-hematopoietic (v)EPOR family.

In conclusion, we have identified non-hematopoietic EPO splice variants with cytoprotective properties. These molecules may be part of an endogenous tissue-protective system, which extends far beyond EPO/EPOR. The alternatively spliced EPO variants may be attractive drug candidates for cytoprotective therapies in humans due to the absence of hematopoietic side-effects compared with EPO, and potentially lower immunogenicity compared with synthetic EPO derivatives.

## LITERATURE

Alms, W.J., Atamas, S.P., Yurovsky, V.V., and White, B. (1996). Generation of a variant of human interleukin-4 by alternative splicing. Mol. Immunol. 33, 361-370.

Albensi, B.C., Mattson, M.P. (2000). Evidence for the involvement of TNF and NF-κB in hippocampal synaptic plasticity. Synapse 35, 151-159.

Banks, W.A., Jumbe, NX., Farrell, C.L., Niehoff, ML., and Heatherington, A.C. (2004). Passage of erythropoietic agents across the blood-brain barrier: a comparison of human and murine erythropoietin and the analog darbepoetin alfa. Eur. J. Pharmacol. 505, 93-101.

Barron, C, Migliaccio, A.R., Migliaccio, G., Jiang, Y., Adamson, J.W., and Ottolenghi, S. (1994). Alternatively spliced mRNAs encoding soluble isoforms of the erythropoietin receptor in murine cell lines and bone marrow. Gene 147, 263-268.

Bernaudin, M, Marti, H.H., Roussel, S., Divoux, D., Nouvelot, A., MacKenzie, E.T., and Petit, E. (1999). A potential role for erythropoietin in focal permanent cerebral ischemia in mice. J. Cereb. Blood Flow Metab. 19, 643-651.

Bihl, M.P., Heinimann, K., Rudiger, J.J., Eickelberg, O., Perruchoud, A.P., Tamm, M., and Roth, M. (2002). Identification of a novel IL-6 isoform binding to the endogenous IL-6 receptor. Am. J. Respir. Cell. Mol. Biol. 27, 48-56.
Bordet, T., Schmalbruch, H., Pettmann, B-, Hagege, A., Castelnau-Ptakhine, L., Kahn, A., and Haase, G. (1999). Adenoviral cardiotrophin-1 gene transfer protects pmn mice from progressive motor neuronopathy. J. Clin. Invest. 104, 1077-1085.
Boulanger, M.J., and Garcia, K.C. (2004). Shared cytokine signaling receptors: structural insights from the gp130 System. Adv. Protein Chem. 68, 107-146.
Brasier, A.R. (2006). The NF-κB regulatory network. Cardiovasc. Toxicol. 6, 111-130.
Bravo, J., and Heath, J. K. (2000). Receptor recognition by gp130 cytokines. Embo J, 19(11), 2399-2411.
Brines, M., Grasso, G., Fiordaliso, F., Sfacteria, A., Ghezzi, P., Fratelli, M., Latini, R., Xie, Q.W., Smart, J., Su-Rick, C.J., et al. (2004). Erythropoietin mediates tissue protection through an erythropoietin and common beta-subunit heteroreceptor. Proc. Natl. Acad. Sci. USA 101, 14907-14912.
Brines, M,L., Ghezzi, P., Keenan, S., Agnello, D., de Lanerolle, N.C., Cerami, C, Itri, L.M., and Cerami, A. (2000). Erythropoietin crosses the blood-brain barrier to protect against experimental brain injury. Proc. Natl. Acad. Sci. USA 97, 10526-10531.
Bruce, A. G., Hoggatt, I. H., and Rose, T. M. (1992). Oncostatin M is a differentiation factor for myeloid leukemia cells. J. Immuno. 149(4), 1271-1275.
Calvillo, L., Latini, R., Kajstura, J., Leri, A., Anversa, P., Ghezzi, P., Salio, M., Cerami, A., and Brines, M. (2003). Recombinant human erythropoietin protects the myocardium from ischemia-reperfusion injury and promotes beneficial remodeling. Proc. Natl. Acad. Sci. USA 100, 4802-4806.
Campana, W.M., and Myers, R.R. (2003). Exogenous erythropoietin protects against dorsal root ganglion apoptosis and pain following peripheral nerve injury. Eur. J. Neurosci. 18, 1497-1506.
Carpenter, M. K., Cui, X., Hu, Z. Y., Jackson, J., et al. (1999). In vitro expansion of a multipotent population of human neural progenitor cells. Exp. Neurol. 158(2), 265-278.
Celik, M., Gokmen, N., Erbayraktar, S., Akhisaroglu, M., Konake, S., Ulukus, C, Gene, S., Gene, K., Sagiroglu, E., Cerami, A., et al. (2002). Erythropoietin prevents motor neuron apoptosis and neurologic disability in experimental spinal cord ischemic injury. Proc. Natl. Acad. Sci. USA 99, 2258-2263.
Chang, M.Y., Park, C.H., Son, H., Lee, Y.S., and Lee, S.H. (2004). Developmental stage-dependent self-regulation of embryonic cortical precursor cell survival and differentiation by leukemia inhibitory factor. Cell Death Differ. 11, 985-996.
Cheetham, J.C., Smith, D.M., Aoki, K.H., Stevenson, J.L., Hoeffel, T.J., Syed, R.S., Egrie, J., and Harvey, T.S. (1998). NMR structure of human erythropoietin and a comparison with its receptor bound conformation. Nat. Struct. Biol. 5, 861-866.
Chikuma, M., Masuda, S., Kobayashi, T., Nagao, M., and Sasaki, R. (2000). Tissue-specific regulation of erythropoietin production in the murine kidney, brain, and uterus. Am. J. Physiol. 279, E1242-1248.
Chin, K" Yu, X., Beleslin-Cokic, B., Liu, C, Shen, K., Mohrenweiser, H.W., and Noguchi, CT. (2000). Production and processing of erythropoietin receptor transcripts in brain. Brain Res. 81, 29-42.
Chou, C.F., Tohari, S., Brenner, S., and Venkatesh, B. (2004). Erythropoietin gene from a teleost fish, Fugu rubripes. Blood 104, 1498-1503.
Derouet, D., Rousseau, F., Alfonsi, F., Froger, JL, Hermann, J., Barbier, F., Perret, D., Diveu, C, Guillet, C, Preisser, L., et al. (2004). Neuropoietin, a new IL-6-related cytokine signaling through the ciliary neurotrophic factor receptor. Proc. Natl. Acad. Sci. USA 101, 4827-4832.
Digicaylioglu, M., and Lipton, S.A. (2001). Erythropoietin-mediated neuroprotection involves cross-talk between Jak2 and NF-kappaB signalling cascades. Nature 412, 641-647.
Dolcet, X., Soler, R.M., Gould, T.W., Egea, J., Oppenheim, R.W., and Cornelia, J.X. (2001). Cytokines promote motoneuron survival through the Janus kinase-dependent activation of the phosphatidylinositol 3-kinase pathway. Mol. Cell. Neurosci. 18, 619-631.
Ehrenreich, H., Degner, D., Meiler, J., Brines, M., Behe, M., Hasselblatt, M-, Woldt, H., Falkai, P., Knerlich, F., Jacob, S., et al. (2004). Erythropoietin: a candidate compound for neuroprotection in schizophrenia. Mol. Psychiatry 9, 42-54.
Ehrenreich, H., Hasselblatt, M., Dembowski, C, Cepek, L., Lewczuk, P., Stiefel, M., Rustenbeck, H.H., Breiter, N., Jacob, S., Knerlich, F., et al, (2002). Erythropoietin therapy for acute stroke is both safe and beneficial. Mol. Med. 8, 495-505.
Elson, G. C., Lelievre, E., Guillet, C., Chevalier, S., et al. (2000). CLF associates with CLC to form a functional heteromeric ligand for the CNTF receptor complex. Nat. Neurosc. 3(9), 867-872.
Erbayraktar, S., Grasso, G., Sfacteria, A., Xie, Q.W., Coleman, T., Kreilgaard, M., Torup, L., Sager, T., Erbayraktar, Z., Gokmen, N., et al. (2003). Asialoerythropoietin is a nonerythropoietic cytokine with broad neuroprotective activity in vivo. Proc. Natl. Acad. Sci. USA 700,6741-6746.

Garofalo, R.S, Orena, S.J., Rafidi, K., Torchia, A.J., Stock, J.L., Hildebrandt, A.L., Coskran, T., Black, S.C., Brees, D.J., Wicks, J.R., McNeish, J.D., and Coleman K.G., (2003). Severe diabetes, age-dependent loss of adipose tissue, and mild growth deficiency in mice lacking Akt2/PKB beta. Journal of Clinical Investigation 112, 197-208.

Gearing, D.P., Comeau, M.R., Friend, DJ., Gimpel, S.D., Thut, C.J., McGourty, J" Brasher, K.K., King, J.A., Gillis, S., Mosley, B., et al. (1992). The IL-6 signal transducer, gp130: an oncostatin M receptor and affinity converter for the LIF reeeptor. Science 255, 1434-1437.

Gilmore, T.D. (1999). The Rel/NF-κB signal transduction pathway: introduction. Oncogene 18, 6842-6844.

Gilmore, T.D. (2006). Introduction to NF-κB: players, pathways, perspectives. Oncogene 25, 6680-6684.

Goldberg, M.A., Dunning, S.P., and Bunn, H.F. (1988). Regulation of the erythropoietin gene: evidence that the oxygen sensor is a heme protein. Science 242, 1412-1415.

Ghosh, S., May, M.J., Kopp, E.B. (1998). NF-κB and Rel proteins: evolutionarily conserved mediators of immune responses. Annu. Rev. Immunol. 16, 225-260.

Hisaka, T., Desmouliere, A., Taupin, J.L., Daburon, S., Neaud, V., Senant, N., Blanc, J.F., Moreau, J.F., and Rosenbaum, J. (2004). Expression of leukemia inhibitory factor (LIF) and its receptor gp190 in human liver and in cultured human liver myofibroblasts. Cloning of new isoforms of LIF mRNA. Comp. Hepatol. 3, 10.

Ihle, J.N. (1995). The Janus protein tyrosine kinase family and its role in cytokine signaling. Adv. Immunol. 60, 1-35.

Ikeda, K., Iwasaki, Y., Shiojima, T., and Kinoshita, M. (1996). Neuroprotective effect of various cytokines on developing spinal motoneurons following axotomy. J. Neurol. Sci. 135, 109-113.

Jacobs, M.D., and Harrison, S.C. (1998). Structure of an IkappaBalpha/NF-kappaB complex. Cell 95, 749-758.

Jubinsky, P.T., Krijanovski, O.I., Nathan, D.G., Tavernier, J., and Sieff, CA. (1997). The beta chain of the interleukin-3 receptor functionally associates with the erythropoietin receptor. Blood 90, 1867-1873.

Karin, M. (1999). How NF-kappaB is activated: the role of the IkappaB kinase (IKK) complex. Oncogene 18, 6867-6874.

Karin, M., and Ben-Neriah, Y. (2000). Phosphorylation meets ubiquitination: the control of NF-κB activity. Annu. Rev. Immunol. 18: 621-663.

Kaushansky, K., and Karplus, P.A. (1993). Hematopoietic growth factors: understanding functional diversity in structural terms. Blood 82, 3229-3240.

Kestler, D.P., Goldstein, K.M., Agarwal, S., Fuhr, J.E., Andrews, R., and Hall, R.E. (1999). Hematopoietic differentiation activity of a recombinant human interleukin-6 (IL-6) isoform resulting from alternatively spliced deletion of the second exon. Am. J. Hematol. 61, 169-177.

Keswani, S.C, Leitz, G.J., and Hoke, A. (2004). Erythropoietin is neuroprotective in models of HIV sensory neuropathy. Neurosci. Lett. 371, 102-105.

Kisseleva et al (2002). Signaling through the JAK/STAT pathway, recent advances and future challenges. Gene 285, 1-24.

Lam, K. (1997). Mini-review. Application of combinatorial library methods in cancer research and drug discovery. Anticancer Drug Design 12, 145-167.

Leist, M., Ghezzi, P., Grasso, G., Bianchi, R., Villa, P., Fratelli, M., Savino, C, Bianchi, M., Nielsen, J., Gerwien, J., et al. (2004). Derivatives of erythropoietin that are tissue protective but not erythropoietic. Science 305, 239-242.

Livnah, O., Stura, E.A., Middleton, S.A., Johnson, D.L., Jolliffe, L.K., and Wilson, I.A. (1999). Crystallographic evidence for preformed dimers of erythropoietin receptor before ligand activation. Science 283, 987-990.

Lo, A.C., Li, L., Oppenheim, R.W., Prevette, D., and Houenou, L.J. (1995). Ciliary neurotrophic factor promotes the survival of spinal sensory neurons following axotomy but not during the period of programmed cell death. Exp. Neurol. 134, 49-55.

Marti, H.H., Wenger, R.H., Rivas, L.A., Straumann, U., Digicaylioglu, M, Henn, V., Yonekawa, Y., Bauer, C., and Gassmann, M. (1996). Erythropoietin gene expression in human, monkey and murine brain. Eur. J. Neurosci. 8, 666-676.

Meazza, R., Verdiani, S., Biassoni, R., Coppolecchia, M., Gaggero, A., Orengo, A.M., Colombo, M.P., Azzarone, B., and Ferrini, S. (1996). Identification of a novel interleukin-15 (IL-15) transcript isoform generated by alternative splicing in human small cell lung cancer cell lines. Oncogene 12, 2187-2192.

Mercurio, F., Zhu, H., Murray, B.W., Shevchenko, A., Bennett, B.L., Li, J., Young, D.B., Barbosa, M., Mann, M., Manning, A., Rao, A. (1997). IKK-1 and IKK-2: cytokine-activated IkappaB kinases essential for NF-kappaB activation. Science 278, 860-866.

Metcalf, D. (2008). Hematopoietic cytokines. Blood 111, 485-491.

Middleton, G., Hamanoue, M., Enokido, Y., Wyatt, S., Pennica, D., JafTray, E., Hay, R.T., and Davies, A.M. (2000). Cytokine-induced nuclear factor kappa B activation promotes the survival of developing neurons. J. Cell Biol. 148, 325-332.

Miyake, T., Kung, C.K., and Goldwasser, E. (1977). Purification of human erythropoietin. J. Biol. Chem. 252, 5558-5564.

Mosley, B., De Imus, C., Friend, D., Boiani, N., et al. (1996). Dual oncostatin M (OSM) receptors. Cloning and characterization of an alternative signaling subunit conferring OSM-specific receptor activation. J. Biol. Chem. 271 (51), 32635-32643.

Murphy, M., Reid, K., Hilton, D. J., & Bartlett, P. F. (1991). Generation of sensory neurons is stimulated by leukemia inhibitory factor. Proc. Natl. Acad. Sci. USA 88(8), 3498-3501.

Murphy, M., Dutton, R., Koblar, S., Cheema, S., and Bartlett, P. (1997). Cytokines which signal through the LIF receptor and their actions in the nervous System. Prog. Neurobiol. 52, 355-378.

Nakamura, Y., Komatsu, N., and Nakauchi, H. (1992). A truncated erythropoietin receptor that fails to prevent programmed cell death of erythroid cells. Science 257, 1138-1141.

Noguchi, CT., Asavaritikrai, P., Teng, R., and Jia, Y. (2007). Role of erythropoietin in the brain. Crit Rev. Oncol. Hematol. 64, 159-171.

Ochiai, W., Yanagisawa, M., Takizawa, T., Nakashima, K., et al. (2001). Astrocyte differentiation of fetal neuroepithelial cells involving cardiotrophin-1-induced activation of STAT3. Cytokine 14(5), 264-271.

Ohno, M., and Abe, T. (1991). Rapid colorimetric assay for the quantification of leukemia inhibitory factor (LIF) and interleukin-6 (IL-6). J. Immunol. Method. 145(1-2), 199-203.

Ohno. M., Kohyama, J., Namihira, M., Sanosaka, T., et al. (2006). Neuropoietin induces neuroepithelial cells to differentiate into astrocytes via activation of STAT3. Cytokine 36(1-2), 17-22.

Parsa, C.J., Matsumoto, A., Kim, J., Riel, R.U., Pascal, L.S., Walton, G.B., Thompson, R.B., Petrofski, J.A., Annex, B.H., Stamler, J.S., et al. (2003). A novel protective effect of erythropoietin in the infarcted heart. J. Clin. Invest. 112, 999-1007.

Pennica, D., King, K. L., Shaw, K. J., Luis, E., et al. (1995). Expression cloning of cardiotrophin 1, a cytokine that induces cardiac myocyte hypertrophy. Proc. Natl. Acad. Sci. USA 92(4), 1142-1146.

Perkins, N.D. (2007). Integrating cell-signalling pathways with NF-κB and IKK function. Nat. Rev. Mol. Cell Biol. 8, 49-62.

Prass, K., Scharff, A., Ruscher, K., Lowl, D., Muselmann, C, Victorov, I., Kapinya, K., Dirnagl, U., and Meisel, A. (2003). Hypoxia-induced stroke tolerance in the mouse is mediated by erythropoietin. Stroke 34, 1981-1986.

Puskovic, V., Wolfe, D., Wechuck, J., Krisky, D., Collins, J., Glorioso, J.C., Fink, DJ., and Mata, M. (2006). HSV-mediated delivery of erythropoietin restores dopaminergic function in MPTP-treated mice. Mol. Ther. 14, 710-715.

Rajan, P., and McKay, R. D. (1998). Multiple routes to astrocytic differentiation in the CNS. J Neurosc. 18(10), 3620-3629.

Ramaswamy, S., Nakamura, N., Vazquez, F., Batt, D.B., Perera, S., Roberts, T.M., Sellers, W.R. (1999). Regulation of G1 progression by the PTEN tumor suppressor protein is linked to inhibition of the phosphatidylinositol 3-kinase/Akt pathway. Proc. Natl. Acad. Sci. USA 96, 2110-2115.

Régnier, C.H., Song, H.Y., Gao, X., Goeddel, D.V., Cao, Z., Rothe, M. (1997). Identification and characterization of an IkappaB kinase. Cell 90, 373-83.

Garofalo, R.S., Orena, S.J., Rafidi, K., Torchia, A.J., Stock, J.L., Hildebrandt, A.L., Coskran, T., Black, S.C., Brees, D.J.,. Wicks, J.R., McNeish, J.D., and Coleman K.G. (2003). Severe diabetes, age-dependent loss of adipose tissue, and mild growth deficiency in mice lacking Akt2/PKB beta Journal of Clinical Investigation 112, 197-208.

Robledo, O., Fourcin, M., Chevalier, S., Guillet, C., et al. (1997). Signaling of the cardiotrophin-1 receptor. Evidence for a third receptor component. J. Biol. Chem. 272(8), 4855-4863.

Rose, T. M., Weiford, D. M., Gunderson, N. L., and Bruce, A. G. (1994). Oncostatin M (OSM) inhibits the differentiation of pluripotent embryonic stem cells in vitro. Cytokine 6(1), 48-54.

Ruscher, K., Freyer, D., Karsch, M., Isaev, N., Megow, D., Sawitzki, B., Priller, J., Dirtiagl, U., and Meisel, A. (2002). Erythropoietin is a paracrine mediator of ischemic tolerance in the brain: evidence from an in vitro model. J. Neurosci. 22, 10291-10301.

Sakanaka, M, Wen, T.C., Matsuda, S., Masuda, S., Morishita, E., Nagao, M., and Sasaki, R. (1998). In vivo evidence that erythropoietin protects neurons from ischemic damage. Proc. Natl. Acad. Sci. USA 95, 4635-4640.

Sattler, M.B., Merkler, D., Maier, K., Stadelmann, C, Ehrenreich, H., Bahr, M, and Diem, R. (2004). Neuroprotective effects and intracellular signaling pathways of erythropoietin in a rat model of multiple sclerosis. Cell Death Differ. 11 Suppl 2, S181-192.

Schuettauf, F., Zurakowski, D., Quinto, K., Varde, M.A., Besch, D., Laties, A., Anderson, R., and Wen, R. (2005). Neuroprotective effects of cardiotrophin-like cytokine on retinal ganglion cells. Graefes Arch. Clin. Exp. Ophthalmo1. 243, 1036-1042.

Senftleben U, Cao Y, Xiao G, Greten FR, Krähn G, Bonizzi G, Chen Y, Hu Y, Fong A, Sun SC, Karin M (2001). Activation by IKKalpha of a second, evolutionary conserved, NF-κB signaling pathway. Science 293, 1495-1499.

Siren, A.L., Fratelli, M., Brines, M., Goemans, C, Casagrande, S., Lewczuk, P., Keenan, S., Gleiter, C, Pasquali, C, Capobianco, A., et al. (2001). Erythropoietin prevents neuronal apoptosis after cerebral ischemia and metabofic stress. Proc. Natl. Acad. Sci. USA 98, 4044-4049.

Sorg, R.V., Enczmann, J., Sorg, U.R., Schneider, E.M., and Wernet, P. (1993). Identification of an altematively spliced transcript of human interleukin-4 lacking the sequence encoded by exon 2. Experimental hematology 21, 560-563.

Statler, P.A., McPherson, R.J., Bauer, L.A., Kellert, B.A., and Juul, S.E. (2007). Pharmacokinetics of high-dose recombinant erythropoietin in plasma and brain of neonatal rats. Pediatr. Res. 61, 671-675.

Tian, B., Brasier, A.R. (2003). Identification of a nuclear factor κ B-dependent gene network. Recent Prog. Horm. Res. 58, 95-130.

Tsai, P. T., Ohab, J. J., Kertesz, N., Groszer, M., et al. (2006). A critical role of erythropoietin receptor in neurogenesis and post-stroke recovery. J Neurosci. 26(4), 1269-1274.

Uemura, A., Takizawa, T., Ochiai, W., Yanagisawa, M., et al. (2002). Cardiotrophin-like cytokine induces astrocyte differentiation of fetal neuroepithelial cells via activation of STAT3. Cytokine 18(1), 1-7.

Vesey, D.A., Cheung, C, Pat, B., Endre, Z., Gobe, G., and Johnson, D.W. (2004). Erythropoietin protects against ischaemic acute renal injury. Nephrol. Dial. Transplant 19, 348-355.

Wang, L., Zhang, Z. G., Gregg, S. R., Zhang, R. L., et al. (2007). The Sonic hedgehog pathway mediates carbamylated erythropoietin-enhanced proliferation and differentiation of adult neural progenitor cells. J. Biol. Chem. 282(44), 32462-32470.

Weiss, T.W., Samson, A.L., Niego, B., Daniel, P.B., and Medcalf, R.L. (2006). Oncostatin M is a neuroprotective cytokine that inhibits excitotoxic injury in vitro and in vivo. FASEB J. 20,2369-2371.

Wen, D., Boissel, J.P., Showers, M, Ruch, B.C., and Bunn, H.F. (1994). Erythropoietin structure-function relationships. Identification of functionally important domains. J. Biol. Chem. 269, 22839-22846.

Witthuhn, B.A., Quelle, F.W., Silvennoinen, O., Yi, T., Tang, B., Miura, O., and Ihle, J.N. (1993). JAK2 associates with the erythropoietin receptor and is tyrosine phosphorylated and activated following stimulation with erythropoietin. Cell 74, 227-236.

Wu, H., Liu, X., Jaenisch, R., and Lodish, H.F. (1995). Generation of committed erythroid BFU-E and CFU-E progenitors does not require erythropoietin or the erythropoietin receptor. Cell 83, 59-67.

Yatsenko, O.P., Filipenko, M.L., Khrapov, E.A., Voronina, E.N., Kozlov, V.A., and Sennikov, S.V. (2004). Alternative splicing of mRNA of mouse interleukin-4 and interleukin 6. Cytokine 28, 190-196.

Yoshida, K., Taga, T., Saito, M., Suematsu, S., et al. (1996). Targeted disruption of gp130, a common signal transducer for the interleukin 6 family of cytokines, leads to myocardial and hematological disorders. Proc. Natl. Acad. Sci. USA 93(1), 407-411.

Yu, X., Shacka, J.J., Eells, J.B., Suarez-Quian, C, Przygodzki, R.M., Beleslin-Cokic, B., Lin, C.S., Nikodem, V.M., Hempstead, B., Flanders, K.C., et al. (2002). Erythropoietin receptor signalling is required for normal brain development. Development 129, 505-516.

Yang, Z.Z., Tschopp, O., Baudry, A., Dummler, B., Hynx, D.,and Hemmings, B.A. (2004) Physiological functions of protein kinase B/Akt. Biochem. Soc Trans. 32:350-354.

## Claims

1. Use of a protein or protein complex comprising glycoprotein 130 (gp130) and/or leukemia inhibitory factor receptor alpha-chain (LIFRα) for the identification of a cytoprotective compound.

**2.** Use of claim 1, wherein the protein or protein complex comprises or consists of a receptor of the formula:

$$(X / gp130 / Y)_n,$$

wherein
X is

- CNTFRα or
- gp80 or
- absent,

Y is

- leukemia inhibitory factor receptor alpha-chain (LIFRα) or
- interleukin 6 receptor (IL-6 R) or
- interleukin 11 receptor (IL-11 R) or
- interleukin 27 receptor (IL-27 R) or
- oncostatin M receptor α (OSMRα),

and n is 1 or 2.

**3.** Use of claim 1 or 2, wherein the protein or protein complex comprises or consists of gp130 and LIFRα, particularly of a receptor of the formula:

$$X / gp130 / LIFR\alpha$$

wherein X is CNTFRα or gp80 or absent.

**4.** A method of screening for a potential cytoprotective compound comprising

(a) contacting a protein or protein complex as defined in claims 1 to 3 with a substance; and
(b) detecting interaction of the substance with the protein or protein complex, whereby identifying the substance as a potential cytoprotective compound.

**5.** The method of claim 4, wherein the method further comprises

(c) contacting a cell with the potential cytoprotective compound identified in step (b); and
(d) detecting a cytoprotective effect of the potential cytoprotective compound on the cell, whereby identifying the potential cytoprotective compound as cytoprotective compound.

**6.** The method of claim 5, wherein the cell expressing the protein or protein complex as defined in claims 1 to 3, is selected from the group consisting of a myoblast (such as rat heart myoblast cell line H9c2), a neuronal cell, a neural stem cell, a neural progenitor cell, a murine pro-B IL-3 cell (Ba/F3 cells), and a myeloid leukemia cell (such as mouse myeloid leukemia cell line M1), a hematopoietic stem cell, a hematopoietic precursor cell and a mesodermal stromal (stem) cell.

**7.** The method of any of claims 4 to 6, wherein the cytoprotective effect of the potential cytoprotective compound on the cell is determined by detecting viability or survival of the contacted cell, optionally under conditions being disadvantageous to the cell.

**8.** The method of any of claims 4 to 6, wherein the cytoprotective effect of the potential cytoprotective compound on the cell is determined by detecting a component downstream in the signal cascade of the protein, particularly Janus kinase 2 (Jak2), Janus kinase 1 (Jak1), Janus kinase 3 (Jak3), protein kinase B (Akt), IκB kinase (IKK) and/or nuclear factor-kappa B (NF-κB).

**9.** The use of any of claims 1 to 3 or the method of any of claims 4 to 8, wherein the protein or protein complex is a naturally occurring receptor.

**10.** The use of any of claims 1 to 3 or 9 or the method of any of claims 4 to 9, wherein the identifying as a (potential) cytoprotective compound is by detecting binding of the compound to the protein or protein complex, particularly to gp130 and/or LIFRα.

**11.** The use of any of claims 1 to 3, 9 or 10 or the method of any of claims 4 to 10, wherein the compound is neuroprotective and/or modulates differentiation in a neuronal glial lineage.

**12.** The use of any of claims 1 to 3 or 9 to 11 or the method of any of claims 4 to 11, wherein the compound is not hematopoietic.

**13.** The use of any of claims 1 to 3 or 9 to 12 or the method of any of claims 4 to 12, wherein the protein binds to and optionally promotes cytoprotective effects of an erythropoietin (EPO) variant (vEPO).

**14.** The use of claim 13 or the method of claim 13, wherein vEPO is hS3, hS4 or mS.

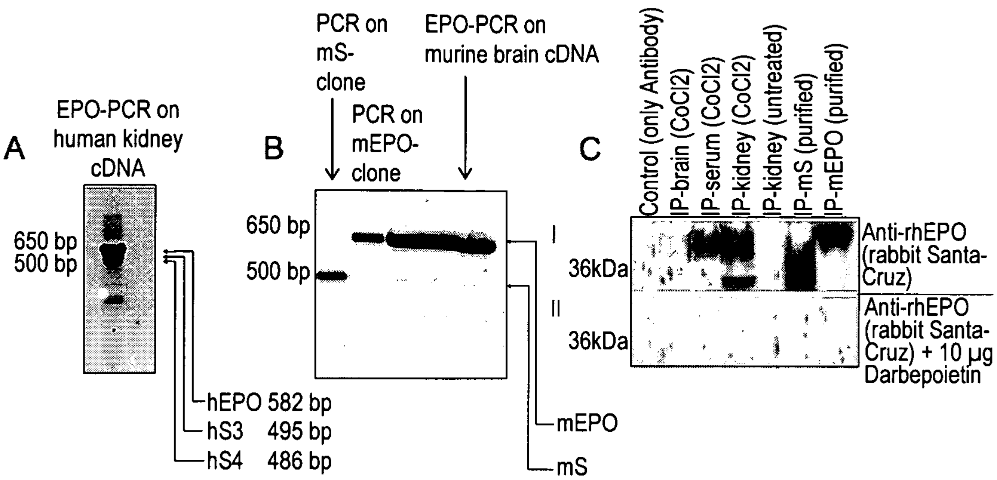


**D**

```
hEPO  (SEQ ID NO:29)   ATGGGGGTGCACGAATGTCCTGCCTGGCTGTGGCTTCTCCTGTCCCTGCTGTCGCTCCCT 60
hS3   (SEQ ID NO:30)   ATGGGGGTGCACGAATGTCCTGCCTGGCTGTGGCTTCTCCTGTCCCTGCTGTCGCTCCCT 60
hS4   (SEQ ID NO:31)   ATGGGGGTGCACGAATGTCCTGCCTGGCTGTGGCTTCTCCTGTCCCTGCTGTCGCTCCCT 60
                       ************************************************************

hEPO                   CTGGGCCTCCCAGTCCTGGGCGCCCCACCACGCCTCATCTGTGACAGCCGAGTCCTGGAG 120
hS3                    CTGGGCCTCCCAGTCCTGGGCGCCCCACCACGCCTCATCTGTGACAGCCGAGTCCTGGAG 120
hS4                    CTGGGCCTCCCAGTCCTGGGCGCCCCACCACGCCTCATCTGTGACAGCCGAGTCCTGGAG 120
                       ************************************************************

hEPO                   AGGTACCTCTTGGAGGCCAAGGAGGCCGAGAATATCACGACGGGCTGTGCTGAACACTGC 180
hS3                    AGGTACCTCTTGGAGGCCAAGGAGGCCGAGAATATCACG--------------------- 159
hS4                    AGGTACCTCTTGGAGGCCAAGGAGGCCGAGAATATCACGACGGGCTGTGCTGAACACTGC 180
                       ***************************************

hEPO                   AGCTTGAATGAGAATATCACTGTCCCAGACACCAAAGTTAATTTCTATGCCTGGAAGAGG 240
hS3                    ------------------------------------------------------------ 159
hS4                    AGCTTGAATGAGAATATCACTGTCCCAGACACCAAAGTTAATTTCTATGCCTGGAAGAGG 240

hEPO                   ATGGAGGTCGGGCAGCAGGCCGTAGAAGTCTGGCAGGGCCTGGCCCTGCTGTCGGAAGCT 300
hS3                    ------GTCGGGCAGCAGGCCGTAGAAGTCTGGCAGGGCCTGGCCCTGCTGTCGGAAGCT 213
hS4                    ATGGAG------------------------------------------------------ 246

hEPO                   GTCCTGCGGGGCCAGGCCCTGTTGGTCAACTCTTCCCAGCCGTGGGAGCCCCTGCAGCTG 360
hS3                    GTCCTGCGGGGCCAGGCCCTGTTGGTCAACTCTTCCCAGCCGTGGGAGCCCCTGCAGCTG 273
hS4                    ---------------------------------------CCGTGGGAGCCCCTGCAGCTG 267
                                                               *********************

hEPO                   CATGTGGATAAAGCCGTCAGTGGCCTTCGCAGCCTCACCACTCTGCTTCGGGCTCTGGGA 420
hS3                    CATGTGGATAAAGCCGTCAGTGGCCTTCGCAGCCTCACCACTCTGCTTCGGGCTCTGGGA 333
hS4                    CATGTGGATAAAGCCGTCAGTGGCCTTCGCAGCCTCACCACTCTGCTTCGGGCTCTGGGA 327
                       ************************************************************

hEPO                   GCCCAGAAGGAAGCCATCTCCCCTCCAGATGCGGCCTCAGCTGCTCCACTCCGAACAATC 480
hS3                    GCCCAGAAGGAAGCCATCTCCCCTCCAGATGCGGCCTCAGCTGCTCCACTCCGAACAATC 393
hS4                    GCCCAGAAGGAAGCCATCTCCCCTCCAGATGCGGCCTCAGCTGCTCCACTCCGAACAATC 387
                       ************************************************************

hEPO                   ACTGCTGACACTTTCCGCAAACTCTTCCGAGTCTACTCCAATTTCCTCCGGGGAAAGCTG 540
hS3                    ACTGCTGACACTTTCCGCAAACTCTTCCGAGTCTACTCCAATTTCCTCCGGGGAAAGCTG 453
hS4                    ACTGCTGACACTTTCCGCAAACTCTTCCGAGTCTACTCCAATTTCCTCCGGGGAAAGCTG 447
                       ************************************************************

hEPO                   AAGCTGTACACAGGGGAGGCCTGCAGGACAGGGGACAGATGA 582
hS3                    AAGCTGTACACAGGGGAGGCCTGCAGGACAGGGGACAGATGA 495
hS4                    AAGCTGTACACAGGGGAGGCCTGCAGGACAGGGGACAGATGA 489
                       ******************************************
```

**Fig. 1 (part 1)**

```
mEPO (SEQ ID NO:32)  ATGGGGGTGCCCGAACGTCCCACCCTGCTGCTTTTACTCTCCTTGCTACTGATTCCTCTG 60
mS   (SEQ ID NO:33)  ATGGGGGTGCCCGAACGTCCCACCCTGCTGCTTTTACTCTCCTTGCTACTGATTCCTCTG 60
                     ************************************************************

mEPO                 GGCCTCCCAGTCCTCTGTGCTCCCCCACGCCTCATCTGCGACAGTCGAGTTCTGGAGAGG 120
mS                   GGCCTCCCAGTCCTCTGTGCTCCCCCACGCCTCATCTGCGACAGTCGAGTTCTGGAGAGG 120
                     ************************************************************

mEPO                 TACATCTTAGAGGCCAAGGAGGCAGAAAATGTCACGATGGGTTGTGCAGAAGGTCCCAGA 180
mS                   TACATCTTAGAGGCCAAGGAGGCAGAAAATGTCACGATGGGTTGTGCAGAAGGTCCCAGA 180
                     ************************************************************

mEPO                 CTGAGTGAAAATATTACAGTCCCAGATACCAAAGTCAACTTCTATGCTTGGAAAAGAATG 240
mS                   CTGAGTGAAAATATTACAGTCCCAGATACCAAAGTCAACTTCTATGCTTGGAAAAGAATG 240
                     ************************************************************

mEPO                 GAGGTGGAAGAACAGGCCATAGAAGTTTGGCAAGGCCTGTCCCTGCTCTCAGAAGCCATC 300
mS                   GAG--------------------------------------------------------- 243
                     ***

mEPO                 CTGCAGGCCCAGGCCCTGCTAGCCAATTCCTCCCAGCCACCAGAGACCCTTCAGCTTCAT 360
mS                   ------------------------------------------------------------ -

mEPO                 ATAGACAAAGCCATCAGTGGTCTACGTAGCCTCACTTCACTGCTTCGGGTACTGGGAGCT 420
mS                   ------------------------------------------------------------ -

mEPO                 CAGAAGGAATTGATGTCGCCTCCAGATACCACCCCACCTGCTCCACTCCGAACACTCACA 480
mS                   ---AAGGAATTGATGTCGCCTCCAGATACCACCCCACCTGCTCCACTCCGAACACTCACA 300
                     *********************************************************

mEPO                 GTGGATACTTTCTGCAAGCTCTTCCGGGTCTACGCCAACTTCCTCCGGGGGAAACTGAAG 540
mS                   GTGGATACTTTCTGCAAGCTCTTCCGGGTCTACGCCAACTTCCTCCGGGGGAAACTGAAG 360
                     ************************************************************
mEPO                 CTGTACACGGGAGAGGTCTGCAGGAGAGGGGACAGGTGA 579
mS                   CTGTACACGGGAGAGGTCTGCAGGAGAGGGGACAGGTGA 399
                     ***************************************
```

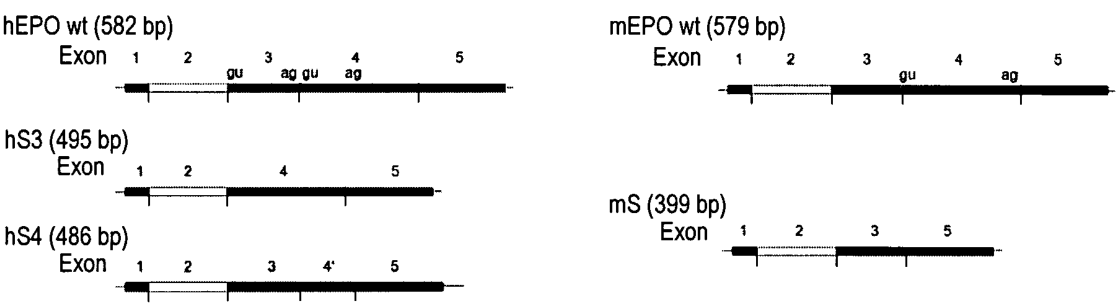


Fig. 1 (part 2)

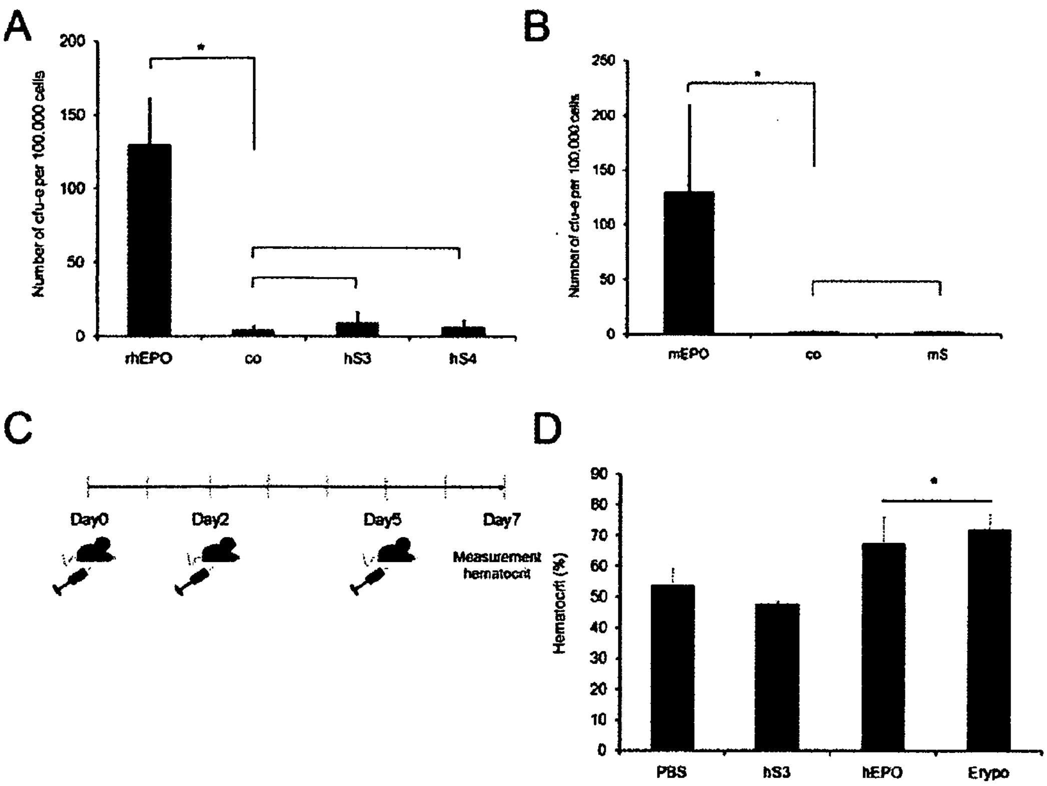
A
B
C
D
Number of cfu-e per 100,000 cells
rhEPO
co
hS3
hS4
mEPO
mS
Day0
Day2
Day5
Day7
Measurement hematocrit
Hematocrit (%)
PBS
hEPO
Erypo

**Fig. 2**

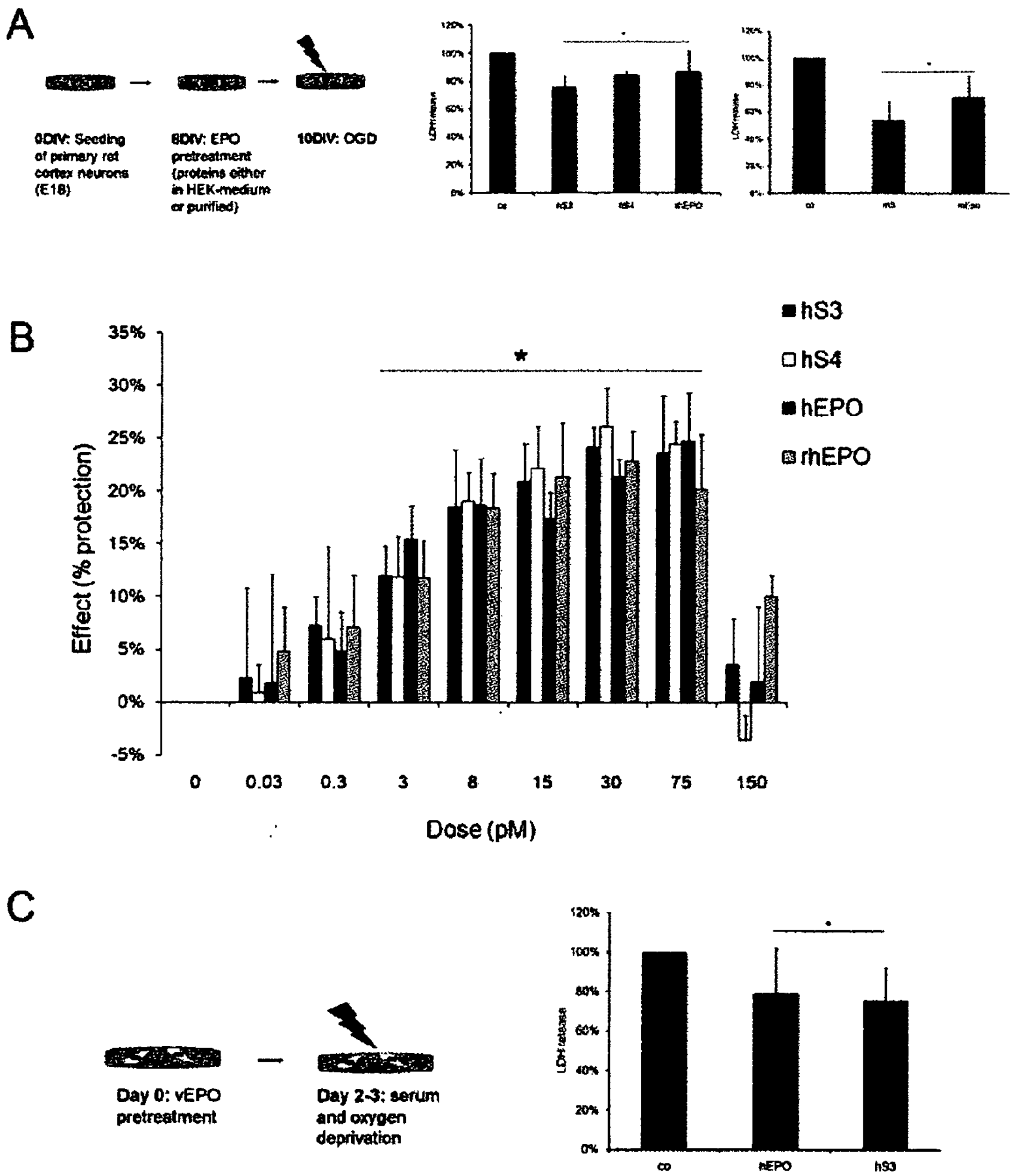
A
0DIV: Seeding of primary rat cortex neurons (E18)
8DIV: EPO pretreatment (proteins either in HEK-medium or purified)
10DIV: OGD
B
hS3
hS4
hEPO
rhEPO
Effect (% protection)
35%
30%
25%
20%
15%
10%
5%
0%
-5%
0
0.03
0.3
3
8
15
30
75
150
Dose (pM)
C
Day 0: vEPO pretreatment
Day 2-3: serum and oxygen deprivation
120%
100%
80%
60%
40%
20%
0%
LDH release
co
hEPO
hS3

**Fig. 3**

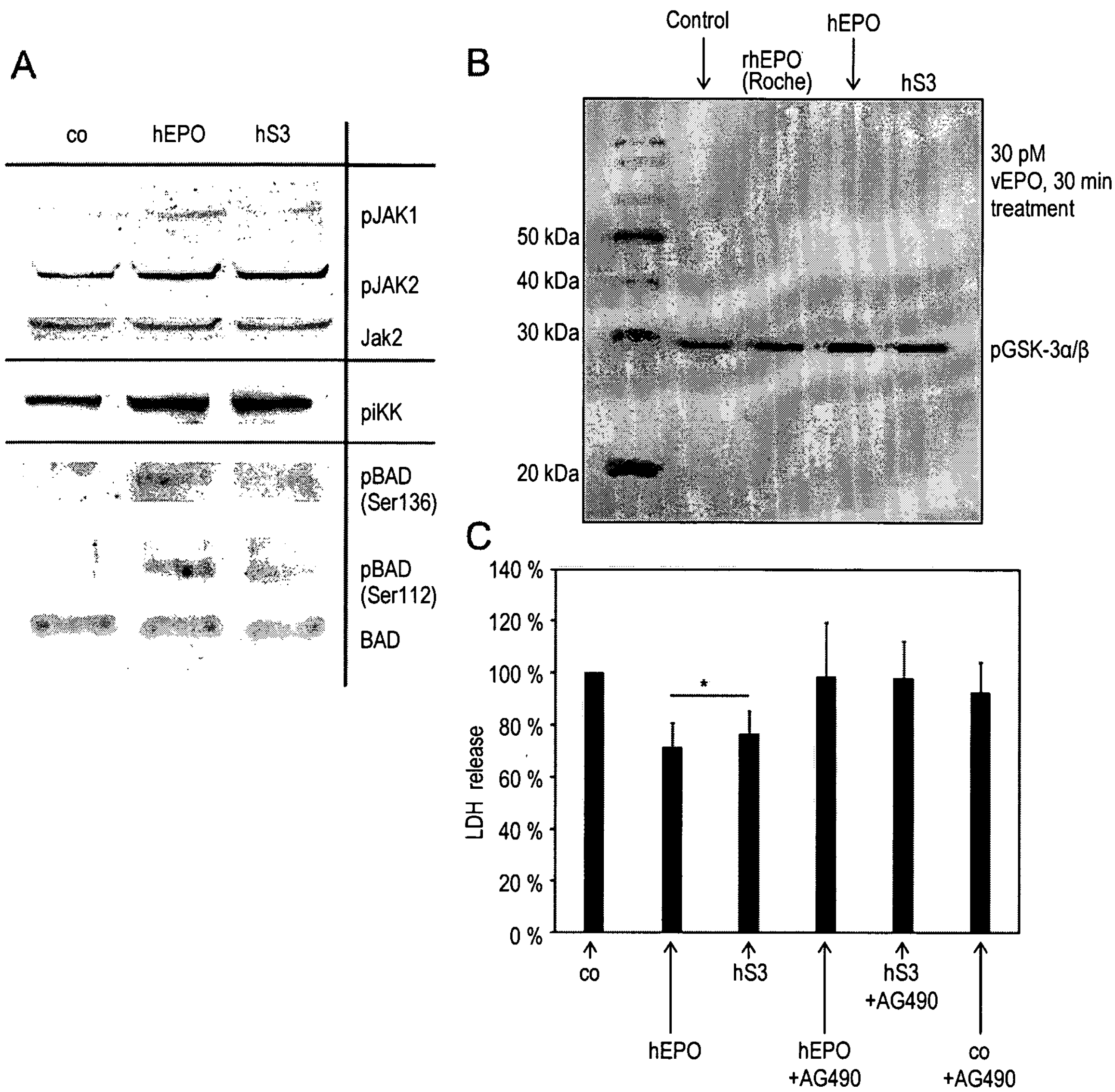
A
co
hEPO
hS3
pJAK1
pJAK2
Jak2
piKK
pBAD (Ser136)
pBAD (Ser112)
BAD
B
Control
rhEPO (Roche)
hEPO
hS3
30 pM vEPO, 30 min treatment
50 kDa
40 kDa
30 kDa
20 kDa
pGSK-3α/β
C
140 %
120 %
100 %
80 %
60 %
40 %
20 %
0 %
LDH release
*
co
hEPO
hS3
hEPO +AG490
hS3 +AG490
co +AG490

Fig. 4

**A**

```
            Leader Sequence   1      10      20      30      40      50      60      70

hEPO   MGVHECPAWLWLLLSLLSLPLGLPVLGAPPRLICDSRVLERYLLEAKEAENITTGCAEHCSLNENITVPDTKVNFYAWKRMEVGQQAVEVWQGLALL
hS3    MGVHECPAWLWLLLSLLSLPLGLPVLGAPPRLICDSRVLERYLLEAKEAENIT-----------------------------VGQQAVEVWQGLALL
hS4    MGVHECPAWLWLLLSLLSLPLGLPVLGAPPRLICDSRVLERYLLEAKEAENITTGCAEHCSLNENITVPDTKVNFYAWKRME---------------

mEPO   MGVPERP-TLLLLLSLLLIPLGLPVLCAPPRLICDSRVLERYILEAKEAENVTMGCAEGPRLSENITVPDTKVNFYAWKRMEVEEQAIEVWQGLSLL
mS     MGVPERP-TLLLLLSLLLIPLGLPVLCAPPRLICDSRVLERYILEAKEAENVTMGCAEGPRLSENITVPDTKVNFYAWKRME---------------

           80      90      100     110     120     130     140     150     160

hEPO   SEAVLRGQALLVNSSQPWEPLQLHVDKAVSGLRSLTTLLRALGAQKEAISPPDAASAAPLRTITADTFRKLFRVYSNFLRGKLKLYTGEACRTGDR
hS3    SEAVLRGQALLVNSSQPWEPLQLHVDKAVSGLRSLTTLLRALGAQKEAISPPDAASAAPLRTITADTFRKLFRVYSNFLRGKLKLYTGEACRTGDR
hS4    ----------------PWEPLQLHVDKAVSGLRSLTTLLRALGAQKEAISPPDAASAAPLRTITADTFRKLFRVYSNFLRGKLKLYTGEACRTGDR

mEPO   SEAILQAQALLANSSQPPETLQLHIDKAISGLRSLTSLLRVLGAQKELMSPPDTTPPAPLRTLTVDTFCKLFRVYANFLRGKLKLYTGEVCRRGDR
mS     ---------------------------------------------KELMSPPDTTPPAPLRTLTVDTFCKLFRVYANFLRGKLKLYTGEVCRRGDR
```

hEPO: SEQ ID NO 1; hS3: SEQ ID NO 2; hS4: SEQ ID NO 3; mEPO: SEQ ID NO 4; mS: SEQ ID NO 5

**B**

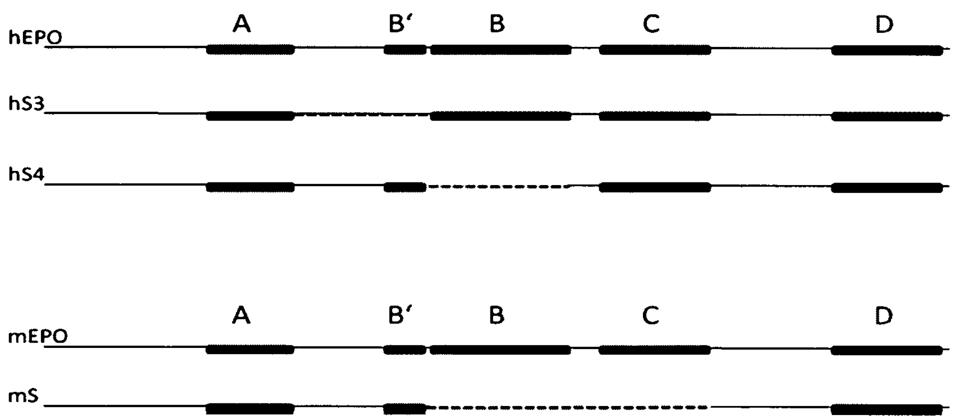


**C**

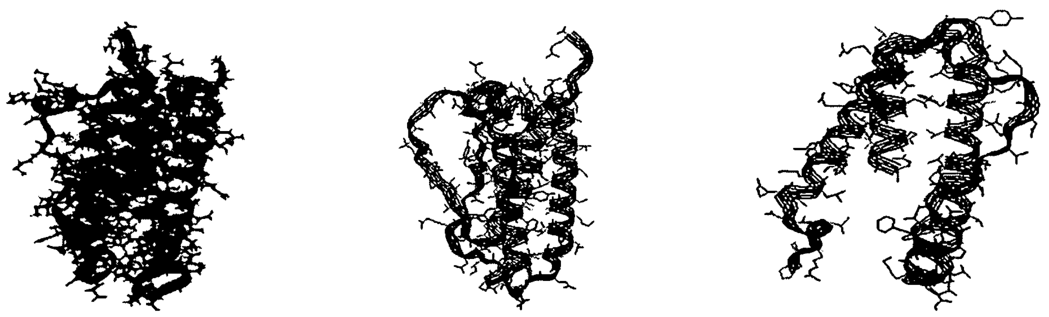

**Fig. 5**

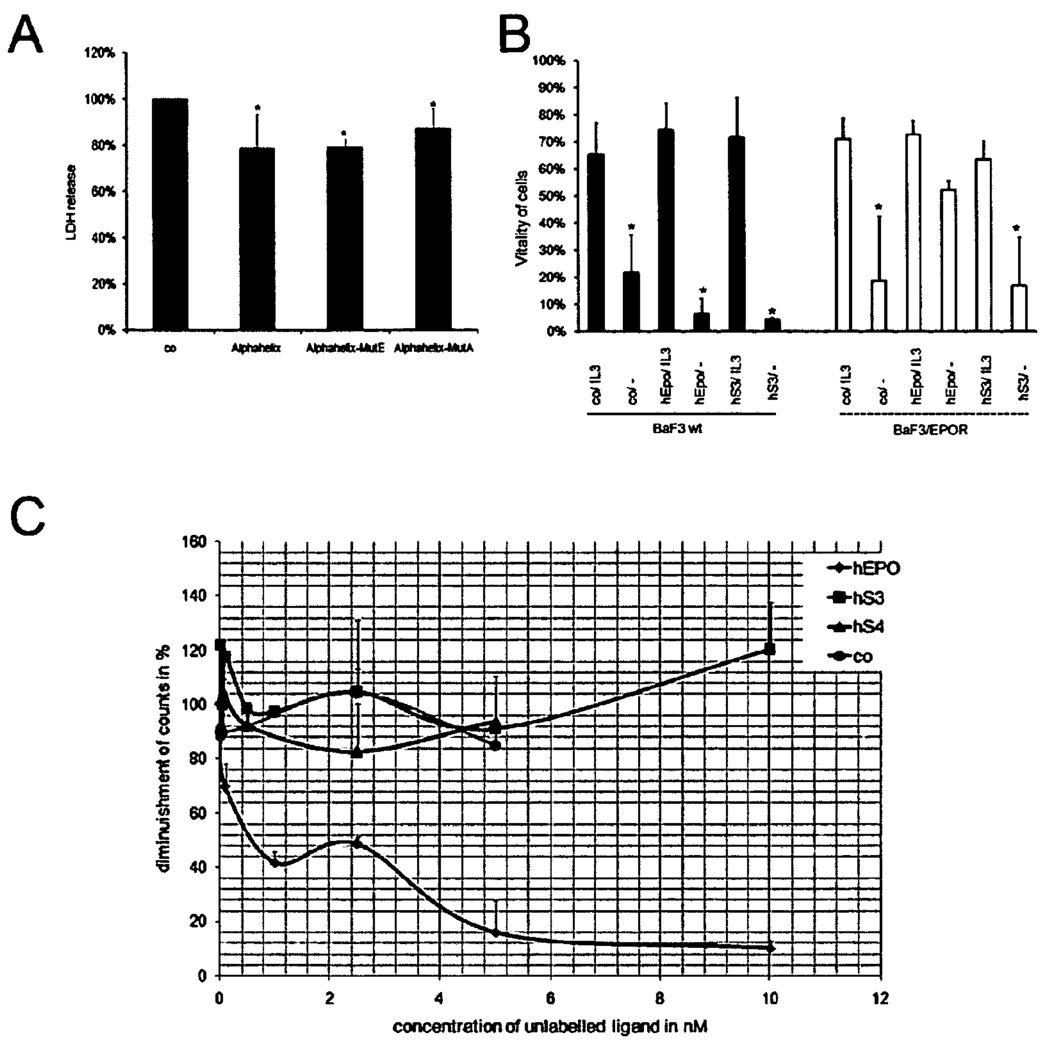
A
LDH release
co
Alphahelix
Alphahelix-MutE
Alphahelix-MutA
B
Vitality of cells
co/ IL3
co/ -
hEpo/ IL3
hEpo/ -
hS3/ IL3
hS3/ -
BaF3 wt
BaF3/EPOR
C
hEPO
hS3
hS4
co
diminuishment of counts in %
concentration of unlabelled ligand in nM

Fig. 6

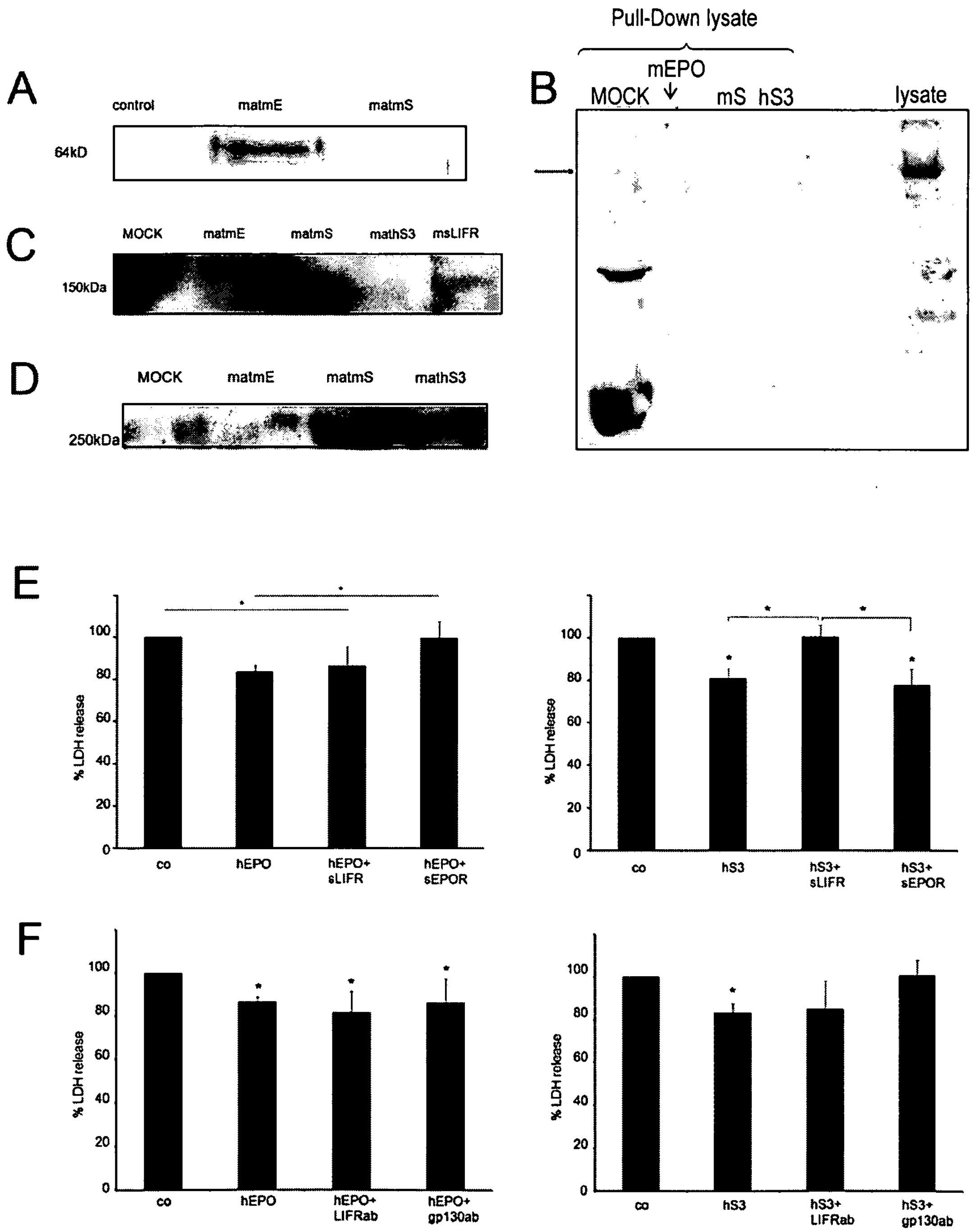
A
control
matmE
matmS
64kD
B
Pull-Down lysate
mEPO
MOCK
mS hS3
lysate
C
MOCK
matmE
matmS
mathS3
msLIFR
150kDa
D
MOCK
matmE
matmS
mathS3
250kDa
E
% LDH release
co
hEPO
hEPO+ sLIFR
hEPO+ sEPOR
co
hS3
hS3+ sLIFR
hS3+ sEPOR
F
% LDH release
co
hEPO
hEPO+ LIFRab
hEPO+ gp130ab
co
hS3
hS3+ LIFRab
hS3+ gp130ab

**Fig. 7**

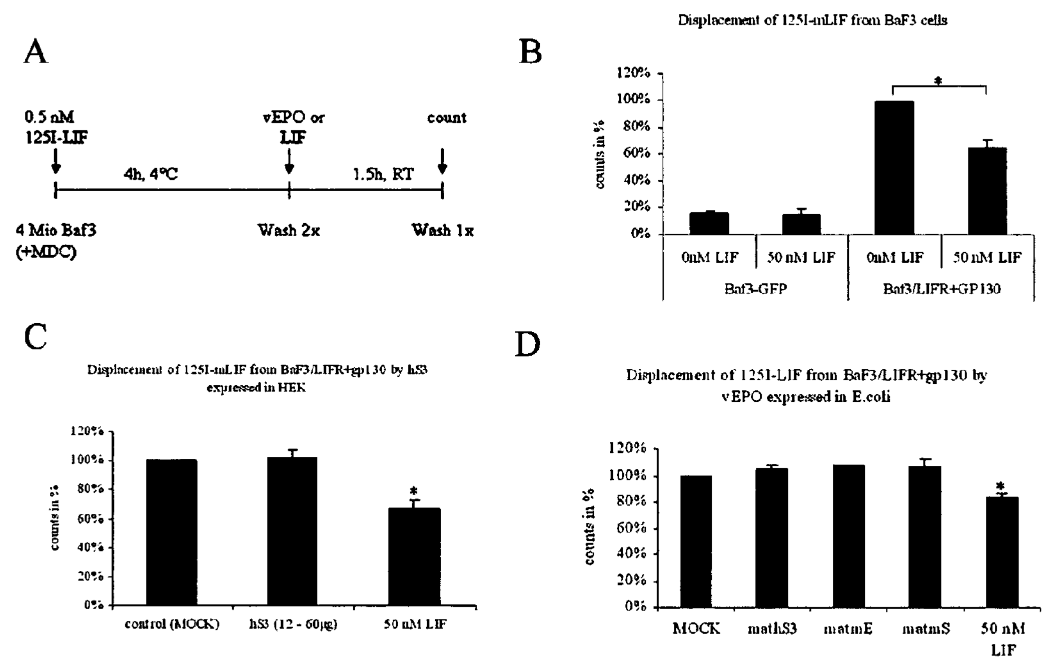
A
0.5 nM
125I-LIF
vEPO or
LIF
count
4h, 4°C
1.5h, RT
4 Mio Baf3
(+MDC)
Wash 2x
Wash 1x
B
Displacement of 125I-mLIF from BaF3 cells
counts in %
0nM LIF
50 nM LIF
0nM LIF
50 nM LIF
Baf3-GFP
Baf3/LIFR+GP130
C
Displacement of 125I-mLIF from BaF3/LIFR+gp130 by hS3 expressed in HEK
counts in %
control (MOCK)
hS3 (12 - 60µg)
50 nM LIF
D
Displacement of 125I-LIF from BaF3/LIFR+gp130 by vEPO expressed in E.coli
counts in %
MOCK
mathS3
matmE
matmS
50 nM LIF

**Fig. 8**

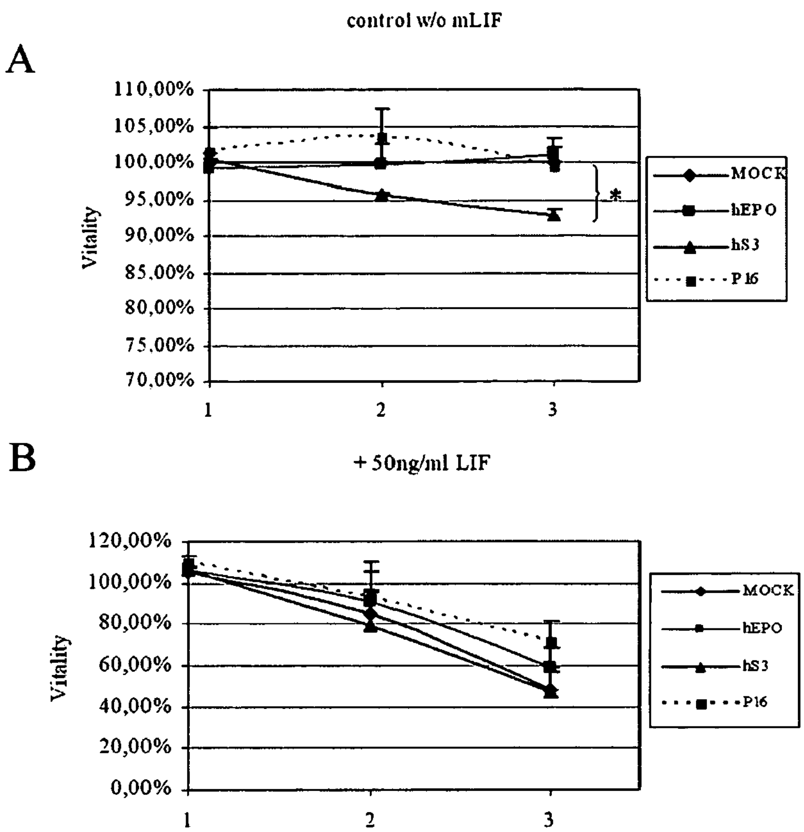
A
control w/o mLIF
110,00%
105,00%
100,00%
95,00%
90,00%
85,00%
80,00%
75,00%
70,00%
Vitality
1
2
3
*
MOCK
hEPO
hS3
P16
B
+ 50ng/ml LIF
120,00%
100,00%
80,00%
60,00%
40,00%
20,00%
0,00%
Vitality
1
2
3
MOCK
hEPO
hS3
P16

**Fig. 9**

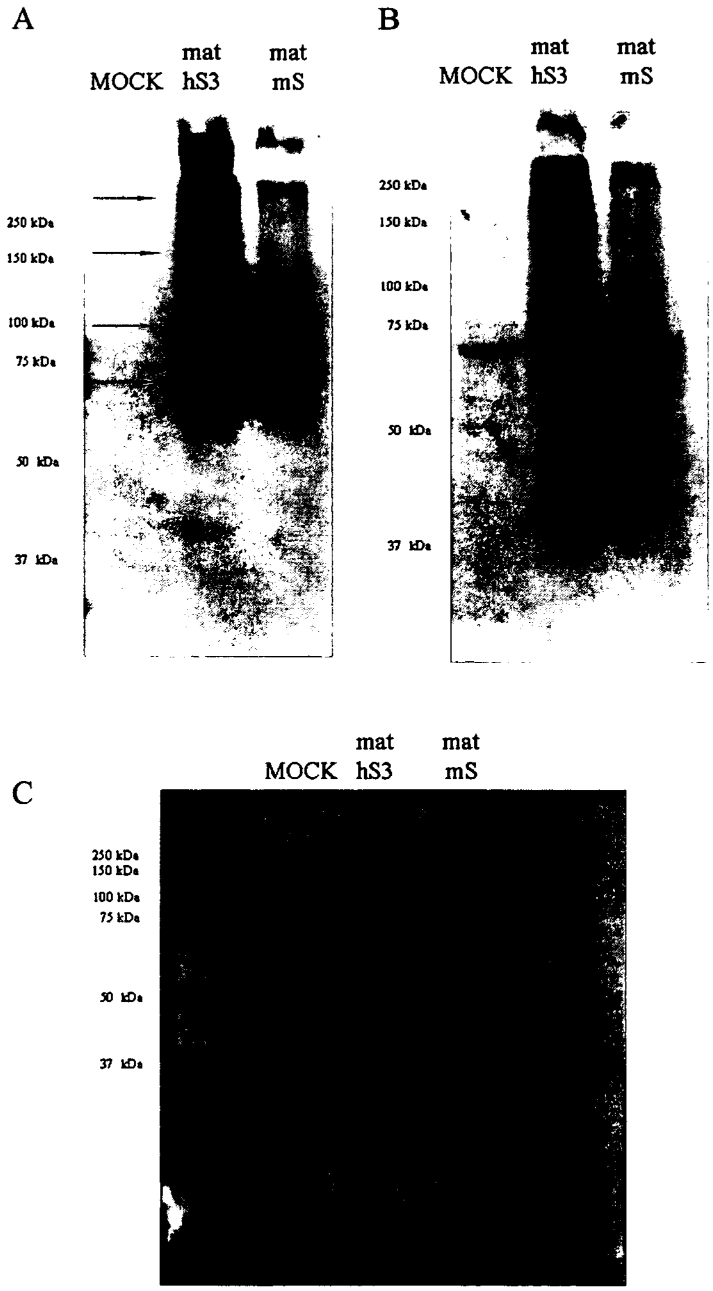
A
MOCK
mat hS3
mat mS
250 kDa
150 kDa
100 kDa
75 kDa
50 kDa
37 kDa
B
MOCK
mat hS3
mat mS
250 kDa
150 kDa
100 kDa
75 kDa
50 kDa
37 kDa
C
MOCK
mat hS3
mat mS
250 kDa
150 kDa
100 kDa
75 kDa
50 kDa
37 kDa

**Fig. 10**

Europäisches Patentamt
European Patent Office
Office européen des brevets

# EUROPEAN SEARCH REPORT

Application Number
EP 09 00 3341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 295 936 A (TAKEDA CHEMICAL INDUSTRIES LTD [JP] TAKEDA PHARMACEUTICAL [JP]) 26 March 2003 (2003-03-26) * paragraphs [0035] - [0050]; claims 1-19 * | 1,2,4-14 | INV. G01N33/50 |
| X | KAMIMURA D ET AL: "IL-6 signal transduction and its physiological roles: the signal orchestration model." REVIEWS OF PHYSIOLOGY, BIOCHEMISTRY AND PHARMACOLOGY 2003, vol. 149, 2003, pages 1-38, XP009121869 ISSN: 0303-4240 * pages 5-10; figure 2; tables 2,3 * | 1,2,4-14 | |
| X | GWECHENBERGER M ET AL: "Cardiac myocytes produce interleukin-6 in culture and in viable border zone of reperfused infarctions" CIRCULATION, LIPPINCOTT WILLIAMS & WILKINS, US, vol. 99, no. 4, 2 February 1999 (1999-02-02), pages 546-551, XP003012037 ISSN: 0009-7322 * page 550, right-hand column * | 1,2,4-14 | |

-/--

TECHNICAL FIELDS SEARCHED (IPC)

G01N

~~The present search report has been drawn up for all claims~~

| Place of search | Date of completion of the search | Examiner |
|---|---|---|
| Munich | 31 August 2009 | Stachowiak, Olaf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6

EPO FORM 1503 03.82 (P04C01)

Europäisches Patentamt
European Patent Office
Office européen des brevets

# EUROPEAN SEARCH REPORT

Application Number
EP 09 00 3341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAPPINGTON ET AL: "The ethyl pyruvate analogues, diethyl oxaloproprionate, 2-acetamidoacrylate, and methyl-2-acetamidoacrylate, exhibit anti-inflammatory properties in vivo and/or in vitro" BIOCHEMICAL PHARMACOLOGY, PERGAMON, OXFORD, GB, vol. 70, no. 11, 25 November 2005 (2005-11-25), pages 1579-1592, XP005135245 ISSN: 0006-2952 * abstract; table 1 * ----- | 1,2,4-14 | |
| A | GAO BIN: "Cytokines, STATs and liver disease." CELLULAR & MOLECULAR IMMUNOLOGY APR 2005, vol. 2, no. 2, April 2005 (2005-04), pages 92-100, XP002543480 ISSN: 1672-7681 * the whole document * ----- | 1,2,4-14 | |
| | | | TECHNICAL FIELDS SEARCHED (IPC) |
| D,A | EP 0 411 946 A (KISHIMOTO TADAMITSU [JP]) 6 February 1991 (1991-02-06) * the whole document * ----- | 1,2,4-14 | |
| A | VERGARA C ET AL: "CNTF, a pleiotropic cytokine: emphasis on its myotrophic role" BRAIN RESEARCH REVIEWS, ELSEVIER, NL, vol. 47, no. 1-3, 1 December 2004 (2004-12-01), pages 161-173, XP004664429 ISSN: 0165-0173 * the whole document * ----- | 1,2,4-14 | |

~~The present search report has been drawn up for all claims~~

| Place of search | Date of completion of the search | Examiner |
|---|---|---|
| Munich | 31 August 2009 | Stachowiak, Olaf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6

EPO FORM 1503 03.82 (P04C01)

Europäisches Patentamt
European Patent Office
Office européen des brevets

Application Number

EP 09 00 3341

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  see annex

- [ ] The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION
SHEET B**

Application Number
EP 09 00 3341

The Search Division considers that the present European patent application does not comply with the requirements of unity of invention and relates to several inventions or groups of inventions, namely:

1. claims: 1-2, 4-14 (all partly)

   Uses and methods for identifying cytoprotective compounds employing a protein complex comprising gp130.

   ---

2. claims: 1-14 (all partly)

   Uses and methods for identifying cytoprotective compounds employing a protein complex comprising gp130 and LIFR-alpha.

   ---

3. claims: 1-14 (all partly)

   Uses and methods for identifying cytoprotective compounds employing a protein complex comprising CNTFR-alpha, gp130 and LIFR-alpha.

   ---

4. claims: 1-14 (all partly)

   Uses and methods for identifying cytoprotective compounds employing a protein complex comprising gp80, gp130 and LIFR-alpha.

   ---

5. claims: 1-14 (all partly)

   Uses and methods for identifying cytoprotective compounds employing a protein complex comprising gp130 and IL-6R.

   ---

6. claims: 1-14 (all partly)

   Uses and methods for identifying cytoprotective compounds employing a protein complex comprising CNTFR-alpha, gp130 and IL-6R.

   ---

7. claims: 1-14 (all partly)

   Uses and methods for identifying cytoprotective compounds employing a protein complex comprising gp80, gp130 and IL-6R.

   ---

8. claims: 1-14 (all partly)

   Uses and methods for identifying cytoprotective compounds employing a protein complex comprising gp130 and IL-11R.

   ---

Europäisches Patentamt
European Patent Office
Office européen des brevets

**LACK OF UNITY OF INVENTION
SHEET B**

Application Number

EP 09 00 3341

The Search Division considers that the present European patent application does not comply with the requirements of unity of invention and relates to several inventions or groups of inventions, namely:

9. claims: 1-14 (all partly)

Uses and methods for identifying cytoprotective compounds employing a protein complex comprising CNTFR-alpha, gp130 and IL-11R.

---

10. claims: 1-14 (all partly)

Uses and methods for identifying cytoprotective compounds employing a protein complex comprising gp80, gp130 and IL-11R.

---

11. claims: 1-14 (all partly)

Uses and methods for identifying cytoprotective compounds employing a protein complex comprising gp130 and IL-27R.

---

12. claims: 1-14 (all partly)

Uses and methods for identifying cytoprotective compounds employing a protein complex comprising CNTFR-alpha, gp130 and IL-27R.

---

13. claims: 1-14 (all partly)

Uses and methods for identifying cytoprotective compounds employing a protein complex comprising gp80, gp130 and IL-27R.

---

14. claims: 1-14 (all partly)

Uses and methods for identifying cytoprotective compounds employing a protein complex comprising gp130 and OSMR-alpha.

---

15. claims: 1-14 (all partly)

Uses and methods for identifying cytoprotective compounds employing a protein complex comprising CNTFR-alpha, gp130 and OSMR-alpha.

---

16. claims: 1-14 (all partly)

Europäisches Patentamt
European Patent Office
Office européen des brevets

**LACK OF UNITY OF INVENTION**
**SHEET B**

Application Number
EP 09 00 3341

The Search Division considers that the present European patent application does not comply with the requirements of unity of invention and relates to several inventions or groups of inventions, namely:

Uses and methods for identifying cytoprotective compounds employing a protein complex comprising gp80, gp130 and OSMR-alpha.

---

## ANNEX TO THE EUROPEAN SEARCH REPORT ON EUROPEAN PATENT APPLICATION NO. EP 09 00 3341

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2009

| Patent document cited in search report | Publication date | Patent family member(s) | Publication date |
|---|---|---|---|
| EP 1295936 A | 26-03-2003 | AT 412766 T | 15-11-2008 |
| | | AU 6784401 A | 08-01-2002 |
| | | EP 2090663 A2 | 19-08-2009 |
| | | WO 0200850 A1 | 03-01-2002 |
| | | US 2008160553 A1 | 03-07-2008 |
| | | US 2003175847 A1 | 18-09-2003 |
| EP 0411946 A | 06-02-1991 | AT 147099 T | 15-01-1997 |
| | | AU 635296 B2 | 18-03-1993 |
| | | AU 6003990 A | 07-02-1991 |
| | | CA 2022610 A1 | 04-02-1991 |
| | | DE 69029545 D1 | 13-02-1997 |
| | | DE 69029545 T2 | 24-07-1997 |
| | | ES 2096580 T3 | 16-03-1997 |
| | | IL 95235 A | 27-11-1995 |
| | | JP 2898064 B2 | 31-05-1999 |
| | | JP 4029997 A | 31-01-1992 |
| | | US 5132403 A | 21-07-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0411946 B
- WO 9815830 A

### Non-patent literature cited in the description


- **Ullman et al.** *Proc. Natl. Acad. Sci., USA,* 1994, vol. 91, 5426-5430
- **Alms, W.J. ; Atamas, S.P. ; Yurovsky, V.V. ; White, B.** Generation of a variant of human interleukin-4 by alternative splicing. *Mol. Immunol.,* 1996, vol. 33, 361-370
- **Albensi, B.C. ; Mattson, M.P.** Evidence for the involvement of TNF and NF-κB in hippocampal synaptic plasticity. *Synapse,* 2000, vol. 35, 151-159
- **Banks, W.A. ; Jumbe, NX. ; Farrell, C.L. ; Niehoff, ML. ; Heatherington, A.C.** Passage of erythropoietic agents across the blood-brain barrier: a comparison of human and murine erythropoietin and the analog darbepoetin alfa. *Eur. J. Pharmacol.,* 2004, vol. 505, 93-101
- **Barron, C ; Migliaccio, A.R. ; Migliaccio, G. ; Jiang, Y. ; Adamson, J.W. ; Ottolenghi, S.** Alternatively spliced mRNAs encoding soluble isoforms of the erythropoietin receptor in murine cell lines and bone marrow. *Gene,* 1994, vol. 147, 263-268
- **Bernaudin, M ; Marti, H.H. ; Roussel, S. ; Divoux, D. ; Nouvelot, A. ; MacKenzie, E.T. ; Petit, E.** A potential role for erythropoietin in focal permanent cerebral ischemia in mice. *J. Cereb. Blood Flow Metab.,* 1999, vol. 19, 643-651
- **Bihl, M.P. ; Heinimann, K. ; Rudiger, J.J. ; Eickelberg, O. ; Perruchoud, A.P. ; Tamm, M. ; Roth, M.** Identification of a novel IL-6 isoform binding to the endogenous IL-6 receptor. *Am. J. Respir. Cell. Mol. Biol.,* 2002, vol. 27, 48-56
- **Bordet, T. ; Schmalbruch, H. ; Pettmann, B ; Hagege, A. ; Castelnau-Ptakhine, L. ; Kahn, A. ; Haase, G.** Adenoviral cardiotrophin-1 gene transfer protects pmn mice from progressive motor neuronopathy. *J. Clin. Invest.,* 1999, vol. 104, 1077-1085
- **Boulanger, M.J. ; Garcia, K.C.** Shared cytokine signaling receptors: structural insights from the gp130 System. *Adv. Protein Chem.,* 2004, vol. 68, 107-146
- **Brasier, A.R.** The NF-κB regulatory network. *Cardiovasc. Toxicol.,* 2006, vol. 6, 111-130
- **Bravo, J. ; Heath, J. K.** Receptor recognition by gp130 cytokines. *Embo J,* 2000, vol. 19 (11), 2399-2411
- **Brines, M. ; Grasso, G. ; Fiordaliso, F. ; Sfacteria, A. ; Ghezzi, P. ; Fratelli, M. ; Latini, R. ; Xie, Q.W. ; Smart, J. ; Su-Rick, C.J. et al.** Erythropoietin mediates tissue protection through an erythropoietin and common beta-subunit heteroreceptor. *Proc. Natl. Acad. Sci. USA,* 2004, vol. 101, 14907-14912
- **Brines, M,L. ; Ghezzi, P. ; Keenan, S. ; Agnello, D. ; de Lanerolle, N.C. ; Cerami, C ; Itri, L.M. ; Cerami, A.** Erythropoietin crosses the blood-brain barrier to protect against experimental brain injury. *Proc. Natl. Acad. Sci. USA,* 2000, vol. 97, 10526-10531
- **Bruce, A. G. ; Hoggatt, I. H. ; Rose, T. M.** Oncostatin M is a differentiation factor for myeloid leukemia cells. *J. Immuno.,* 1992, vol. 149 (4), 1271-1275
- **Calvillo, L. ; Latini, R. ; Kajstura, J. ; Leri, A. ; Anversa, P. ; Ghezzi, P. ; Salio, M. ; Cerami, A. ; Brines, M.** Recombinant human erythropoietin protects the myocardium from ischemia-reperfusion injury and promotes beneficial remodeling. *Proc. Natl. Acad. Sci. USA,* 2003, vol. 100, 4802-4806
- **Campana, W.M. ; Myers, R.R.** Exogenous erythropoietin protects against dorsal root ganglion apoptosis and pain following peripheral nerve injury. *Eur. J. Neurosci.,* 2003, vol. 18, 1497-1506
- **Carpenter, M. K. ; Cui, X. ; Hu, Z. Y. ; Jackson, J. et al.** In vitro expansion of a multipotent population of human neural progenitor cells. *Exp. Neurol.,* 1999, vol. 158 (2), 265-278
- **Celik, M. ; Gokmen, N. ; Erbayraktar, S. ; Akhisaroglu, M. ; Konake, S. ; Ulukus, C ; Gene, S. ; Gene, K. ; Sagiroglu, E. ; Cerami, A. et al.** Erythropoietin prevents motor neuron apoptosis and neurologic disability in experimental spinal cord ischemic injury. *Proc. Natl. Acad. Sci. USA,* 2002, vol. 99, 2258-2263

- **Chang, M.Y. ; Park, C.H. ; Son, H. ; Lee, Y.S. ; Lee, S.H.** Developmental stage-dependent self-regulation of embryonic cortical precursor cell survival and differentiation by leukemia inhibitory factor. *Cell Death Differ.,* 2004, vol. 11, 985-996
- **Cheetham, J.C. ; Smith, D.M. ; Aoki, K.H. ; Stevenson, J.L. ; Hoeffel, T.J. ; Syed, R.S. ; Egrie, J. ; Harvey, T.S.** NMR structure of human erythropoietin and a comparison with its receptor bound conformation. *Nat. Struct. Biol.,* 1998, vol. 5, 861-866
- **Chikuma, M. ; Masuda, S. ; Kobayashi, T. ; Nagao, M. ; Sasaki, R.** Tissue-specific regulation of erythropoietin production in the murine kidney, brain, and uterus. *Am. J. Physiol.,* 2000, vol. 279, E1242-1248
- **Chin, K'' Yu, X. ; Beleslin-Cokic, B. ; Liu, C ; Shen, K. ; Mohrenweiser, H.W. ; Noguchi, CT.** Production and processing of erythropoietin receptor transcripts in brain. *Brain Res.,* 2000, vol. 81, 29-42
- **Chou, C.F. ; Tohari, S. ; Brenner, S. ; Venkatesh, B.** Erythropoietin gene from a teleost fish, Fugu rubripes. *Blood,* 2004, vol. 104, 1498-1503
- **Derouet, D. ; Rousseau, F. ; Alfonsi, F. ; Froger, JL ; Hermann, J. ; Barbier, F. ; Perret, D. ; Diveu, C ; Guillet, C ; Preisser, L. et al.** Neuropoietin, a new IL-6-related cytokine signaling through the ciliary neurotrophic factor receptor. *Proc. Natl. Acad. Sci. USA,* 2004, vol. 101, 4827-4832
- **Digicaylioglu, M. ; Lipton, S.A.** Erythropoietin-mediated neuroprotection involves cross-talk between Jak2 and NF-kappaB signalling cascades. *Nature,* 2001, vol. 412, 641-647
- **Dolcet, X. ; Soler, R.M. ; Gould, T.W. ; Egea, J. ; Oppenheim, R.W. ; Cornelia, J.X.** Cytokines promote motoneuron survival through the Janus kinase-dependent activation of the phosphatidylinositol 3-kinase pathway. *Mol. Cell. Neurosci.,* 2001, vol. 18, 619-631
- **Ehrenreich, H. ; Degner, D. ; Meiler, J. ; Brines, M. ; Behe, M. ; Hasselblatt, M ; Woldt, H. ; Falkai, P. ; Knerlich, F. ; Jacob, S. et al.** Erythropoietin: a candidate compound for neuroprotection in schizophrenia. *Mol. Psychiatry,* 2004, vol. 9, 42-54
- **Ehrenreich, H. ; Hasselblatt, M. ; Dembowski, C ; Cepek, L. ; Lewczuk, P. ; Stiefel, M. ; Rustenbeck, H.H. ; Breiter, N. ; Jacob, S. ; Knerlich, F. et al.** Erythropoietin therapy for acute stroke is both safe and beneficial. *Mol. Med.,* 2002, vol. 8, 495-505
- **Elson, G. C. ; Lelievre, E. ; Guillet, C. ; Chevalier, S. et al.** CLF associates with CLC to form a functional heteromeric ligand for the CNTF receptor complex. *Nat. Neurosc.,* 2000, vol. 3 (9), 867-872
- **Erbayraktar, S. ; Grasso, G. ; Sfacteria, A. ; Xie, Q.W. ; Coleman, T. ; Kreilgaard, M. ; Torup, L. ; Sager, T. ; Erbayraktar, Z. ; Gokmen, N. et al.** Asialoerythropoietin is a nonerythropoietic cytokine with broad neuroprotective activity in vivo. *Proc. Natl. Acad. Sci. USA,* 2003, vol. 700, 6741-6746
- **Garofalo, R.S ; Orena, S.J. ; Rafidi, K. ; Torchia, A.J. ; Stock, J.L. ; Hildebrandt, A.L. ; Coskran, T. ; Black, S.C. ; Brees, D.J. ; Wicks, J.R.** Severe diabetes, age-dependent loss of adipose tissue, and mild growth deficiency in mice lacking Akt2/PKB beta. *Journal of Clinical Investigation,* 2003, vol. 112, 197-208
- **Gearing, D.P. ; Comeau, M.R. ; Friend, DJ. ; Gimpel, S.D. ; Thut, C.J. ; McGourty, J ; Brasher, K.K. ; King, J.A. ; Gillis, S. ; Mosley, B. et al.** The IL-6 signal transducer, gp130: an oncostatin M receptor and affinity converter for the LIF reeeptor. *Science,* 1992, vol. 255, 1434-1437
- **Gilmore, T.D.** The Rel/NF-κB signal transduction pathway: introduction. *Oncogene,* 1999, vol. 18, 6842-6844
- **Gilmore, T.D.** Introduction to NF-κB: players, pathways, perspectives. *Oncogene,* 2006, vol. 25, 6680-6684
- **Goldberg, M.A. ; Dunning, S.P. ; Bunn, H.F.** Regulation of the erythropoietin gene: evidence that the oxygen sensor is a heme protein. *Science,* 1988, vol. 242, 1412-1415
- **Ghosh, S. ; May, M.J. ; Kopp, E.B.** NF-κB and Rel proteins: evolutionarily conserved mediators of immune responses. *Annu. Rev. Immunol.,* 1998, vol. 16, 225-260
- **Hisaka, T. ; Desmouliere, A. ; Taupin, J.L. ; Daburon, S. ; Neaud, V. ; Senant, N. ; Blanc, J.F. ; Moreau, J.F. ; Rosenbaum, J.** Expression of leukemia inhibitory factor (LIF) and its receptor gp190 in human liver and in cultured human liver myofibroblasts. Cloning of new isoforms of LIF mRNA. *Comp. Hepatol.,* 2004, vol. 3, 10
- **Ihle, J.N.** The Janus protein tyrosine kinase family and its role in cytokine signaling. *Adv. Immunol.,* 1995, vol. 60, 1-35
- **Ikeda, K. ; Iwasaki, Y. ; Shiojima, T. ; Kinoshita, M.** Neuroprotective effect of various cytokines on developing spinal motoneurons following axotomy. *J. Neurol. Sci.,* 1996, vol. 135, 109-113
- **Jacobs, M.D. ; Harrison, S.C.** Structure of an IkappaBalpha/NF-kappaB complex. *Cell,* 1998, vol. 95, 749-758
- **Jubinsky, P.T. ; Krijanovski, O.I. ; Nathan, D.G. ; Tavernier, J. ; Sieff, CA.** The beta chain of the interleukin-3 receptor functionally associates with the erythropoietin receptor. *Blood,* 1997, vol. 90, 1867-1873
- **Karin, M.** How NF-kappaB is activated: the role of the IkappaB kinase (IKK) complex. *Oncogene,* 1999, vol. 18, 6867-6874

- **Karin, M. ; Ben-Neriah, Y.** Phosphorylation meets ubiquitination: the control of NF-κB activity. *Annu. Rev. Immunol.,* 2000, vol. 18, 621-663
- **Kaushansky, K. ; Karplus, P.A.** Hematopoietic growth factors: understanding functional diversity in structural terms. *Blood,* 1993, vol. 82, 3229-3240
- **Kestler, D.P. ; Goldstein, K.M. ; Agarwal, S. ; Fuhr, J.E. ; Andrews, R. ; Hall, R.E.** Hematopoietic differentiation activity of a recombinant human interleukin-6 (IL-6) isoform resulting from alternatively spliced deletion of the second exon. *Am. J. Hematol.,* 1999, vol. 61, 169-177
- **Keswani, S.C ; Leitz, G.J. ; Hoke, A.** Erythropoietin is neuroprotective in models of HIV sensory neuropathy. *Neurosci. Lett.,* 2004, vol. 371, 102-105
- **Kisseleva et al.** Signaling through the JAK/STAT pathway, recent advances and future challenges. *Gene,* 2002, vol. 285, 1-24
- **Lam, K.** Mini-review. Application of combinatorial library methods in cancer research and drug discovery. *Anticancer Drug Design,* 1997, vol. 12, 145-167
- **Leist, M. ; Ghezzi, P. ; Grasso, G. ; Bianchi, R. ; Villa, P. ; Fratelli, M. ; Savino, C ; Bianchi, M. ; Nielsen, J. ; Gerwien, J. et al.** Derivatives of erythropoietin that are tissue protective but not erythropoietic. *Science,* 2004, vol. 305, 239-242
- **Livnah, O. ; Stura, E.A. ; Middleton, S.A. ; Johnson, D.L. ; Jolliffe, L.K. ; Wilson, I.A.** Crystallographic evidence for preformed dimers of erythropoietin receptor before ligand activation. *Science,* 1999, vol. 283, 987-990
- **Lo, A.C. ; Li, L. ; Oppenheim, R.W. ; Prevette, D. ; Houenou, L.J.** Ciliary neurotrophic factor promotes the survival of spinal sensory neurons following axotomy but not during the period of programmed cell death. *Exp. Neurol.,* 1995, vol. 134, 49-55
- **Marti, H.H. ; Wenger, R.H. ; Rivas, L.A. ; Straumann, U. ; Digicaylioglu, M ; Henn, V. ; Yonekawa, Y. ; Bauer, C. ; Gassmann, M.** Erythropoietin gene expression in human, monkey and murine brain. *Eur. J. Neurosci.,* 1996, vol. 8, 666-676
- **Meazza, R. ; Verdiani, S. ; Biassoni, R. ; Coppolecchia, M. ; Gaggero, A. ; Orengo, A.M. ; Colombo, M.P. ; Azzarone, B. ; Ferrini, S.** Identification of a novel interleukin-15 (IL-15) transcript isoform generated by alternative splicing in human small cell lung cancer cell lines. *Oncogene,* 1996, vol. 12, 2187-2192
- **Mercurio, F. ; Zhu, H. ; Murray, B.W. ; Shevchenko, A. ; Bennett, B.L. ; Li, J. ; Young, D.B. ; Barbosa, M. ; Mann, M. ; Manning, A.** IKK-1 and IKK-2: cytokine-activated IkappaB kinases essential for NF-kappaB activation. *Science,* 1997, vol. 278, 860-866
- **Metcalf, D.** Hematopoietic cytokines. *Blood,* 2008, vol. 111, 485-491
- **Middleton, G. ; Hamanoue, M. ; Enokido, Y. ; Wyatt, S. ; Pennica, D. ; JafTray, E. ; Hay, R.T. ; Davies, A.M.** Cytokine-induced nuclear factor kappa B activation promotes the survival of developing neurons. *J. Cell Biol.,* 2000, vol. 148, 325-332
- **Miyake, T. ; Kung, C.K. ; Goldwasser, E.** Purification of human erythropoietin. *J. Biol. Chem.,* 1977, vol. 252, 5558-5564
- **Mosley, B. ; De Imus, C. ; Friend, D. ; Boiani, N. et al.** Dual oncostatin M (OSM) receptors. Cloning and characterization of an alternative signaling subunit conferring OSM-specific receptor activation. *J. Biol. Chem.,* 1996, vol. 271 (51), 32635-32643
- **Murphy, M. ; Reid, K. ; Hilton, D. J. ; Bartlett, P. F.** Generation of sensory neurons is stimulated by leukemia inhibitory factor. *Proc. Natl. Acad. Sci. USA,* 1991, vol. 88 (8), 3498-3501
- **Murphy, M. ; Dutton, R. ; Koblar, S. ; Cheema, S. ; Bartlett, P.** Cytokines which signal through the LIF receptor and their actions in the nervous System. *Prog. Neurobiol.,* 1997, vol. 52, 355-378
- **Nakamura, Y. ; Komatsu, N. ; Nakauchi, H.** A truncated erythropoietin receptor that fails to prevent programmed cell death of erythroid cells. *Science,* 1992, vol. 257, 1138-1141
- **Noguchi, CT. ; Asavaritikrai, P. ; Teng, R. ; Jia, Y.** Role of erythropoietin in the brain. *Crit Rev. Oncol. Hematol.,* 2007, vol. 64, 159-171
- **Ochiai, W. ; Yanagisawa, M. ; Takizawa, T. ; Nakashima, K. et al.** Astrocyte differentiation of fetal neuroepithelial cells involving cardiotrophin-1-induced activation of STAT3. *Cytokine,* 2001, vol. 14 (5), 264-271
- **Ohno, M. ; Abe, T.** Rapid colorimetric assay for the quantification of leukemia inhibitory factor (LIF) and interleukin-6 (IL-6). *J. Immunol. Method.,* 1991, vol. 145 (1-2), 199-203
- **Ohno. M. ; Kohyama, J. ; Namihira, M. ; Sanosaka, T. et al.** Neuropoietin induces neuroepithelial cells to differentiate into astrocytes via activation of STAT3. *Cytokine,* 2006, vol. 36 (1-2), 17-22
- **Parsa, C.J. ; Matsumoto, A. ; Kim, J. ; Riel, R.U. ; Pascal, L.S. ; Walton, G.B. ; Thompson, R.B. ; Petrofski, J.A. ; Annex, B.H. ; Stamler, J.S. et al.** A novel protective effect of erythropoietin in the infarcted heart. *J. Clin. Invest.,* 2003, vol. 112, 999-1007
- **Pennica, D. ; King, K. L. ; Shaw, K. J. ; Luis, E. et al.** Expression cloning of cardiotrophin 1, a cytokine that induces cardiac myocyte hypertrophy. *Proc. Natl. Acad. Sci. USA,* 1995, vol. 92 (4), 1142-1146
- **Perkins, N.D.** Integrating cell-signalling pathways with NF-κB and IKK function. *Nat. Rev. Mol. Cell Biol.,* 2007, vol. 8, 49-62

- **Prass, K. ; Scharff, A. ; Ruscher, K. ; Lowl, D. ; Muselmann, C ; Victorov, I. ; Kapinya, K. ; Dirnagl, U. ; Meisel, A.** Hypoxia-induced stroke tolerance in the mouse is mediated by erythropoietin. *Stroke,* 2003, vol. 34, 1981-1986
- **Puskovic, V. ; Wolfe, D. ; Wechuck, J. ; Krisky, D. ; Collins, J. ; Glorioso, J.C. ; Fink, DJ. ; Mata, M.** HSV-mediated delivery of erythropoietin restores dopaminergic function in MPTP-treated mice. *Mol. Ther.,* 2006, vol. 14, 710-715
- **Rajan, P. ; McKay, R. D.** Multiple routes to astrocytic differentiation in the CNS. *J Neurosc.,* 1998, vol. 18 (10), 3620-3629
- **Ramaswamy, S. ; Nakamura, N. ; Vazquez, F. ; Batt, D.B. ; Perera, S. ; Roberts, T.M. ; Sellers, W.R.** Regulation of G1 progression by the PTEN tumor suppressor protein is linked to inhibition of the phosphatidylinositol 3-kinase/Akt pathway. *Proc. Natl. Acad. Sci. USA,* 1999, vol. 96, 2110-2115
- **Régnier, C.H. ; Song, H.Y. ; Gao, X. ; Goeddel, D.V. ; Cao, Z. ; Rothe, M.** Identification and characterization of an IkappaB kinase. *Cell,* 1997, vol. 90, 373-83
- **Garofalo, R.S. ; Orena, S.J. ; Rafidi, K. ; Torchia, A.J. ; Stock, J.L. ; Hildebrandt, A.L. ; Coskran, T. ; Black, S.C. ; Brees, D.J. ; Wicks, J.R.** Severe diabetes, age-dependent loss of adipose tissue, and mild growth deficiency in mice lacking Akt2/PKB beta. *Journal of Clinical Investigation,* 2003, vol. 112, 197-208
- **Robledo, O. ; Fourcin, M. ; Chevalier, S. ; Guillet, C. et al.** Signaling of the cardiotrophin-1 receptor. Evidence for a third receptor component. *J. Biol. Chem.,* 1997, vol. 272 (8), 4855-4863
- **Rose, T. M. ; Weiford, D. M. ; Gunderson, N. L. ; Bruce, A. G.** Oncostatin M (OSM) inhibits the differentiation of pluripotent embryonic stem cells in vitro. *Cytokine,* 1994, vol. 6 (1), 48-54
- **Ruscher, K. ; Freyer, D. ; Karsch, M. ; Isaev, N. ; Megow, D. ; Sawitzki, B. ; Priller, J. ; Dirtiagl, U. ; Meisel, A.** Erythropoietin is a paracrine mediator of ischemic tolerance in the brain: evidence from an in vitro model. *J. Neurosci.,* 2002, vol. 22, 10291-10301
- **Sakanaka, M ; Wen, T.C. ; Matsuda, S. ; Masuda, S. ; Morishita, E. ; Nagao, M. ; Sasaki, R.** In vivo evidence that erythropoietin protects neurons from ischemic damage. *Proc. Natl. Acad. Sci. USA,* 1998, vol. 95, 4635-4640
- **Sattler, M.B. ; Merkler, D. ; Maier, K. ; Stadelmann, C ; Ehrenreich, H. ; Bahr, M ; Diem, R.** Neuroprotective effects and intracellular signaling pathways of erythropoietin in a rat model of multiple sclerosis. *Cell Death Differ.,* 2004, vol. 11 (2), 181-192
- **Schuettauf, F. ; Zurakowski, D. ; Quinto, K. ; Varde, M.A. ; Besch, D. ; Laties, A. ; Anderson, R. ; Wen, R.** Neuroprotective effects of cardiotrophin-like cytokine on retinal ganglion cells. *Graefes Arch. Clin. Exp. Ophthalmo1.,* 2005, vol. 243, 1036-1042
- **Senftleben U ; Cao Y ; Xiao G ; Greten FR ; Krähn G ; Bonizzi G ; Chen Y ; Hu Y ; Fong A ; Sun SC.** Activation by IKKalpha of a second, evolutionary conserved, NF-κB signaling pathway. *Science,* 2001, vol. 293, 1495-1499
- **Siren, A.L. ; Fratelli, M. ; Brines, M. ; Goemans, C ; Casagrande, S. ; Lewczuk, P. ; Keenan, S. ; Gleiter, C ; Pasquali, C ; Capobianco, A. et al.** Erythropoietin prevents neuronal apoptosis after cerebral ischemia and metabofic stress. *Proc. Natl. Acad. Sci. USA,* 2001, vol. 98, 4044-4049
- **Sorg, R.V. ; Enczmann, J. ; Sorg, U.R. ; Schneider, E.M. ; Wernet, P.** Identification of an altematively spliced transcript of human interleukin-4 lacking the sequence encoded by exon 2. *Experimental hematology,* 1993, vol. 21, 560-563
- **Statler, P.A. ; McPherson, R.J. ; Bauer, L.A. ; Kellert, B.A. ; Juul, S.E.** Pharmacokinetics of high-dose recombinant erythropoietin in plasma and brain of neonatal rats. *Pediatr. Res.,* 2007, vol. 61, 671-675
- **Tian, B. ; Brasier, A.R.** Identification of a nuclear factor κ B-dependent gene network. *Recent Prog. Horm. Res.,* 2003, vol. 58, 95-130
- **Tsai, P. T. ; Ohab, J. J. ; Kertesz, N. ; Groszer, M. et al.** A critical role of erythropoietin receptor in neurogenesis and post-stroke recovery. *J Neurosci.,* 2006, vol. 26 (4), 1269-1274
- **Uemura, A. ; Takizawa, T. ; Ochiai, W. ; Yanagisawa, M. et al.** Cardiotrophin-like cytokine induces astrocyte differentiation of fetal neuroepithelial cells via activation of STAT3. *Cytokine,* 2002, vol. 18 (1), 1-7
- **Vesey, D.A. ; Cheung, C ; Pat, B. ; Endre, Z. ; Gobe, G. ; Johnson, D.W.** Erythropoietin protects against ischaemic acute renal injury. *Nephrol. Dial. Transplant,* 2004, vol. 19, 348-355
- **Wang, L. ; Zhang, Z. G. ; Gregg, S. R. ; Zhang, R. L. et al.** The Sonic hedgehog pathway mediates carbamylated erythropoietin-enhanced proliferation and differentiation of adult neural progenitor cells. *J. Biol. Chem.,* 2007, vol. 282 (44), 32462-32470
- **Weiss, T.W. ; Samson, A.L. ; Niego, B. ; Daniel, P.B. ; Medcalf, R.L.** Oncostatin M is a neuroprotective cytokine that inhibits excitotoxic injury in vitro and in vivo. *FASEB J.,* 2006, vol. 20, 2369-2371
- **Wen, D. ; Boissel, J.P. ; Showers, M ; Ruch, B.C. ; Bunn, H.F.** Erythropoietin structure-function relationships. Identification of functionally important domains. *J. Biol. Chem.,* 1994, vol. 269, 22839-22846

- **Witthuhn, B.A. ; Quelle, F.W. ; Silvennoinen, O. ; Yi, T. ; Tang, B. ; Miura, O. ; Ihle, J.N.** JAK2 associates with the erythropoietin receptor and is tyrosine phosphorylated and activated following stimulation with erythropoietin. *Cell,* 1993, vol. 74, 227-236
- **Wu, H. ; Liu, X. ; Jaenisch, R. ; Lodish, H.F.** Generation of committed erythroid BFU-E and CFU-E progenitors does not require erythropoietin or the erythropoietin receptor. *Cell,* 1995, vol. 83, 59-67
- **Yatsenko, O.P. ; Filipenko, M.L. ; Khrapov, E.A. ; Voronina, E.N. ; Kozlov, V.A. ; Sennikov, S.V.** Alternative splicing of mRNA of mouse interleukin-4 and interleukin 6. *Cytokine,* 2004, vol. 28, 190-196
- **Yoshida, K. ; Taga, T. ; Saito, M. ; Suematsu, S. et al.** Targeted disruption of gp130, a common signal transducer for the interleukin 6 family of cytokines, leads to myocardial and hematological disorders. *Proc. Natl. Acad. Sci. USA,* 1996, vol. 93 (1), 407-411
- **Yu, X. ; Shacka, J.J. ; Eells, J.B. ; Suarez-Quian, C ; Przygodzki, R.M. ; Beleslin-Cokic, B. ; Lin, C.S. ; Nikodem, V.M. ; Hempstead, B. ; Flanders, K.C. et al.** Erythropoietin receptor signalling is required for normal brain development. *Development,* 2002, vol. 129, 505-516
- **Yang, Z.Z. ; Tschopp, O. ; Baudry, A. ; Dummler, B. ; Hynx, D. ; Hemmings, B.A.** Physiological functions of protein kinase B/Akt. *Biochem. Soc Trans.,* 2004, vol. 32, 350-354

SEQUENCE LISTING

<110> Charite
Charité - Universitätsmedizin Berlin, Gliedkörperschaft der Freien Universität Berlin und der Humboldt-Universität zu Berlin

<120> Uses and methods for the identification of a cytoprotective compound involving gp130 or LIFRa

<130> I65974EP

<160> 33

<170> PatentIn version 3.5

<210> 1
<211> 193
<212> PRT
<213> Homo sapiens

<400> 1

```
Met Gly Val His Glu Cys Pro Ala Trp Leu Trp Leu Leu Leu Ser Leu
1               5                   10                  15

Leu Ser Leu Pro Leu Gly Leu Pro Val Leu Gly Ala Pro Pro Arg Leu
            20                  25                  30

Ile Cys Asp Ser Arg Val Leu Glu Arg Tyr Leu Leu Glu Ala Lys Glu
        35                  40                  45

Ala Glu Asn Ile Thr Thr Gly Cys Ala Glu His Cys Ser Leu Asn Glu
    50                  55                  60

Asn Ile Thr Val Pro Asp Thr Lys Val Asn Phe Tyr Ala Trp Lys Arg
65                  70                  75                  80

Met Glu Val Gly Gln Gln Ala Val Glu Val Trp Gln Gly Leu Ala Leu
                85                  90                  95

Leu Ser Glu Ala Val Leu Arg Gly Gln Ala Leu Leu Val Asn Ser Ser
            100                 105                 110

Gln Pro Trp Glu Pro Leu Gln Leu His Val Asp Lys Ala Val Ser Gly
        115                 120                 125

Leu Arg Ser Leu Thr Thr Leu Leu Arg Ala Leu Gly Ala Gln Lys Glu
    130                 135                 140

Ala Ile Ser Pro Pro Asp Ala Ala Ser Ala Ala Pro Leu Arg Thr Ile
145                 150                 155                 160

Thr Ala Asp Thr Phe Arg Lys Leu Phe Arg Val Tyr Ser Asn Phe Leu
                165                 170                 175

Arg Gly Lys Leu Lys Leu Tyr Thr Gly Glu Ala Cys Arg Thr Gly Asp
            180                 185                 190
```

Arg

<210> 2
<211> 164
<212> PRT
<213> Homo sapiens

<400> 2

Met Gly Val His Glu Cys Pro Ala Trp Leu Trp Leu Leu Leu Ser Leu
1 5 10 15

Leu Ser Leu Pro Leu Gly Leu Pro Val Leu Gly Ala Pro Pro Arg Leu
20 25 30

Ile Cys Asp Ser Arg Val Leu Glu Arg Tyr Leu Leu Glu Ala Lys Glu
35 40 45

Ala Glu Asn Ile Thr Val Gly Gln Gln Ala Val Glu Val Trp Gln Gly
50 55 60

Leu Ala Leu Leu Ser Glu Ala Val Leu Arg Gly Gln Ala Leu Leu Val
65 70 75 80

Asn Ser Ser Gln Pro Trp Glu Pro Leu Gln Leu His Val Asp Lys Ala
85 90 95

Val Ser Gly Leu Arg Ser Leu Thr Thr Leu Leu Arg Ala Leu Gly Ala
100 105 110

Gln Lys Glu Ala Ile Ser Pro Pro Asp Ala Ala Ser Ala Ala Pro Leu
115 120 125

Arg Thr Ile Thr Ala Asp Thr Phe Arg Lys Leu Phe Arg Val Tyr Ser
130 135 140

Asn Phe Leu Arg Gly Lys Leu Lys Leu Tyr Thr Gly Glu Ala Cys Arg
145 150 155 160

Thr Gly Asp Arg

<210> 3
<211> 162
<212> PRT
<213> Homo sapiens

<400> 3

Met Gly Val His Glu Cys Pro Ala Trp Leu Trp Leu Leu Leu Ser Leu
1 5 10 15

Leu Ser Leu Pro Leu Gly Leu Pro Val Leu Gly Ala Pro Pro Arg Leu
20 25 30

Ile Cys Asp Ser Arg Val Leu Glu Arg Tyr Leu Leu Glu Ala Lys Glu
35 40 45

Ala Glu Asn Ile Thr Thr Gly Cys Ala Glu His Cys Ser Leu Asn Glu
50 55 60

Asn Ile Thr Val Pro Asp Thr Lys Val Asn Phe Tyr Ala Trp Lys Arg
65 70 75 80

Met Glu Pro Trp Glu Pro Leu Gln Leu His Val Asp Lys Ala Val Ser
85 90 95

Gly Leu Arg Ser Leu Thr Thr Leu Leu Arg Ala Leu Gly Ala Gln Lys
100 105 110

Glu Ala Ile Ser Pro Pro Asp Ala Ala Ser Ala Ala Pro Leu Arg Thr
115 120 125

Ile Thr Ala Asp Thr Phe Arg Lys Leu Phe Arg Val Tyr Ser Asn Phe
130 135 140

Leu Arg Gly Lys Leu Lys Leu Tyr Thr Gly Glu Ala Cys Arg Thr Gly
145 150 155 160

Asp Arg

<210> 4
<211> 192
<212> PRT
<213> Mus musculus

<400> 4

Met Gly Val Pro Glu Arg Pro Thr Leu Leu Leu Leu Leu Ser Leu Leu
1 5 10 15

Leu Ile Pro Leu Gly Leu Pro Val Leu Cys Ala Pro Pro Arg Leu Ile
20 25 30

Cys Asp Ser Arg Val Leu Glu Arg Tyr Ile Leu Glu Ala Lys Glu Ala
35 40 45

Glu Asn Val Thr Met Gly Cys Ala Glu Gly Pro Arg Leu Ser Glu Asn
50 55 60

Ile Thr Val Pro Asp Thr Lys Val Asn Phe Tyr Ala Trp Lys Arg Met
65 70 75 80

Glu Val Glu Glu Gln Ala Ile Glu Val Trp Gln Gly Leu Ser Leu Leu

85 90 95

Ser Glu Ala Ile Leu Gln Ala Gln Ala Leu Leu Ala Asn Ser Ser Gln
100 105 110

Pro Pro Glu Thr Leu Gln Leu His Ile Asp Lys Ala Ile Ser Gly Leu
115 120 125

Arg Ser Leu Thr Ser Leu Leu Arg Val Leu Gly Ala Gln Lys Glu Leu
130 135 140

Met Ser Pro Pro Asp Thr Thr Pro Pro Ala Pro Leu Arg Thr Leu Thr
145 150 155 160

Val Asp Thr Phe Cys Lys Leu Phe Arg Val Tyr Ala Asn Phe Leu Arg
165 170 175

Gly Lys Leu Lys Leu Tyr Thr Gly Glu Val Cys Arg Arg Gly Asp Arg
180 185 190

<210> 5
<211> 132
<212> PRT
<213> Mus musculus

<400> 5

Met Gly Val Pro Glu Arg Pro Thr Leu Leu Leu Leu Leu Ser Leu Leu
1 5 10 15

Leu Ile Pro Leu Gly Leu Pro Val Leu Cys Ala Pro Pro Arg Leu Ile
20 25 30

Cys Asp Ser Arg Val Leu Glu Arg Tyr Ile Leu Glu Ala Lys Glu Ala
35 40 45

Glu Asn Val Thr Met Gly Cys Ala Glu Gly Pro Arg Leu Ser Glu Asn
50 55 60

Ile Thr Val Pro Asp Thr Lys Val Asn Phe Tyr Ala Trp Lys Arg Met
65 70 75 80

Glu Lys Glu Leu Met Ser Pro Pro Asp Thr Thr Pro Pro Ala Pro Leu
85 90 95

Arg Thr Leu Thr Val Asp Thr Phe Cys Lys Leu Phe Arg Val Tyr Ala
100 105 110

Asn Phe Leu Arg Gly Lys Leu Lys Leu Tyr Thr Gly Glu Val Cys Arg
115 120 125

Arg Gly Asp Arg
130

<210> 6
<211> 50
<212> DNA
<213> Artificial Sequence

<220>
<223> Primer

<400> 6
gaacttccaa ggatgaagac ttgcagcgtg gcagcagcat gtcacctgtc 50

<210> 7
<211> 32
<212> DNA
<213> Artificial Sequence

<220>
<223> Primer

<400> 7
tatggatcca tgggggtgcc cgaacgtccc ac 32

<210> 8
<211> 33
<212> DNA
<213> Artificial Sequence

<220>
<223> Primer

<400> 8
tatggatcct cacctgtccc ctctcctgca gac 33

<210> 9
<211> 24
<212> DNA
<213> Artificial Sequence

<220>
<223> Primer

<400> 9
gatgggggtg cacgaatgtc ctgc 24

<210> 10
<211> 25
<212> DNA
<213> Artificial Sequence

<220>
<223> Primer

<400> 10
cacacctggt catctgtccc ctgtc 25

<210> 11
<211> 27
<212> DNA
<213> Artificial Sequence

<220>

<223> Primer

<400> 11
tatggatcca tgggggtgca cgaatgt 27

<210> 12
<211> 31
<212> DNA
<213> Artificial Sequence

<220>
<223> Primer

<400> 12
caagatatct cacgtgatat tctcggcctc c 31

<210> 13
<211> 19
<212> DNA
<213> Artificial Sequence

<220>
<223> Primer

<400> 13
agtcctggag gcgtacctc 19

<210> 14
<211> 18
<212> DNA
<213> Artificial Sequence

<220>
<223> Primer

<400> 14
gaggtacgcc tccaggac 18

<210> 15
<211> 19
<212> DNA
<213> Artificial Sequence

<220>
<223> Primer

<400> 15
agtcctggag gcgtacctc 19

<210> 16
<211> 19
<212> DNA
<213> Artificial Sequence

<220>
<223> Primer

<400> 16
gaggtactcc tccaggact 19

<210> 17
<211> 32

<212> DNA
<213> Artificial Sequence

<220>
<223> Primer

<400> 17
tatggatcca tgggggtgcc cgaacgtccc ac 32

<210> 18
<211> 33
<212> DNA
<213> Artificial Sequence

<220>
<223> Primer

<400> 18
tcggaattct cacctgtccc ctctcctgca gac 33

<210> 19
<211> 27
<212> DNA
<213> Artificial Sequence

<220>
<223> Primer

<400> 19
tatggatcca tgggggtgca cgaatgt 27

<210> 20
<211> 27
<212> DNA
<213> Artificial Sequence

<220>
<223> Primer

<400> 20
agagaattct ctgtcccctg tcctgca 27

<210> 21
<211> 30
<212> DNA
<213> Artificial Sequence

<220>
<223> Primer

<400> 21
aaccatgggg gtgcacgaat gtcctgcctg 30

<210> 22
<211> 37
<212> DNA
<213> Artificial Sequence

<220>
<223> Primer

<400> 22
aaggatcctc aatggtgatg gtgatgatga ccggtac 37

<210> 23
<211> 30
<212> DNA
<213> Artificial Sequence

<220>
<223> Primer

<400> 23
tatggatccg ctcccccacg cctcatctgc 30

<210> 24
<211> 33
<212> DNA
<213> Artificial Sequence

<220>
<223> Primer

<400> 24
tcggaattct cacctgtccc ctctcctgca gac 33

<210> 25
<211> 30
<212> DNA
<213> Artificial Sequence

<220>
<223> Primer

<400> 25
tatggatccg ccccaccacg cctcatctgt 30

<210> 26
<211> 32
<212> DNA
<213> Artificial Sequence

<220>
<223> Primer

<400> 26
tcggaattct catctgtccc ctgtcctgca gg 32

<210> 27
<211> 35
<212> DNA
<213> Artificial sequence

<220>
<223> Primer

<400> 27
atctcgagat gtcagcacca aggatttggc tagcg 35

<210> 28
<211> 34
<212> DNA
<213> Artificial sequence

<220>

<223> Primer

<400> 28
aggtttaaac tcactgcggc atgtagccac cttg 34

<210> 29
<211> 582
<212> DNA
<213> Homo sapiens

<400> 29
atgggggtgc acgaatgtcc tgcctggctg tggcttctcc tgtccctgct gtcgctccct 60

ctgggcctcc cagtcctggg cgccccacca cgcctcatct gtgacagccg agtcctggag 120

aggtacctct tggaggccaa ggaggccgag aatatcacga cgggctgtgc tgaacactgc 180

agcttgaatg agaatatcac tgtcccagac accaaagtta atttctatgc ctggaagagg 240

atggaggtcg ggcagcaggc cgtagaagtc tggcagggcc tggccctgct gtcggaagct 300

gtcctgcggg gccaggccct gttggtcaac tcttcccagc cgtgggagcc cctgcagctg 360

catgtggata aagccgtcag tggccttcgc agcctcacca ctctgcttcg ggctctggga 420

gcccagaagg aagccatctc ccctccagat gcggcctcag ctgctccact ccgaacaatc 480

actgctgaca ctttccgcaa actcttccga gtctactcca atttcctccg gggaaagctg 540

aagctgtaca caggggaggc ctgcaggaca ggggacagat ga 582

<210> 30
<211> 495
<212> DNA
<213> Homo sapiens

<400> 30
atgggggtgc acgaatgtcc tgcctggctg tggcttctcc tgtccctgct gtcgctccct 60

ctgggcctcc cagtcctggg cgccccacca cgcctcatct gtgacagccg agtcctggag 120

aggtacctct tggaggccaa ggaggccgag aatatcacgg tcgggcagca ggccgtagaa 180

gtctggcagg gcctggccct gctgtcggaa gctgtcctgc ggggccaggc cctgttggtc 240

aactcttccc agccgtggga gcccctgcag ctgcatgtgg ataaagccgt cagtggcctt 300

cgcagcctca ccactctgct tcgggctctg ggagcccaga aggaagccat ctcccctcca 360

gatgcggcct cagctgctcc actccgaaca atcactgctg acactttccg caaactcttc 420

cgagtctact ccaatttcct ccggggaaag ctgaagctgt acacagggga ggcctgcagg 480

acaggggaca gatga 495

<210> 31
<211> 489
<212> DNA
<213> Homo sapiens

<400> 31
atgggggtgc acgaatgtcc tgcctggctg tggcttctcc tgtccctgct gtcgctccct 60

ctgggcctcc cagtcctggg cgccccacca cgcctcatct gtgacagccg agtcctggag 120

```
aggtacctct tggaggccaa ggaggccgag aatatcacga cgggctgtgc tgaacactgc      180
agcttgaatg agaatatcac tgtcccagac accaaagtta atttctatgc ctggaagagg      240
atggagccgt gggagcccct gcagctgcat gtggataaag ccgtcagtgg ccttcgcagc      300
ctcaccactc tgcttcgggc tctgggagcc cagaaggaag ccatctcccc tccagatgcg      360
gcctcagctg ctccactccg aacaatcact gctgacactt tccgcaaact cttccgagtc      420
tactccaatt tcctccgggg aaagctgaag ctgtacacag gggaggcctg caggacaggg      480
gacagatga                                                              489

<210>  32
<211>  579
<212>  DNA
<213>  Mus musculus

<400>  32
atgggggtgc ccgaacgtcc caccctgctg cttttactct ccttgctact gattcctctg       60
ggcctcccag tcctctgtgc tcccccacgc ctcatctgcg acagtcgagt tctggagagg      120
tacatcttag aggccaagga ggcagaaaat gtcacgatgg gttgtgcaga aggtcccaga      180
ctgagtgaaa atattacagt cccagatacc aaagtcaact tctatgcttg gaaaagaatg      240
gaggtggaag aacaggccat agaagtttgg caaggcctgt ccctgctctc agaagccatc      300
ctgcaggccc aggccctgct agccaattcc tcccagccac cagagaccct tcagcttcat      360
atagacaaag ccatcagtgg tctacgtagc ctcacttcac tgcttcgggt actgggagct      420
cagaaggaat tgatgtcgcc tccagatacc accccacctg ctccactccg aacactcaca      480
gtggatactt tctgcaagct cttccgggtc tacgccaact tcctccgggg gaaactgaag      540
ctgtacacgg gagaggtctg caggagaggg gacaggtga                             579

<210>  33
<211>  399
<212>  DNA
<213>  Mus musculus

<400>  33
atgggggtgc ccgaacgtcc caccctgctg cttttactct ccttgctact gattcctctg       60
ggcctcccag tcctctgtgc tcccccacgc ctcatctgcg acagtcgagt tctggagagg      120
tacatcttag aggccaagga ggcagaaaat gtcacgatgg gttgtgcaga aggtcccaga      180
ctgagtgaaa atattacagt cccagatacc aaagtcaact tctatgcttg gaaaagaatg      240
gagaaggaat tgatgtcgcc tccagatacc accccacctg ctccactccg aacactcaca      300
gtggatactt tctgcaagct cttccgggtc tacgccaact tcctccgggg gaaactgaag      360
ctgtacacgg gagaggtctg caggagaggg gacaggtga                             399
```